# EUROPEAN PATENT SPECIFICATION

(11) **EP 2 175 835 B1**
(45) Date of publication and mention of the grant of the patent: **20.04.2016**
(21) Application number: 08780044.7
(22) Date of filing: 09.07.2008
(51) Int. Cl.: A61K 9/127, A61K 39/29

(54) **METHODS FOR GENERATING IMMUNE RESPONSE USING CATIONIC-LIPOSOME-MEDIATED NUCLEIC ACID DELIVERY**
VERFAHREN ZUR ERZEUGUNG EINER IMMUNANTWORT MIT DURCH KATIONISCHEM LIPOSOM VERMITTELTER NUKLEINSÄURE-ABGABE
PROCÉDÉS DE GÉNÉRATION D'UNE RÉPONSE IMMUNITAIRE PAR ADMINISTRATION D'ACIDE NUCLÉIQUE INDUIT PAR LIPOSOME CATIONIQUE

(30) Priority: 09.07.2007 US 929685 P
(43) Date of publication of application: 21.04.2010
(73) Proprietor: Georgetown University, Washington, DC 20057 (US)
(72) Inventor: CHANG, Esther, H., Potomac, MD 20854 (US); PIROLLO, Kathleen, F., Rockville, MD 20850 (US)
(74) Representative: Daniels, Jeffrey Nicholas
(86) International application number: PCT/US2008/008394
(87) International publication number: WO 2009/009054

(56) References cited:
- US-A- 4 957 735
- US-A1- 2006 223 769
- US-A1- 2007 065 432
- US-A1- 2007 065 499
- US-A1- 2007 065 499
- JIAO X ET AL: "MODULATION OF CELLULAR IMMUNE RESPONSE AGAINST HEPATITIS C VIRUS NONSTRUCTURAL PROTEIN 3 BY CATIONIC LIPOSOME ENCAPSULATED DNA IMMUNIZATION", HEPATOLOGY, WILLIAMS AND WILKINS, BALTIMORE, MD, US, vol. 37, no. 2, 1 February 2003 (2003-02-01), pages 452-460, XP008032301, ISSN: 0270-9139, DOI: DOI:10.1053/JHEP.2003.50051
- JIAO XUANMAO ET AL: "Enhanced hepatitis C virus NS3 specific Th1 immune responses induced by co-delivery of protein antigen and CpG with cationic liposomes", JOURNAL OF GENERAL VIROLOGY, vol. 85, no. Part 6, June 2004 (2004-06), pages 1545-1553, XP002644537, ISSN: 0022-1317
- YU WEI ET AL: "Enhanced transfection efficiency of a systemically delivered tumor-targeting immunolipoplex by inclusion of a pH-sensitive histidylated oligolysine peptide -", NUCLEIC ACIDS RESEARCH, vol. 32, no. 5, March 2004 (2004-03), XP008129590, ISSN: 0305-1048
- YU W. ET AL.: 'Enhanced transfection efficiency of a systemically delivered tumor-targeting immunolipoplex by inclusion of a pH-sensitive histidylated oligolysine peptide' NUCLEIC ACIDS RESEARCH vol. 32, no. 5, 2004, pages 1 - 10, XP008129590
- XU ET AL.: 'Synstemic p53 Gene Therapy of Cancer with Immunolipoplexes Targeted by Anti-Transferrin Receptor scFv' MOLECULAR MEDICINE vol. 7, no. 10, 2001, pages 723 - 734, XP008129592
- GURSEL I ET AL: "Sterically stabilized cationic liposomes improve the uptake and immunostimulatory activity of CpG oligonucleotides.", JOURNAL OF IMMUNOLOGY (BALTIMORE, MD. : 1950) 15 SEP 2001, vol. 167, no. 6, 15 September 2001 (2001-09-15), pages 3324-3328, ISSN: 0022-1767
- HAMZAH JULIANA ET AL: "Targeted Liposomal Delivery of TLR9 Ligands Activates Spontaneous Antitumor Immunity in an Autochthonous Cancer Model", JOURNAL OF IMMUNOLOGY, vol. 183, no. 2, July 2009 (2009-07), pages 1091-1098, ISSN: 0022-1767
- HENNESSY ELIZABETH J ET AL: "Targeting Toll-like receptors: emerging therapeutics?", NATURE REVIEWS DRUG DISCOVERY, vol. 9, no. 4, April 2010 (2010-04), pages 293-307, ISSN: 1474-1776
- SCHMIDT CHARLIE: "Clinical setbacks for toll-like receptor 9 agonists in cancer.", NATURE BIOTECHNOLOGY AUG 2007, vol. 25, no. 8, August 2007 (2007-08), pages 825-826, ISSN: 1087-0156
- BROCKER CHAD ET AL: "Evolutionary divergence and functions of the human interleukin (IL) gene family", HUMAN GENOMICS, vol. 5, no. 1, October 2010 (2010-10), pages 30-55, ISSN: 1473-9542(print)
- HAMZA THERWA ET AL: "Interleukin 12 a key immunoregulatory cytokine in infection applications.", INTERNATIONAL JOURNAL OF MOLECULAR SCIENCES 2010, vol. 11, no. 3, 2010, pages 789-806, ISSN: 1422-0067
- David Male: "Immunology, An Illustrated Outline (4th Edition)", 2004, Mosby publishers, Spain * page 64 - page 65 *
- Male et al.: "Immunology (7th Edition)", 2006, Mosby/Elsevier, Canada * page 7 * * page 531 *

## Description

### BACKGROUND OF THE INVENTION

### Field of the Invention

The present invention is in the field of cationic liposome-based vaccines in embodiments, this invention provides methods of making ligand-targeted (anti-transferrin receptor scFv-targeted) liposomes useful for the delivery of molecules to induce immune responses against viral antigens or to treat or prevent viral diseases. The specificity of the delivery system is derived from the targeting ligands.

### Background of the Invention

Vaccines represent a powerful weapon against highly transmissible infectious pathogens and have served to significantly reduce the burden of infectious diseases in the past century. However, the threat to global health posed by infectious diseases has not receded but rather has become a national and global priority. The impact of viral pathogens such as Tuberculosis and Malaria, for example, continues to be felt on a global scale, and newly emerging pathogens such as HIV/AIDS, SARS-coronavirus and the recently emerging pandemic influenza or 'bird flu' virus present new challenges for public health.

Most vaccine technologies involve either inactivated vaccine formulations, protein subunit based or live-attenuated in nature. For example, current production and manufacturing technologies for influenza vaccines are based on inoculation of chicken eggs and remains a time and cost-intensive process. Smallpox vaccines, which are live-attenuated viruses, are associated with serious adverse events in immunocompromised populations. Thus, current vaccine technologies are outdated and insufficient to meet the national or global demand for vaccines.

US 2006/0223769 to Dow et al. discloses a vaccine and a method for immune activation for eliciting both a systemic, non-antigen specific immune response and a strong antigen-specific immune response in a mammal. The method is for protecting a mammal from a disease including cancer, a disease associated with allergic inflammation, an infectious disease, or a condition associated with a deleterious activity of a self-antigen. Also disclosed are therapeutic compositions useful in such a method.

There is, therefore, an urgent need to develop new vaccine technologies that will address the challenges posed by highly mutable and emerging pathogens. The present invention fulfills these needs by providing a cation-liposome-based drug delivery system for use in inducing immune responses to antigens and for treatment or prevention of viral diseases in patients.

### BRIEF SUMMARY OF THE INVENTION

The present invention provides methods of preparing a ligands-targeted cationic liposome. Such methods comprise preparing a ligand TfRscFv and mixing the ligand with a cationic liposome to form a ligand-targeted cationic liposome. The ligand is directly associated/complexed with the cationic liposome, but is not chemically conjugated to the cationic liposome. Then, the ligand-targeted cationic liposome is mixed with one or more nucleic acid molecules encoding one or more viral proteins, and/or one or more nucleic acid molecules encoding one or more interleukins, to form the ligand-targeted cationic liposome complex. Accordingly, the present invention provides a ligand-targeted cationic lipsome complex comprising a cationic liposome, a ligand which is an anti-transferrin receptor scFV one or more nucleic acid molecules encoding one or more viral proteins, and one or more nucleic acid molecules encoding one or more interleukins, wherein said antibody or antibody fragment is directly complexed with, but not chemically conjugated to, said cationic liposome. The present invention also provides a method of preparing such a ligand-targeted cationic immunoliposome complex, which comprises: (a) preparing the ligand; (b) mixing the ligand with a cationic liposome to form a ligand-targeted cationic liposomes, wherein the ligand is directly complexed with, but not chemically conjugated, to the cationic liposome, and (c) mixing the ligand-targeted cationic liposome with: one or more nucleic acid molecules encoding one or more viral proteins; and one or more nucleic acid molecules encoding one or more interleukins, to form the ligand-targeted-cationic liposome complex, which complex is optionally used in the manufacture of a medicament for inducing an immune response against a viral antigen in a mammal and/or for treating or preventing a viral disease in a mammal, wherein said viral antigen is from a virus selected from the group consisting of influenza virus, HIV virus, SARS virus, bird flu virus, Ebola virus, Hepatitis B virus and small pox virus, wherein said complex is administered as a bolus or as an infusion via route selected from the group consisting of intravenous administration, oral administration intramuscular administration, intralesional administration, intradermal administration, transdermal administration, intraocular administration, intraperitoneal administration, percutaneous administration, aerosol administration intranasal administration, intraorgan administration, intracereberal administration, topical administration, subcutaneous administration, endoscopic administration, slow release implant and administration via an osmotic or mechanical pump. In further embodiments, the methods can further comprise mixing the ligand-targeted cationic liposome with a peptide comprising the K[K(H)KKK]5-K(H)KKC (HoKC)(SEQ ID NO: 1) peptide.

Suitably, the ligand TfRscFv is mixed with the cationic liposome at a ratio in the range of about 1 : 1 to about 1: 100; suitably about 1 :10 to about 1:50 (w:w), more suitably about of about 1 :20 to about 1:40. In exemplary embodiments, the cationic liposome comprises a mixture of dioleoyltrimethylammonium phosphate (DOTAP) with dioleoylphosphatidylethanolamine (DOPE) and/or cholesterol (chol); or a mixture of dimethyldioctadecylammonium bromide (DDAB) with dioleoylphosphatidylethanolamine and/or cholesterol.

The nucleic acids are suitably present at a molar ratio of about 0.1 mole of one or more nucleic acid molecules encoding one or more viral proteins, to about 10 mole of one or more nucleic acid molecules encoding one or more interleukins; about 10 mole of one or more nucleic acid molecules encoding one or more viral proteins, to about 0.1 mole of one or more nucleic acid molecules encoding one or more interleukins. For example, a ratio of about 1 mole of one or more nucleic acid molecules encoding one or more viral proteins, to about 1 mole of one or more nucleic acid molecules encoding one or more interleukins can be utilized. In exemplary embodiments, the cationic immunoliposome is mixed with the one or more nucleic acid molecules encoding one or more viral proteins, and the one or more nucleic acid molecules encoding one or more interleukins, at a weight ratio of between about 1 :1 to about 1 :40 (µg total nucleic acid:µg liposome), suitably about 1 :5 to about 1 :20 (µg total nucleic acid:µg liposome), e.g., about 1 : 10 (µg total nucleic acid:µg liposome).

Examples of viral proteins that can be encoded by the nucleic acid molecules include, but are not limited to, viral proteins selected from the group consisting of HFV viral proteins, influenza viral proteins, Hepatitis A, B or C viral proteins, SARS viral proteins, bird flu viral proteins, Ebola viral proteins, West Nile viral proteins and small pox viral proteins. Examples of interleukins that can be encoded by the nucleic acid molecules include, but are not limited to, interleukins selected from the group consisting of IL- 1, IL-2, IL-3, IL-4, IL-5, IL-6, IL-7, IL-8, IL-9, IL-10, IL-12, IL-13, IL-15, IL-17, IL-18 and IL-23. In suitable embodiments, the nucleic acids encode IL-2, IL-12 and IL-15. The present invention also provides cationic immunoliposome complexes prepared by the various methods of the present invention.

The present invention provides anti-transferrin scFV (TfRscFv)--targeted cationic liposome complexes comprising a cationic liposome, the ligand (TfRscFv ), one or more nucleic acid molecules encoding one or more viral proteins, and one or more nucleic acid molecules encoding one or more interleukins, wherein the ligand is directly complexed with, but is not chemically conjugated to, the cationic liposome. In a further disclosure, the ligand can be chemically conjugated to the liposome.

The present invention also provides pharmaceutical compositions comprising the various liposome complexes of the present invention. Additional pharmaceutical compositions of the present invention comprise a first ligand-targeted cationic liposome, complex comprising a cationic liposome, the ligand (TfRscFv) and one or more nucleic acid molecules encoding one or more viral proteins, wherein the ligand is directly complexed with, but is not chemically conjugated to, the cationic liposome. In a further disclosure, the ligand can be chemically conjugated to the cationic liposome. In suitable embodiments, the pharmaceutical compositions also further comprise a second ligand-targeted cationic liposome complex comprising a cationic liposome, a ligand (e.g., protein/peptide, antibody or antibody fragment) and one or more nucleic acid molecules encoding one or more interleukins, wherein the ligand is directly complexed with, but is not chemically conjugated to, the cationic liposome. In a further disclosure, the ligand can be chemically conjugated to the cationic liposome.

Also described are methods of delivering one or more active agents to antigen presenting cells (APCs) of a mammal comprising administering to the mammal an anti-transferrin receptor single chain Fv (TfRscFv)-targeted cationic immunoliposome complex comprising a cationic liposome, a TfRscFv, and one or more active agents, wherein the TfRscFv is directly associated/complexed with, but is not chemically conjugated to, the cationic liposome. In further embodiments, the TfRscFv can be chemically conjugated to the cationic liposome. Suitably, the methods deliver agents (e.g., nucleic acids) to professional and non-professional APCs, including APCs located in the liver, spleen, lymph nodes, gut and/or respiratory tract.

The present specification also describes methods of inducing an immune response against a viral antigen in a mammal, comprising administering the various cationic liposome, complexes of the present invention, or the pharmaceutical compositions of the present invention, to the mammal.

Also described are methods of treating or preventing a viral disease in a mammal comprising administering the various cationic liposome complexes of the present invention, or the pharmaceutical compositions of the present invention, to the mammal.
The present invention also provides an anti-transferrin receptor single chain Fv (TfRscFv)-targeted cationic immunoliposome complex for TfRscFv-targeted delivery for use in delivering nucleic acid molecules to professional or unprofessional antigen presenting cells (APCs) of a mammal, comprising a cationic liposome, a TfRscFv, and nucleic acid molecules, wherein the TfRscFv is directly complexed with, but not chemically conjugated to, the cationic liposome, and preferably, wherein the APCs are located in the liver, spleen, lymph nodes, gut and/or respiratory tract, wherein said complex is administered as a bolus or as an infusion via route selected from the group consisting of intravenous administration, oral administration intramuscular administration, intralesional administration, intradermal administration, transdermal administration, intraocular administration, intraperitoneal administration, percutaneous administration, aerosol administration intranasal administration, intraorgan administration, intracereberal administration, topical administration, subcutaneous administration, endoscopic administration, slow release implant and administration via an osmotic or mechanical pump, wherein said nucleic acid molecules encode, one or more viral proteins selected from the group consisting of HIV viral proteins, Ebola viral proteins, influenza viral proteins, SARS viral proteins, bird flu viral proteins, Hepatitis B viral proteins and small pox viral proteins, and wherein said nucleic acid molecules also encode one or or more interleukins selected from the group consisting of IL-1, IL-2, IL-3, IL-4, IL-5, IL-6, IL-7, IL-8, IL-9, IL-I0, IL-12, IL-13, IL-15, IL-17, IL-18 and IL-23. The present invention is defined by the claims. Subject matter outside the scope of the claims is not in accordance with the present invention and is provided for comparison only.

### BRIEF DESCRIPTION OF THE DRAWINGS/FIGURES

The patent or application file contains at least one drawing executed in color.
Copies of this patent or patent application publication with color drawing(s) will be provided by the Office upon request and payment of the necessary fee.
Figure 1 shows luciferase activity in Hep3B cells transfected with liposomes (with or without inclusion of the HoKC peptide) complexed with the anti-transferrin receptor single-chain antibody fragment (sc) and the luciferase reporter gene.
Figure 2 shows luciferase expression in primary chimpanzee hepatocytes transfected with three different liposomes (A, D and G) complexed with sc, Gal or L-37-pA, and DNA expressing the luciferase reporter gene. All data are shown as the means +SD (n=3).
Figure 3 shows a western blot showing GFP expression in liver extracts from duplicate mice inoculated with 20-25µg of DNA expressing EGFP complexed with TfRscFv ligand (sc) conjugated with Lipid A (scLA), Lipid A and HoKC (scLAHK), Lipid D and HoKC (scLDHK), Lipid G and HoKC (scLGHK), Galactose conjugated with Lipid A (GalLA) and L-37pA conjugated with Lipid A (L-37pALA) compared to untreated mice (UT).
Figure 4 shows western blots of cells transfected with DNA plasmids expressing HCV antigens NS3-NS5B (pAG410). Blots were probed with monoclonal antibody to NS5B (left) and serum from a chronically infected chimpanzee (right).
Figure 5 shows comparative expression of IL-2 under the pSCMV and CMV promoters.
Figure 6 shows DNA PCR amplification ofNS3 in mouse liver after i.v. injection of scL complexed or free pAG410.
Figure 7 shows inhibition of HCV recombinant vaccinia virus replication in mice inoculated with scL-HCVIscL-IL12.
Figure 8A and 8B show HCV anti-HVR1 antibodies (bars) and RNA profiles (black triangles) during vaccination and challenge with HCV of a naive animal (Ch1601) and a recovered animal (Ch1587). Anti-HVR1 expressed as P/N ratios. P/N is calculated by dividing the OD405 of test sera by that obtained for a pre-vaccine sample from the same chimpanzee. Cut-off value is P/N=2.
Figures 9A-9C show the disease profile during T cell-based vaccination and challenge with HCV of two recovered chimpanzees (Ch1588 and Ch1606) and a naïve chimpanzee (Ch6394). RNA titers (black squares), ALT levels (black triangles). HCV challenge of the immunized animals was carried out at week 0 (100 CID₅₀ of cHCV).
Figure 10A-10C show elispot responses against peptide pools in chimpanzees immunized with scL-HCV and boosted with recombinant adenovirus (Ch1611 and Ch6407) or immunized with scL-empty vector and boosted with non-recombinant adenovirus (Ch0257).

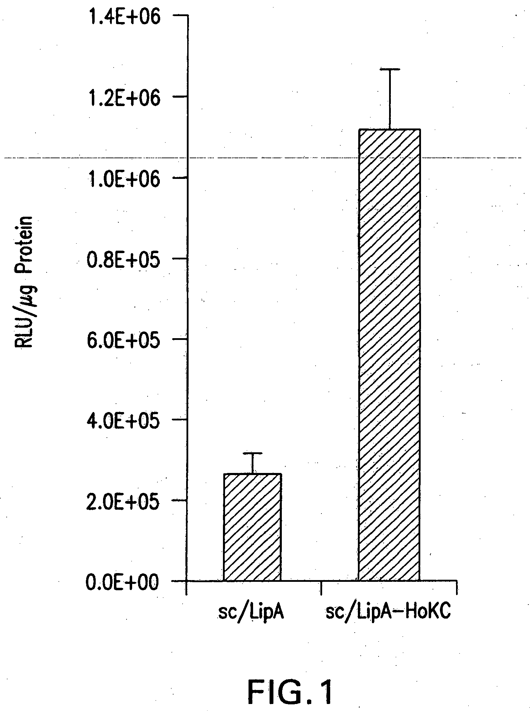

Figure 1 IA- 1 1C show Elispot responses in immunized (Ch6407 and 161 1) and control (Ch 0257) chimps before and after challenge.

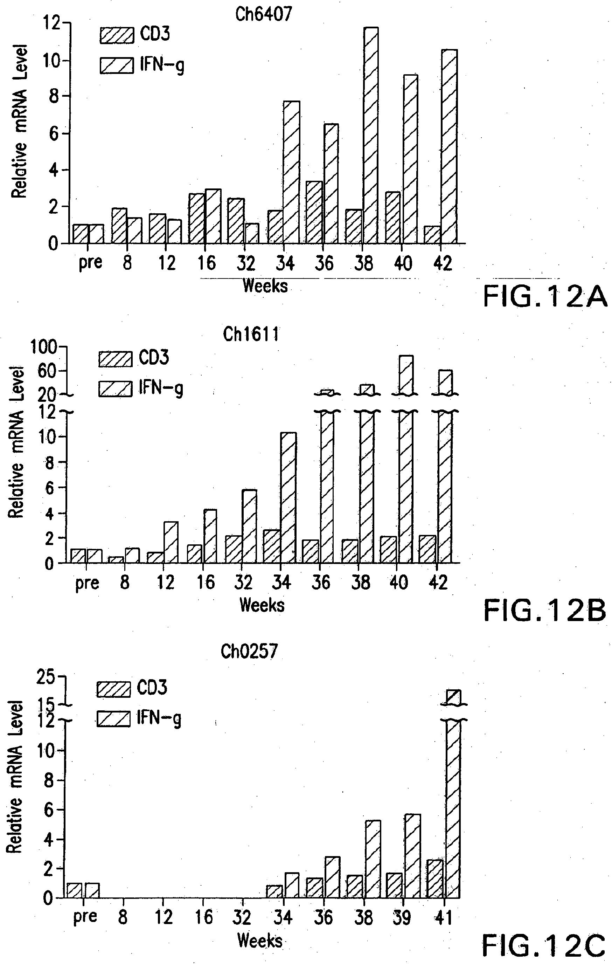

Figure 12A-12C show intrahepatic cytokine mRNA levels in vaccinated (Ch6407 and 1611) and control (Ch 0257) chimpanzees.

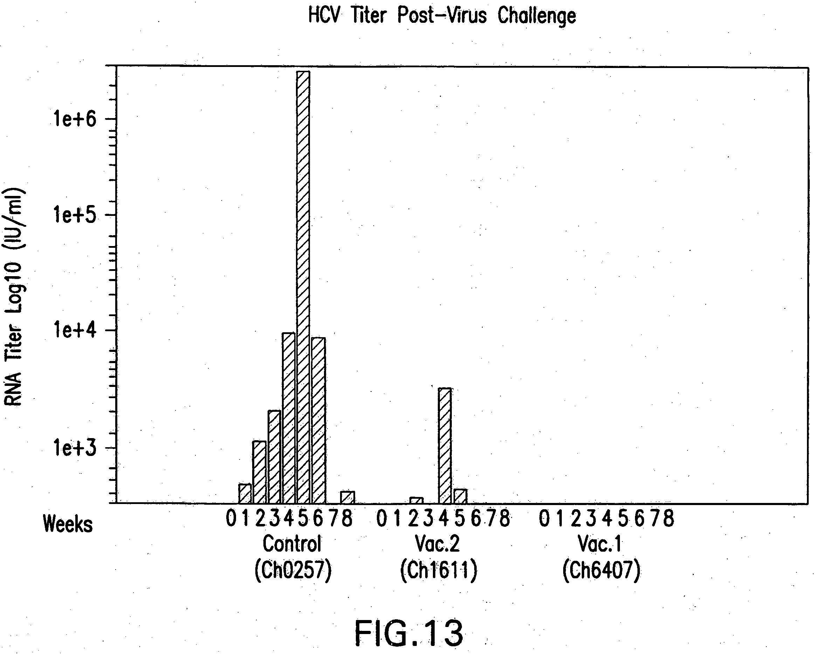

Figure 13 shows HCV RNA titers post challenge in control (Ch 0257) and vaccinated (Ch6407 and 161 1) chimpanzees.

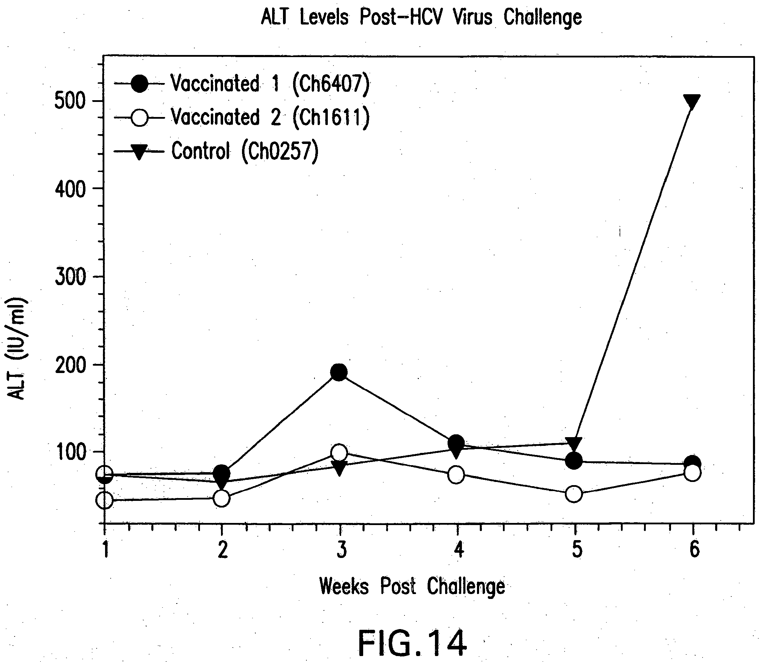

Figure 14 shows Serum ALT levels post-challenge in control (Ch 0257) and vaccinated (Ch6407 and 161 1) chimpanzees.

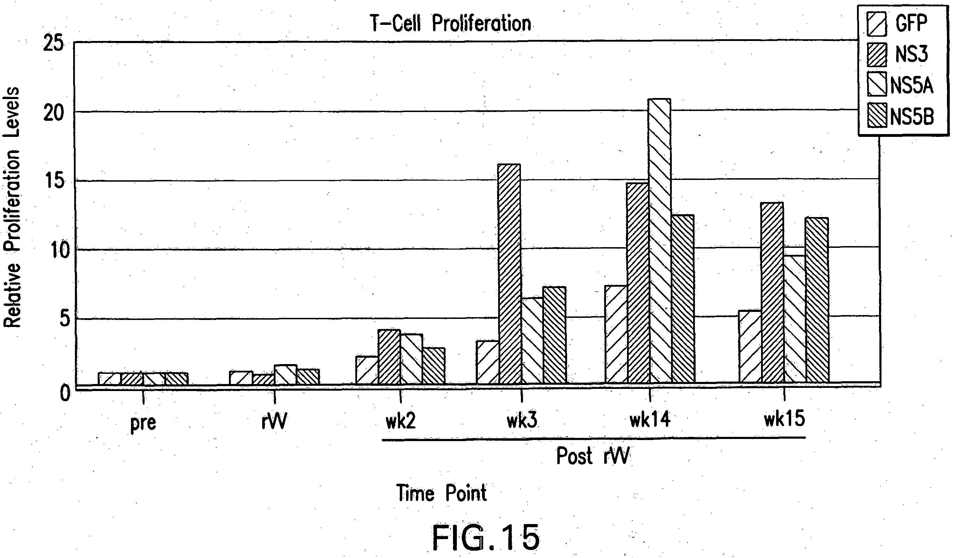

Figure 15 shows PBMC T-cell responses in a chronically infected chimpanzee.

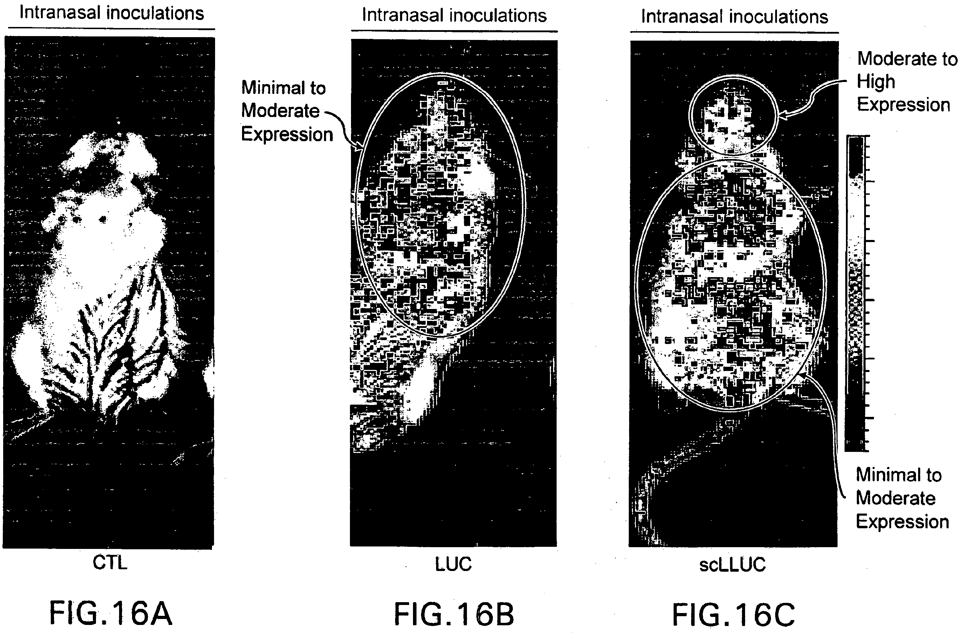

Figures 16A-16C show optical imaging of BALB/c mice following IN administration of plasmid DNA that expresses the Luciferase gene either as free (naked) plasmid DNA (LUC) (B) or encapsulated in the scL nanoimmunoliposome complex (scLLUC) (C) followed by subsequent luciferin substrate injection. Panel A represents an untreated control.

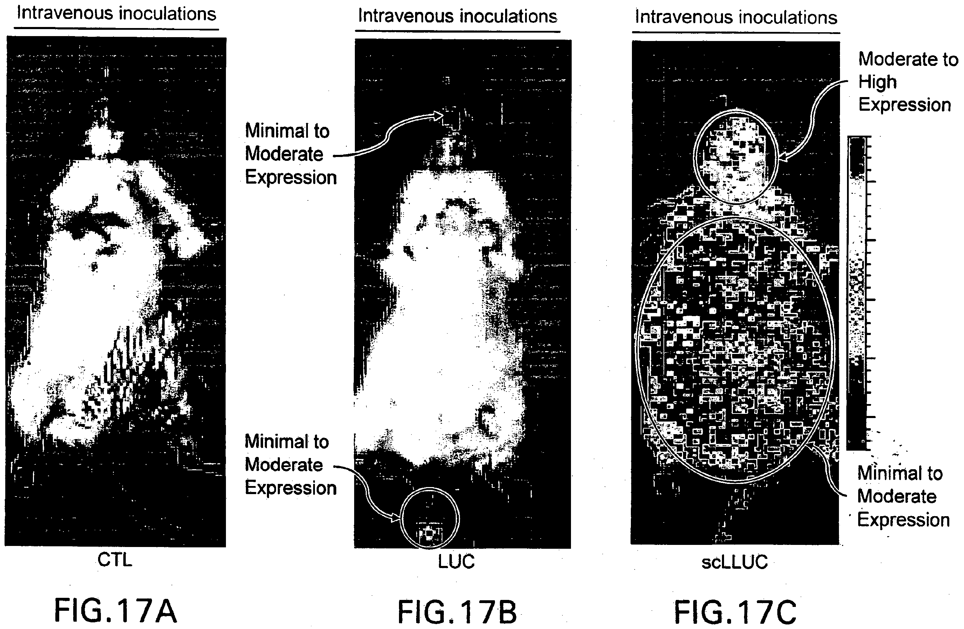

Figures 17A-17C shows optical imaging of BALB/c mice following IV administration of plasmid DNA that expresses the Luciferase gene either as free (naked) plasmid DNA (LUC) (B) or encapsulated in the scL nanoimmunoliposome complex (scLLUC) (C) followed by subsequent luciferin substrate injection. Panel A represents an untreated control.

### DETAILED DESCRIPTION OF THE INVENTION

Ligand-targeted (e.g., protein/peptide, antibody- or antibody fragment- targeted) cationic liposome or cationic polymer complexes in accordance with embodiments of this invention are suitably made by a simple and efficient non-chemical conjugation method in which the components of the desired complex are mixed together in a defined ratio and in a defined order. Ligand-targeted cationic liposome or cationic polymer complexes may be made by chemical conjugation method in which the ligands are chemically conjugated to the surface of the cationic liposomes via a chemical bond.

The terms "immunocomplex," "immunoliposome," "complex," "nanocomplex," "immunonanocomplex," "liposome complex" and "nanoimmunoliposome" are used interchangeably throughout to refer to the cationic liposomes of the present invention. Exemplary cationic liposomes for use in the practice of the present invention and methods of production thereof are disclosed in U.S. Published Patent Application No. 2003/0044407 and U.S. Patent Application No. 11/520,796, filed September 14, 2006

As used herein the term "about" refers to the recited value, as well as values within 10% of the recited value. For example, "about 100 run" includes the values of 90 nm to 110 nm, including values in between this range.

As used herein the term "ligand" refers to any suitable targeting moiety that can be either chemically conjugated to, or directly associated/complexed with, but not chemically conjugated to, the cationic liposomes. Exemplary ligands for use in the practice of the present invention include, but are not limited to, proteins (e.g., transferrin or folate), peptides (e,g., L-37pA), antibodies, antibody fragments (including Fab' fragments and single chain Fv fragments) and sugars (e.g., galactose), as well as other targeting molecules.

Exemplary methods and compositions in which the complexes in accordance with embodiments of this invention are made by a simple and efficient non-chemical conjugation are disclosed in U.S. Published Patent Application No. 2003/0044407 and U.S. Patent Application No. 11/520,796, filed September 14, 2006. Exemplary methods and compositions in which the complexes in accordance with embodiments of this invention are made via chemical conjugation are disclosed in U.S. Patent Application No. 09/914,046, filed October 1, 2001.

In exemplary embodiments, a whole antibody or an antibody fragment can be used as the ligand to make the complexes of this invention. In a suitable embodiment, an antibody fragment is used, including Fab fragments and single chain Fv fragments (scFv) of an antibody. One suitable antibody is an anti-Transferrin receptor (anti-TfR) monoclonal antibody, and a suitable antibody fragment is an scFv based on an anti=TfR monoclonal antibody. A suitable anti-TfR monoclonal antibody is 5E9 (see, e.g., Hayes, B. F., et al., "Characterization of a Monoclonal Antibody (5E9) that Defines a Human Cell Surface Antigen of Cell Activation," J. Immunol. 727:347-352 (1981); Batra, J.K., et al., "Single-chain Immunotoxins Directed at the Human Transferrin Receptor Containing Pseudomonas Exotoxin A or Diphtheria Toxin: Anti-TFR(Fv)-PE40 and DT388-Anti-TFR(Fv),"Mol. Cell. Biol. 77:2200-2205 (1991); the disclosures of which are incorporated herein by reference). An scFv based on 5E9 antibody contains the complete antibody binding site for the epitope of the TfR recognized by this MAb as a single polypeptide chain of approximate molecular weight 26,000. An scFv is formed by connecting the component VH and VL variable domains from the heavy and light chains, respectively, with an appropriately designed peptide, which bridges the C-terminus of the first variable region and N-terminus of the second, ordered as either VH-peptide-VL or VL-peptide-VH. Additional ligands, such as those described throughout, can also be used in the practice of the present invention.

In one embodiment, a cysteine moiety is added to the C-terminus of the scFv. Although not wishing to be bound by theory, it is believed that the cysteine, which provides a free sulfhydryl group, may enhance the formation of the complex between the antibody. and the liposome in both the chemically conjugated and non-chemically conjugated embodiments. With or without the cysteine, the protein can be expressed in *E.coli* inclusion bodies and then refolded to produce the antibody fragment in active form.

Unless it is desired to use a sterically stabilized immunoliposome in the formation of the complexes, a first step in making exemplary non-chemically conjugated complexes of the present invention comprise mixing a cationic liposomes or combination of liposomes. with the antibody or antibody fragment of choice. A wide variety of cationic liposomes are useful in the preparation of the complexes of this invention. Published PCT application WO99/25320 describes the preparation of several cationic liposomes. Examples of suitable liposomes include phosphatidylcholine (PC), phosphatidylserine (PS) and those that comprise a mixture of dioleoyltrimethylammonium phosphate (DOTAP) and dioleoylphosphatidylethanolamine (DOPE) and/or cholesterol (chol); a mixture of dimethyldioctadecylammonium bromide (DDAB) and DOPE and/or chol. The ratio of the lipids can be varied to optimize the efficiency of uptake of the therapeutic molecule for he specific target cell type. The liposome can comprise a mixture of one or more cationic lipids and one or more neutral or helper lipids. A desirable ratio of cationic lipid(s) to neutral or helper lipid(s) is about 1:(0.5-3), preferably 1:(1-2) (molar ratio).

Suitable ligands, for example, proteins/peptides, antibody or antibody fragments, are those which will bind to the surface of the target cell, and preferably to a receptor that is differentially expressed on the target cell. The ligands are mixed with the cationic liposome or polymer at room temperature and at a ligand (e.g., protein):lipid ratio (weight:weight) in the range of about 1 : 10 to about 1 :50, suitably about 1 :20 to about 1 :40 (w.w).

The ligand (e.g., the protein/peptide, antibody or antibody fragment) and the liposome are allowed to incubate at room temperature for a short period of time, typically for about 10-15 minutes, then the mixture is mixed with a therapeutic or diagnostic agent of choice. Examples of therapeutic molecules or agents which can be complexed to the liposome, complexes include genes, high molecular weight DNA (genomic DNA), plasmids DNA, antisense oligonucleotides, peptides, ribozymes, nucleic acids (including siRNA and antisense), small molecules, viral particles, immunomodulating agents, contrast agents for imaging, proteins and chemical agents.

The ligand (e.g., the protein/peptide, antibody or antibody fragment) and liposome combination is mixed with the therapeutic agent at a ratio in the range of about 0.5: 1 to about 1:40 (µg of agent:nmol of total lipid), suitably about 1:10 to 1 :20 (µg of agent:nmole of total lipid) arid incubated at room temperature for a short period of time, typically about 10 to 15 minutes. The size of the liposome complex is typically within the range of about 50-500 nm as measured by dynamic light scattering using a Malvern ZETASIZER® 3000 or a Malvern ZETASIZER^{®} NANO-ZS. See U.S. Published Patent Application No. 2003/0044407 and U.S. Patent Application No. 11/520,796.

In one embodiment of this invention, the liposome used to form the complex is a sterically stabilized liposome. Sterically stabilized liposomes are liposomes into which a hydrophilic polymer, such as PEG, poly(2-ethylacrylic acid), or poly(n-isopropylacrylamide (PNPAM) has been integrated. Such modified liposomes can be particularly useful when complexed with therapeutic agents, as they typically are not cleared from the bloodstream by the reticuloendothelial system as quickly as are comparable liposomes that have not been so modified. To make a sterically stabilized liposome complex of the present invention, the order of mixing the antibody or antibody fragment, the liposome and the therapeutic or diagnostic agent is reversed from the order set forth above. In a first step, a cationic liposome as described above is first mixed with a therapeutic agent as described above at a ratio in the range of about 0.5: 1 to about 1 :40 (µg of agent:nmol of lipid), suitably about 1:10 to 1 :20 (µg of agent:nmole of lipid). To this lipoplex is added a solution of a PEG polymer in a physiologically acceptable buffer at a ratio of about 0.1:100 (nmol of PEG:nmol of liposome), suitably, about 0.5:50, for example, about 1:40 (nmol of PEG:nmol of liposome). The resultant solution is incubated at room temperature for a time sufficient to allow the polymer to integrate into the liposome complex. The ligand (e.g., protein/peptide, antibody or antibody fragment) then is mixed with the stabilized liposome complex at room temperature and at a ligand (e.g., protein):lipid ratio in the range of about 1:5 to about 1:40 (w:w).

The liposomal complexes prepared in accordance with the present invention can be formulated as a pharmacologically acceptable formulation for *in vivo* administration. The complexes can be combined with a pharmacologically compatible vehicle or carrier. The compositions can be formulated, for example, for intravenous administration to a mammal, for example a human patient to be benefited by administration-of the therapeutic molecule, or other payload, in the complex. The complexes are sized appropriately so that they are distributed throughout the body following i.v. administration. Alternatively, the complexes can be delivered via other routes of administration, such as intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump. Preparation of formulations for delivery via such methods, and delivery using such methods, are well known in the art.

The complexes Can be optimized for target cell type through the choice and ratio of lipids, the ratio of ligand (e.g., protein/peptide, antibody or antibody fragment) to liposome, the ratio of ligand and liposome to the therapeutic agent, and the choice of ligand and therapeutic agent.

The complexes made in accordance with the methods of this invention can be provided in the form of kits for use in the systemic delivery of a nucleic acid, therapeutic molecule, or other payload by the complex. Suitable kits can comprise, in separate, suitable containers, the liposome, the ligand (e.g., protein/peptide, antibody or antibody fragment), and the nucleic acid, the therapeutic or diagnostic agent. The components can be mixed under sterile conditions in the appropriate order and administered to a patient within a reasonable period of time, generally from about 30 minutes to about 24 hours, after preparation. The kit components preferably are provided as solutions or as dried powders. Components provided in solution form preferably are formulated in sterile water-for-injection, along with appropriate buffers, osmolality control agents, etc. The complete complex can also be formulated as a dried powder (lyophilized) (see, e.g., U.S. Published Patent Application No. 2005/0002998).

As discussed throughout U.S. Published Patent Application No. 2003/0044407 and U.S. Patent Application No. 11/520,796, the cationic immunoliposome complexes described throughout have successfully delivered various therapeutic and diagnostic agents to tumor cells, via targeting using the anti-transferring single chain antibody fragment (TfRscFv). Specifically, nucleic acid molecules, e.g., antisense and siRNA, as well as plasmids DNA (such as p53 and RB94), have been successfully delivered using the immunoliposome complexes of the present invention (see U.S. Published Patent Application No. 2003/0044407 and U.S. Patent Application No. 1 1/520,796). Small molecules have also been delivered by the methods and liposomes disclosed throughout (see, U.S. Patent Application No. 11/798,296, filed May 11, 2007). As discussed throughout, it has now been discovered that vaccine constructs can be prepared, targeted to, and efficiently transfect antigen presenting cells (APCs) wherever they occur in the body, including but not limited to, liver hepatocytes, Kupffer cells, liver sinusoidal epithelial cells (LSEC) and dendritic cells, using the TfRscFv as a targeting moiety.

### Cationic Liposome Complexes for Vaccination

In one embodiment, the present invention provides methods for inducing an immune response against a viral antigen in a mammal (as used herein, mammals include dogs, cats, pigs, sheep, horses, cattle, rats, mice, chimpanzees, monkeys, apes, etc., as well as humans) art the primary site of viral replication, e.g the liver with Hepatitis C virus (HCV), and specifically antigen presenting cells (APC), using cationic liposome complexes. As one example, in HCV, the T cell response has been determined to be more rapid and more effective at the viral replication site (liver) upon infection. The present invention therefore provides a method to mimic the T cell priming that takes place in HCV recovered mammals by inducing an immune response (e.g., a prophylactic vaccine) that targets cells located in the liver (e.g. Kupffer cells, liver sinusoidal epithelial cells, dendritic cells and hepatocytes) in vivo through cationic liposomes to deliver recombinant plasmid DNA, and viral-vectored immunization. The present invention also provides vaccines for use as immunotherapeutics for the prevention and treatment of chronic (and acute) pathogenic infections. The ability to deliver the various ligand-targeted cationic liposome complexes to normal cells (i.e., not tumor cells) of the body is an unexpected and surprising result. It should be understood that while exemplary methods of the present invention are used to treat mammals (e.g., humans), the methods, liposome formulations and compositions of the present invention can also be utilized to treat other animals, including birds, fish, etc.

The cationic immunoliposome complexes of the present invention suitably comprise an anti-transferrin receptor single chain antibody molecule (TfRscFv) on their surface. It has been determined that this targeting molecule enhances delivery and uptake by APCs of encapsulated/associated nucleic acid (e.g., plasmids DNA) that expresses viral proteins. *See e.g.,* U.S. Published Patent Application No. 2003/0044407 and U.S. Patent Application No. 11/520,796. In addition, the present invention provides for simultaneous administration of nucleic acids expressing interleukin (IL) adjuvants in the same immunoliposome complexes to stimulate the immune response.

### Cytokine enhancement of immune responses

Cytokines are small proteins (∼25kDa) that are released by cells in the body in response to an activating stimulus. They induce responses in autocrine and paracrine manners by binding to specific receptors to regulate the innate and adaptive immune responses. They have been widely used to improve the activity of vaccines through co-administration of either the purified protein (Berzofsky J.A., et al., Immunological Reviews, 170:151-172 (1999)) or DNA plasmids expressing the desired cytokine product (Toka F.N., et al., Immunological Reviews, 199:100-112 (2004)). Most investigations regarding the genetic delivery of these adjuvants have focused on the intramuscular or intradermal routes of administration, intravenous delivery has not been extensively assessed (*see Toka*). CD4 cells are divided into two major types based on their cytokine gene transcription and secretion. Tb1 cells are characterized by their secretion of IL-2 and gamma interferon (IFN-γ), whereas Th2 cells produce IL-4, IL-5, and IL-10. Adjuvants may induce a T-cell response skewed in either the Th1 or the Th2 direction, which may have a profound influence on the outcome of a vaccine. To improve immunization and stimulate an efficient Thl-type response, nucleic acids, e.g., plasmids, expressing IL-2, IL-12 or IL-15 are suitably associated/encapsulated in the cationic liposomes as described throughout.

IL-2: The majority of IL-2 (a 15.5-kDa glycoprotein) is produced by CD4 Th1 type cells and its major action is to stimulate, helper and cytotoxic T cell (CTL) growth, natural killer (NK) cells, lymphokine-activated killer cells, monocytes, and macrophages. IL-2 is known to play an important role in the initiation and maintenance of antigen-specific immune responses and has been shown to enhance immune responses induced by several DNA vaccines. The mechanism appears to be upregulation in the expression of CD48 and CD80 in DCs as well as upregulation of their respective ligands, CD2 and CD28 on CD8+ T cells (*see Toka*).

IL-12: Human IL-12 is a heterodimer composed of two subunits p35 and p40. It is a proinflammatory cytokine that is produced by phagocytes and DCs in response to pathogen infection and induces IFN-γ production. This cytokine favors differentiation of Th1 over Th2 cells and links innate resistance with adaptive immunity. In mice immunized with a DNA-based HCV vaccine, delivery of IL-12 DNA improved CTL responses and clearance of core-rVV.

IL-15: IL-15 is a 14kDa glycoprotein that acts in a pleiotropic and multifunctional manner. It is expressed by a wide variety of cells including monocytes, macrophages, DCs, Kupffer cells, hepatocytes and endothelial cells. It has many biological activities in common with IL-2 as the two cytokines use the same receptor components. IL-15 can enhance cytolytic activity of NK cells and CTL and induce IFN-γ production from NK and T cells. In addition it induces proliferation of CD8+ memory and effector T cells, making it an attractive candidate for inclusion as an adjuvant when inducing antiviral CD8+ memory T cell responses.

In exemplary embodiments, cationic liposomes comprise the single - chain transferrin receptor antibody, with or without the HK peptide (scL or scLHK) and one or more plasmids expressing the NS3, NS4A, NS4B, NS5A and NS5B (NS3-NS5B)portions of the HCV genome (scL-HCV or scLHK-HCV). It should be noted however, as described throughout, that any nucleic acid molecule which expresses any viral protein(s), car< be encapsulated/associated with the liposomes of the present invention.

Results in vaccinated naive chimpanzees indicate that the systemic scL-HCV approach, including plasmid DNA expressing IL-12 as an adjuvant, and boosted with a recombinant adenovirus, can trigger an HCV-specific immune response. HCV replication was dramatically suppressed after challenge and was associated with HCV-specific T cell responses. In a mouse cellular immunity experiment to assess T cell response "challenge" with recombinant vaccinia virus expressing HCV NS3 protein post inoculation also showed inhibition of viral replication in the scL-HCV/IL group, these results support the use of this approach for a prophylactic vaccination to induce and immune response against a viral antigen.

In an exemplary embodiment, the present invention provides methods of preparing a ligand-targeted (e.g., protein/peptide, antibody- or antibody fragment-targeted) cationic liposome complex. In suitable embodiments, such methods comprise preparing a ligand (e.g., protein/peptide, antibody or antibody fragment), and mixing the ligand with a cationic liposome to form a ligand-targeted cationic liposome, wherein the ligand is directly associated/complexed with, but is not chemically conjugated to, the cationic liposome. The ligand-targeted cationic liposome is then mixed with one or more nucleic acid molecules encoding one or more viral proteins, and one or more nucleic acid molecules encoding one or more interleukins, to form the ligand-targeted cationic liposome complex. In another exemplary embodiment, the present invention provides methods of preparing a ligand-targeted (e.g., protein/peptide, antibody- or antibody fragment-targeted) cationic liposome complex. In suitable embodiments, such methods comprise preparing a ligand (e.g., protein/peptide, antibody or antibody fragment), and mixing the ligand- with a cationic liposome to form a ligand-targeted cationic liposome, wherein the ligand is directly complexed with the cationic liposome via chemical conjugation. The ligand-targeted cationic liposome is then mixed with one or more nucleic acid molecules encoding one or more viral proteins, and/or one or more nucleic acid molecules encoding one or more interleukins, to form the ligand-targeted cationic liposome complex.

As described herein, nucleic acid molecules are suitably encapsulated, contained or complexed/associated with the liposome complexes of the present invention by simply mixing the one or more nucleic acid molecules with the liposomes during processing. Suitable ratios of nucleic acid molecules:liposome complexes are readily determined by the ordinarily skilled artisan. In exemplary embodiments, the nucleic acids are present at a molar ratio of about 1 mole of one or more nucleic acid molecules encoding one or more viral proteins, to about 1 mole of one or more nucleic acid molecules encoding one or more interleukins. For example, the nucleic acid molecules encoding one or more viral proteins are mixed together with the nucleic acid molecules encoding one or more interleukins at a molar ratio in the range of about 0.1:10 to about 10:0.1, suitably about 0.5:1 to about 2:1, and more suitably about 1:1 to form a nucleic acid solution, for example, prior to complexing with the liposomes, though additional molar ratios outside of these ranges can also be used. In other embodiments, the nucleic acid molecules can be complexed separately to the liposomes (i.e., in different solutions), while still maintaining the desired molar ratios.

Suitably, the molar ratio of nucleic acid molecules to liposome complex is in the range of about 0.5:1 to about 1:40 (µg total nucleic acid:µg liposome), suitably about 1:5 to about 1:20 (µg total nucleic acid:µg liposome), more suitably about 1:1:0 (µg total nucleic acid:µg liposome). As utilized herein, the molar ratio of nucleic acid molecules to liposome complex includes both "populations" of nucleic acids, i.e., the total amount of nucleic acid includes one or more nucleic acid molecules encoding one or more viral proteins, and one or more nucleic acid molecules encoding one or more interleukins.

As described throughout, examples of desirable cationic liposomes for delivery of nucleic acid molecules include those that comprise a mixture of dioleoyltrimethylammonium phosphate (DOTAP) and dioleoylphosphatidylethanolamine (DOPE) and/or cholesterol. (chol); and a mixture of dimethyldioctadecylammonium bromide (DDAB) and DOPE and/or chol. The ratio of the lipids can be varied to optimize the efficiency of uptake of the nucleic acid molecules for the specific target cell type. The liposome can comprise a mixture of one or more cationic lipids and one or more neutral or helper lipids. A desirable ratio of cationic lipid(s) to neutral or helper lipid(s) is about 1:(0.5-3), preferably about 1:(1-2) (molar ratio). Examples of ratios of various lipids useful in the practice of the present invention include, but are not limited to:

| | | |
|---|---|---|
| LipA | DOTAP/DOPE | 1:1 molar ratio |
| LipB | DDAB/DOPE | 1:1 molar ratio |
| LipC | DDAB/DOPE | 1:2 molar ratio |
| LipD | DOTAP/Chol | 1:1 molar ratio |
| LipE | DDAB/Chol | 1:1 molar ratio |
| LipG | DOTAP/DOPE/Chol | 2:1:1 molar ratio |
| LipH | DDAB/DOPE/Chol | 2:1:1 molar ratio |

| | | |
|---|---|---|
| (DOTAP = dioleoyltrimethylaminnonium phosphate, DDAB = dimethyldioctadecylammonium bromide; DOPE = dioleoylphosphatidylethanolamine; chol = cholesterol). | | |

As discussed throughout, in suitable embodiments, the ligand used in the compositions and methods of the present invention is an antibody fragment, for example a single chain Fv fragment, such as, an anti-transferrin receptor single chain Fv (TfRscFv). Suitably, the antibody or antibody fragment is mixed with the cationic liposome at a ratio in the range of about 1:1 to about 1:100, suitably about 1:10 to about 1:50 (w:w), more suitably about 1:30, or about 1:33 (antibody or antibody fragment:lipid) to form the targeted cationic immunoliposome. In additional embodiments, the liposomes can also comprise endosomal disrupting peptides, such as the K[K(H)KKK]₅-K(H)KKC (HoKC) (HK) (SEQ ID NO: 1) peptide manufactured by Sigma-Genosys (The Woodlands; TX), associated with the liposomes. The endosomal disrupting peptide HoKC may help the release of agents in the cytoplasm of the cells.

In exemplary embodiments, cationic liposomal formulations A (DOTAP:DOPE at a 1:1 molar ratio), B (DDAB:DOPE at 1:1), G (DOTAP:DOPE:cholesterol at 1:1:1) and H (DDAB:DOPE:cholesterol at 1:1:1) or any of the lipid formulations described throughout, are prepared using the ethanol injection method as described herein. Each liposome, formulation also suitably includes MPB-DOPE at 5 molar percent of total lipid Since the HoKC peptide (K[K(H)KKK]₅-K(H)KKC) carries a terminal cysteine, MPB-DOPE was included in all of the liposome compositions to allow conjugation of peptide to the liposome. The Lip-HoKC liposomes were prepared using the coupling reaction between the cationic liposomes carrying the maleimide group (Lip-MPB) and the peptide. An aliquot of 0.1 mmol of the peptide with a free thiol group on cysteine was added to 2 mmol of Lip-MPB in 10 mM HEPES, pH 7.4, solution and rotated at room temperature (20-30 r.p.m.) for 2 h. The resulting Lip-HoKC has a lipid concentration of 1.4 mM.

The full complex is formed in a manner identical to that used to produce the TfRscFv:Lip:DNA complex without HoKC. Here also the anti-transferrin receptor single chain antibody fragment (TfRscFv) is mixed with Lip-HoKC by gentle inversion at a specific ratio, and incubated at room temperature for 10 min. DNA is then added to the TfRscFv:Lip-HoKC solution, mixed by gentle inversion and again incubated at room temperature for 15 min, after which dextrose or sucrose is added to a final concentration of 5-10% and again mixed by gentle inversion. A suitable ratio for the TfRscFv:Lip-HoKC:DNA complex is 0.3 mg:7 nmol:l mg.

Ln additional embodiments, the methods of the present invention comprise preparing a ligand (e.g., protein/peptide, antibody or antibody fragment), and chemically conjugating the ligand to the surface of a cationic liposome to form a cationic immunoliposome. The cationic liposome is then mixed with one or more nucleic acid molecules encoding one or more viral proteins, and one or more nucleic acid molecules encoding one or more interleukins, to form the ligand-targeted liposome complex. Exemplary methods, composition, ratios and conditions for chemically conjugating ligands (e.g., proteins/peptides, antibodies or antibody fragments) to cationic liposomes are disclosed in U.S. Patent Application No. 09/914,046, filed October 1,2001.

Exemplary nucleic acid molecules for use in the practice of the present invention include DNA and RNA, including plasmids and vectors. The nucleic acid molecules encoding one or more viral proteins can encode any viral protein. As used herein the terms "protein" "peptide" and "polypeptide" are used interchangeably to mean any chain or chains of two or more amino acids, and does not refer to a specific length of product. Thus, peptides, dipeptides, tripeptides, oligopeptides, "protein," "amino acid chain," or any other term used to refer to a chain or chains of two or more amino acids, are included within the definition of the terms "protein," "polypeptide," and "peptide."

Examples of viral peptides/proteins that can be encoded by the nucleic acids delivered using the present invention include viral pathogens (e.g., influenza, HIV, Ebola, poxviruses (smallpox), West Nile Virus, SARS, etc.), and the like. Additional examples of viral proteins that can be encoded by the nucleic acids utilized in the present invention include, but are not limited to, adenovirus polypeptides, alphavirus polypeptides, calicivirus polypeptides, e.g., a calicivirus capsid antigen, coronavirus polypeptides, distemper virus polypeptides, Ebola virus polypeptides, enterovirus polypeptides, flavivirus polypeptides, e.g., West Nile virus E-glycoprotein, hepatitis virus (AE) polypeptides, e.g., a hepatitis A, hepatitis B and/or hepatitis C, herpesvirus polypeptides, e.g., a herpes simplex virus or varicella zoster virus glycoprotein, immunodeficiency virus polypeptides, e.g., the human immunodeficiency virus envelope or protease, infectious peritonitis virus polypeptides, influenza virus polypeptides, e.g., influenza A polypeptides such as hemagglutinin or extracellular M2 protein (M2e), neuraminidase, or nucleoprotein, leukemia virus polypeptides, Marburg virus polypeptides, orthomyxovirus polypeptides, papilloma virus polypeptides, lassa fever polypeptides, parainfluenza virus polypeptides, e.g., the hemagglutinin/neuraminidase, paramyxovirus polypeptides, parvovirus polypeptides, pestivirus polypeptides, picorna virus polypeptides, e.g., a poliovirus capsid polypeptide, pox virus polypeptides, e.g., a vaccinia virus polypeptide (e.g. small pox), rabies virus polypeptides, e.g., a rabies virus glycoprotein G, reovirus polypeptides, retrovirus polypeptides, and rotavirus polypeptides. Nucleic acid molecules encoding such viral proteins are available in the art, including vectors and plasmids comprising such nucleic acid molecules. For example, public databases such as GENBANK^{®} contain various examples of nucleic acid molecules that can be utilized in the practice of the present invention:

Nucleic acid molecules encoding one or more interleukins are also well known in the art and readily available from public databases such as GENBANK^{®}. Exemplary interleukins which can be encoded by the nucleic acid molecules useful the practice of the present invention include, but are not limited to, IL-1, IL-2, IL-3, IL-4, IL-5, IL-6, EL-7, IL-8, IL-9, IL-10, IL-12, IL-13, IL-15, IL-17, IL-18 and IL-23. In suitable embodiments, the nucleic acids encode IL-2, II,-12 or IL-15.

The present invention also provides cationic immunoliposome complexes prepared according to the methods described throughout. For example, the present invention provides ligand-targeted (e.g., protein/peptide, antibody- or antibody fragment-targeted) cationic liposome complexes comprising a cationic liposome, a ligand (e.g., protein/peptide, antibody or antibody fragment), one or more nucleic acid molecules encoding one or more viral proteins, and one or more nucleic acid molecules encoding one or more interleukins, wherein the ligand is directly complexed/associated with, but not chemically conjugated to the cationic liposome. The ligand (e.g., protein/peptide; antibody or antibody fragment) is suitably associated with the liposome via an interaction (e.g., electrostatic, van der Walls, or other non-chemically conjugated interaction) between the ligand and the liposome. In general, a linker or spacer molecule (e.g., a polymer or other molecule) is not used to attach the ligands and the liposome when noun-chemically conjugated.

As described herein, in additional embodiments, the ligand (e.g., protein/peptide, antibody or antibody fragment) is chemically conjugated to the cationic liposomes, for example, via a chemical interaction between the cationic liposomes which contains a maleimidyl group or other sulfhydryl-reacting group, and a sulfur atom on the ligand (e.g., protein/peptide, antibody or antibody fragment). The nucleic acids are then added to the liposome to form the liposome-DNA complexes, or the nucleic acids can be added first, and then complexed with the ligands. Such methods are disclosed in U.S. Patent Application No. 09/914,046, filed October 1.

Transerrin (Tf)-conjugated liposomes comprising nucleic acids (Tf- Lip-DNA) are prepared according to the formulation described previously (Xu, et al., "Transferrin-liposome-mediated systemic p53 gene therapy in combination with radiation results in regression of human head and neck cancer xenografts," Hum Gene Ther. 10(18):2941-952. (1999)), the disclosure of which is incorporated by reference herein in its entirety. Transferrin-liposome-mediated systemic p53 gene therapy in combination with radiation results in regression of human head and neck cancer xenog rafts. Briefly, for a suitable preparation, 25 ml of Tf (5 mg/ml, iron-saturated holo-transferrirl; Sigma, St. Louis, MO) and 50 ml of Lip (2 mM total lipids) plus 75 ml of water are mixed in a polypropylene tube and incubated for 5-15 min at room temperature with frequent rocking. Ten micrograms of plasmid DNA in 120 ml of 20 mM HEPES buffer, pH 7.4, were added to the tube, mixed immediately and thoroughly, and incubated for 20 min at room temperature with frequent rockling. Thirty microliters of 50% dextrose solution is then added to the tube. The final DNA:lipid:Tf ratio is about 1 :10:12.5 (mg/nmol/mg). In additional embodiments, the HEPES buffer can be replaced by water.

The nucleic acid molecules can be encapsulated within the cationic liposome, contained within a hydrocarbon chain region of the cationic liposome, associated with an inner or outer monolayer of the cationic liposome (e.g., the head-group region), or any combination thereof. Suitably, the cationic immunoliposomes of the present invention are unilamellar liposomes (i.e. a single bilayer), though multilamellar liposomes which comprise several concentric bilayers can also be used. Single bilayer cationic liposomes of the present invention comprise an interior aqueous volume in which nucleic acid molecules can be encapsulated. They also comprise a single bilayer which has a hydrocarbon chain region (i.e., the lipid chain region of the lipids) in which nucleic acid molecules that have been conditioned to be neutral or largely uncharged can be contained. In addition, nucleic acid molecules can be complexed or associated with either, or both, the inner monolayer and/or the outer monolayer of the liposome membrane (i.e., the head-group region of the lipids), e.g., via a charge-charge interaction between the negatively charged nucleic acid molecules and the positively charged cationic liposomes. In further embodiments, nucleic acid molecules can be encapsulated/associated/complexed in any or all of these regions of the cationic liposome complexes of the present invention.

As discussed throughout, suitably the nucleic acid molecules are present at a molar ratio of about 0.1 mole of one or more nucleic acid molecules encoding one or more viral proteins, to about 10 mole of one or more nucleic acid molecules encoding one or more interleukins; to 10 mole of one or more nucleic acid molecules encoding one or more viral proteins, to about 0.1 mole of one or more nucleic acid molecules encoding one or more interleukins; in particular, about 1 mole of one or more nucleic acid molecules encoding one or more viral proteins, to about 1 mole of one or more nucleic acid molecules encoding one or more interleukins can be used in the liposome complexes. Suitable amounts/ratios of lipids, targeting ligands and nucleic acid molecules are also described throughout. It exemplary embodiments, the one or more nucleic acid molecules encoding one or more viral proteins, the one or more nucleic acid molecules encoding one or more interleukins, and the liposomes, are present at a weight ratio of between about 0.5:1 to about 1:40(µg total nucleic acid:µg lipid), suitably about 1:5 to about 1:20(µg total nucleic acid:µg lipid), e.g., about 1:10(µg total nucleic acid:µg lipid). An exemplary liposome composition of the present invention comprises total nucleic acid, lipid and a ligand, such as a single chain antibody (e.g., TfscFv) at a weight ratio of about 1:10:0.33 (µg total nucleic acid:µg lipid:µg single chain antibody).

Exemplary viral proteins which can be encoded by the nucleic acid molecules utilized in the cationic liposomes of the present invention include those described throughout, for example, HIV viral proteins, influenza viral proteins, SARS viral proteins, hepatitis A, Band/or C viral proteins, bird flu viral proteins and small pox viral proteins, as well as others. Exemplary interleukins which can be encoded by the nucleic acid molecules utilized in the cationic liposomes of the present invention include those described throughout, including IL-1, IL-2, IL-3, IL-4, IL-5, IL-6, IL-7, IL-8, IL-9, IL-10, IL-12, IL-13, IL-15, IL-17, IL-18 and IL-23, and suitably, IL-2, IL-12 and IL-15.

In exemplary embodiments, the nucleic acids encoding the viral proteins and/or the interleukins for use in the practice of the present invention are placed downstream from a nucleic acid promoter, for example, a promoter as described in U.S. Published patent application No. 2007/0065432.

The present invention also provides pharmaceutical compositions comprising the ligand-targeted cationic liposome complexes described throughout. In suitable embodiments, the pharmaceutical compositions further comprise one or more excipients selected from the group consisting of one or more antibacterials (e.g., amphotericin B, chloretracycline, gentamicin, neomycin), one or more preservatives (e.g., benzethonium chloride, EDTA, formaldehyde, 2-phenoxyethanol), one or more buffers (e.g., phosphate buffers, sodium borate, sodium chloride), one or more surfactants (polysorbate 20, 80), one or more protein stabilizers (e.g., albumin, lactose, potassium glutamate), sugars e.g. sucrose or dextrose, and adjuvants (e.g., aluminum hydroxide, aluminum phosphate). Additional excipients are well known in the art and can be readily used in the practice of the present invention.

The present invention also provides pharmaceutical compositions comprising a first ligand-targeted cationic liposome complex comprising a cationic liposome, a ligand (e.g., protein/peptide, antibody or antibody fragment), and one or more nucleic acid molecules encoding one or more viral proteins, wherein the ligand directly complexed/associated with, but not chemically conjugated to the cationic liposome. In further embodiments, the ligand can be chemically conjugated to the cationic liposome. The pharmaceutical compositions also suitably comprise a second ligand-targeted cationic liposome complex comprising a cationic liposome, a ligand (e:g., protein/peptide, antibody or antibody fragment), and one or more nucleic acid molecules encoding one or more interleukins, wherein the ligand is directly complexed/associated with, but not chemically conjugated to the cationic liposome. In further embodiments, the ligand can be chemically conjugated to the cationic liposome.

In certain such embodiments, the nucleic acid molecules encoding one or more viral proteins are delivered in the same composition as the nucleic acids encoded one or more interleukins, but rather than being encapsulated/associated with the same cationic liposome, they are associated with two (or more) different cationic liposomes and then delivered in the same composition (i.e., two different liposome populations mixed together). In other embodiments the nucleic acid molecules encoding one or more viral proteins and encoding one or more interleukins can be contained together within the same plasmid vector. The present invention also encompasses administering to a mammal two separate immunoliposome compositions, one which comprises nucleic acids encoding viral proteins, and one which comprises nucleic acids encoding interleukins. Examples of suitable lipids, targeting molecules and nucleic acid molecules, and ratios of such components for use the pharmaceutical compositions of the present invention are described throughout.

The present invention also provides methods of delivering one or more active agents to antigen presenting cells (APCs) of a mammal. In suitable embodiments, such methods comprise administering to the mammal an anti-transfenrin receptor single chain Fv (TfRscFv)-targeted cationic immunoliposome complex comprising a cationic liposome, a TfRscFv, and one or more active agents, wherein the TfRscFv is directly complexed/associated with, but not chemically conjugated to the cationic liposome. In further embodiments, the TfRscFv is chemically conjugated to the cationic liposome. Such methods of the present invention can be used to deliver agents to APCs in any organ or tissue of the body, for example the liver, the lymph nodes, the gut and/or the respiratory tract. Suitably, the APCs can be, for example, professional, or,non-professional APCs. "Professional APCs" refers to those that express MHC class II molecules on their surface.

In suitable embodiments, the agents that are delivered are nucleic acid molecules, though other agents, such as chemical agents, including small molecules, as well as peptides and proteins can also be delivered. Agents can be delivered to professional as well as non-professional APCs, including APCs located in the liver, spleen, lymph nodes, gut and/or respiratory tract. In exemplary embodiments, the agents that are delivered encode one or more viral proteins (for example HIV viral proteins, Ebola viral proteins, influenza viral proteins, SARS viral proteins, bird flu viral proteins or small pox viral proteins) and/or one or more interleukins (for example IL-1, IL-2, IL-3, IL-4, IL-5, IL-6, IL-7, IL-8, IL-9, IL- 10, IL- 12, IL-13,IL-15, IL-17, IL-18 or IL-23).

In further embodiments, the present invention provides methods of inducing an immune response against a viral antigen in a mammal, comprising administering to the mammal a ligand-targeted cationic liposome complex. In suitable embodiments, the cationic liposome complex comprises a cationic liposome; a ligand directly complexed/associated with, but not chemically conjugated to, the cationic liposome; one or more nucleic acid molecules encoding one or more viral proteins associated with the cationic liposome; and one or more nucleic acid molecules encoding one or more interleukins associated with the cationic liposome. In further embodiments, the ligand is chemically conjugated to the cationic liposome, for example, through a chemical conjugation between a sulfur group on the ligand (e.g., on an antibody or antibody fragment) and a group on the liposomes such as a maleimide group. Exemplary ligands are described throughout, and include, but are not limited to, galactose, L-37pA, an antibody and an antibody fragment (e.g., an scFv antibody fragment such as an anti-transferrin receptor single chain Fv (TfRscFv)).

As used herein, "inducing an immune response against a viral antigen" refers to the process by which a mammal's (e.g., any mammal such as a human) immune system is triggered to identify and remove or otherwise respond to the presence of a viral antigen. Antigens include any viral substance that causes the production of antibodies against it. By inducing an immune response to a particular viral antigen or antigens, the mammal is thereby vaccinated and any further compromise by the antigen will result in the response/clearance of the antigen.

Examples of viral antigens to which an immune response can be generated include any viral antigen known in the art, including those described throughout, such as an antigen associated with hepatitis (A, B or C) virus, influenza virus, HIV virus, Ebola virus, SARS virus, bird flu virus, small pox virus, etc. The ability of the cationic liposome to target APCs enables delivery of nucleic acid molecules directly and efficiently to the cells involved in priming an immune response, thereby providing a very efficient and powerful method of inducing and immune response against a viral antigen in a mammal.

Suitable viral proteins which can be expressed by the nucleic acid molecules utilized in the methods of treatment of the present invention are described throughout, and include, but are not limited to, HIV viral proteins, Ebola viral proteins, influenza viral proteins, SARS viral proteins, bird flu viral proteins and small pox viral proteins. Exemplary interleukins which can be encoded by the nucleic acid molecules include those described throughout, such as IL-1, IL,-2, IL-3, IL-4, IL-5, IL-6, IL-7, IL-8, IL-9, IL-10, IL-12, IL-13, IL-15, IL-17, IL-18 and IL-23. In exemplary embodiments, the ligand-targeted cationic liposomes delivered in the various methods of the present invention further comprise a peptide comprising a K[K(H)KKK]**₅**-K(H)KKC (HoKC) (SEQ ID NO: 1) peptide associated with the cationic liposomes.

In additional embodiments, the present invention provides methods of treating or preventing a viral disease in a mammal. Suitably, the methods comprise administering to the mammal a cationic liposome complex, wherein the cationic liposome complex comprises: a cationic liposome; a ligand directly complexed with, but not chemically conjugated to, the cationic liposome; one or more nucleic acid molecules encoding one or more viral proteins associated with the cationic liposome; and one or more nucleic acid molecules encoding one or more interleukins associated with the cationic liposome Methods of administration include, but are not limited to, intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal! (IN), intracereberal (IC), intraorgan (e.g. intrahepatic), slow release implant, for subcutaneous administration, or via administration using an osmotic or mechanical pump. They can be administered as a bolus or as an infusion. In additional embodiments, the ligand can be chemically conjugated to the cationic liposome using the various methods described herein or otherwise known in the art.

Examples of viral diseases that can be treated or prevented using the methods of the present invention include any viral disease known in the art, including, but not limited to, those described throughout, such as hepatitis (A, B or C), influenza, HIV, Ebola, SARS, bird flu, small pox, etc. Thus, in addition to inducing an immune response to a viral antigen in a mammal, the present invention also provides methods of treating or preventing viral diseases in a mammal. This includes mammals that have been infected within a recent time period (e.g., less than about 1 year, suitably less than about 6 months or less than about 1 month), as well as mammals with chronic infections (e.g., mammals who have been infected for example 1 year or more).

Exemplary ligands for use in the methods of treatment or prevention of the present invention are described throughout, and includes, various peptides and proteins, such as transferrin, galactose, L-37pA, an antibody and an antibody fragment (.eg., a single chain Fv antibody fragment such as an anti-transferrin receptor single chain Fv (TfRscFv)). Exemplary viral proteins and interleukins that can be encoded by the nucleic acid molecules delivered using the various methods of treatment and prevention of the present invention are described throughout. In exemplary embodiments, the ligands-targeted cationic liposomes delivered in the various treatment and prevention methods of the present invention further comprise a peptide comprising a K[K(H)KKK]₅-K(H)KKC (HoKC) (SEQ ID NO: 1) peptide associated with the cationic liposomes.

The present invention also provides methods of inducing an immune response against a viral antigen in a mammal comprising administering to the mammal ligand-targeted cationic liposome complexes prepared by the various methods described throughout. In addition, methods of treating or preventing a viral disease in a mammal comprising administering to the mammal ligand-targeted cationic liposome complexes prepared by the various methods/processes of the present invention. Suitable ligands, liposomes and nucleic acids for use in these methods are described throughout.

In the various treatment/prevention methods of the present invention, including inducing an immune response and treating or preventing a viral disease, as well delivery to APCs, the various liposome complexes of the present invention can be administered via any desired route. Exemplary delivery routes include, but are not limited to, intravenous, oral, topical, via inhalation, intramuscular injection, intratumoral injection, intradermal injection, transdermal injection, subcutaneous injection, intraperitoneal injection, intranasal injection, intraocular injection or drops, intracranial injection, intraorgan administration (e.g. intrahepatic) or other routes, such as slow release implant or via an osmotic or mechanical pump. They can be administered as a bolus or as an infusion.

The present invention also provides pharmaceutical compositions comprising the various ligand-targeted cationic liposome complexes of the present invention. The pharmaceutical compositions of the present invention may also further comprise one or more excipients, for example, one or more antibacterials, one or more preservatives, one or more buffers and/or one or more surfactants.

In additional embodiments, the present invention also provides pharmaceutical composition two or more ligand-targeted cationic liposome complexes. For example, the compositions can comprise a first ligand-targeted cationic liposome complex comprising a cationic liposome, a ligand, and one or more nucleic acid molecules encoding one or more viral proteins. In suitable embodiments, the ligand is directly complexed with, but is not chemically conjugated to, the cationic liposome. In other embodiments, the ligand is chemically conjugated to the cationic liposome.

The compositions can further comprise a second ligand-targeted cationic liposome complex comprising a cationic liposome, a ligand, and one or more nucleic acid molecules encoding one or more interleukins. Suitably, the ligand is directly complexed with, but is not chemically conjugated to, said cationic liposome, while in other embodiments, the ligand is chemically conjugated to the cationic liposome.

While the Examples set forth below describe the production of immunoliposome complexes for delivery of hepatitis C viral (HCV) proteins, and the prophylactic vaccination/treatment of HCV infected mammals, it should be understood that nucleic acid molecules encoding any viral protein can utilized in the various methods of the present invention and associated/encapsulated in the immunoliposome complexes described throughout. The final viral protein that is encoded by the nucleic acid molecules has no impact on the ability to form the immunoliposome complexes of the present invention. Simply the negative charge of the nucleic acid molecule appears to allow nucleic acid molecules to associate/encapsulate in the cationic immunoliposomes. Therefore, the present invention is not limited to the delivery of HCV proteins, and instead, encompasses the delivery of any nucleic acid (e.g., plasmid, vector) encoding any viral protein and/or any interleukin(s).

In addition, several different nucleic acids encoding different viral proteins can be encapsulated/associated with the immunoliposome complexes, and as such, the present invention therefore provides complexes and methods for vaccinating/treating a wide variety of viral infections, either with a single liposome system, or using multiple, different liposome complexes.

It will be readily apparent to one of ordinary skill in the relevant arts that other suitable modifications and adaptations to the methods and applications described herein may be made without departing from the scope of the invention or any embodiment thereof. Having now described the present invention in detail, the same will be more clearly understood by reference to the following examples, which are included herewith for purposes of illustration only and are not intended to be limiting of the invention.

### Example 1

### Preparation and Use of Cationic Liposome-Based Hepatitis C Virus (HCV) Vaccination and Treatment Systems

In contrast to the previous dogma that protection from HCV reinjection is not induced by natural infection in chimpanzees (Farci P., et al., Science 258: 140 (1992); Prince A.M., et al., J. Infect. Dis., 165: 438-443 (1992)), it is now apparent that although re-infection does occur, there are memory immune responses in recovered humans and chimpanzees that lead to immediate conttol of viral replication and rapid clearance upon re-infection. After spontaneous recovery from HCV, resting HCV-specific memory T cells can be detected in the peripheral blood for decades, in many cases in the absence of detectable HCV-antibodies (Takaki A., et al., Nat. Med., 6:578-582 (2000)). HCV-specific cellular immune responses also persist in the liver, as demonstrated in recovered chimpanzees, and mediate protective immunity on re-infection {see, e.g., Bassett S.E., et al, Hepatology, 55:479-1487 (2001)). The second infection is of short duration with a reduced viral titer in the blood and liver in comparison to the initial infection and reduced hepatic inflammation as indicated by alanine amino transferase levels (ALT) (Bassset et al). There are indications from patient studies that similar memory responses are also present in humans following previous exposure that can control and clear HCV infections rapidly (Mehta S.H., et al, Lancet, 359: 1478-1483 (2002)). As described throughout, the present invention provides a T cell-based vaccine, focusing on the non-structural proteins of HCV, efficiently delivered to APCs, including but not limited to those in the liver for both prophylaxis and therapy. The liver is the primary site of HCV replication, and hence, induction of an immune response in the liver mimics the immune responses induced during natural infection and leads to protection or rapid clearance of the virus upon challenge.

Hepatitis C virus (HCV) is an enveloped virus with a single stranded, positive sense RNA genome. The RNA is -9.6 kb in length and consists of a -341 -base 5' non-translated region (NTR), a long open reading frame encoding all virus-specific proteins (-3011 amino acids), and a 3' NTR. Translation of the genome RNA is cap-independent and mediated by the 5'NTR which functions as an interval ribosome entry site. The resulting polyprotein is cleaved, both co- and post-translationally by two host and two viral proteases, to produce the following products: NH2-C-E1-E2-p7-NS2-NS3-NS4A-NS4B-NS5A-NS5B-COOH. The structural proteins (C, E1, and E2) are located at the N-terminus; the nonstructural (NS) components are encoded in the remainder (for review see (Major M. E., et al., Hepatitis C Viruses. In: Fields Virology, Knipe D. M. and Howley P.M. (eds.), Lippincott, Williams & Wilkins, pp. 1127-1161 (2001). As for all RNA viruses, HCV RNA replication is error prone, leading to the continuous generation of variants. Such variants provide a pool or "quasispecies" which can quickly adapt to selection pressure, such as that exerted by the immune response. This variability is likely to be important for HCV survival and evolution, but it also renders precise studies on HCV molecular biology, pathogenesis, immune response and ultimately vaccine development, difficult.

Transmission of HCV is typically by the parenteral route, but per mucosal transmission such as sexual and maternal infant transmission do occur. Significant progress has been made in the therapy of chronic hepatitis C. Current therapies with pegylated IFN-α and Ribavirin clear HCV infection in approximately 50% of cases, but treatment remains very expensive, requires 6-12 months of therapy, and carries a significant risk of serious side effects. Prophylactic vaccines are important for the eventual control of HCV-related disease both in the U.S and worldwide. An immunologic approach to treatment is also needed.

Despite the development of a SCID mouse model with chimeric human livers, the chimpanzee remains the only established animal model for HCV infection (reviewed in Grakoui A., et al, Hepatology, 33:489-495 (2001)). As such, it is the only model in which experimental vaccines can be administered and their effectiveness evaluated Many of the proteins involved in the chimpanzee immune system are either identical to the human orthologue or very closely related, such that the reagents needed for detection are interchangeable. Due to the extreme cost it is not possible to test all promising HCV vaccine approaches in chimpanzees. Vaccine studies to date have used normal immune competent mice to test a number of immunization strategies (*see, e.g*., Forns X:., :et al., Vaccine, 17:1992-2002 (1999)). Transgenic mice expressing human class I MHC molecules have been used for specific HCV epitope analysis and surrogate protection systems (Qiao M., et al., Hepatology, 37:52-59 (2003)). Vaccine strategies have thus far been based on recombinant proteins, viral vectors, virus-like particles and DNA-based immunization. There is strong support for the involvement of immune responses to HCV NS3 in clearance of acute infection (Diepolder H.M., et al.; J. Virol., 71:6011-6019 (1997)). This antigen is now considered an important component of any HCV T cell vaccine. Reports have indicated that inclusion of NS4A in constructs expressing the NS3 protein result in better expression and improved immune responses in mice (Frelin L., et al., Gene Ther., 10:686-699 (2003)). Broad, multi-specific T cell responses have also been identified as important in clearance of HCV infections, therefore the NS5A and NS5B proteins would be important T cell vaccine components.

HCV is associated with 40-60% of chronic liver disease in the U.S. Of these patients, one third goes on to develop progressive fibrosis and cirrhosis (Liang T.J., et al,. Ann. Intern. Med., 132:296-305 (2000)). Hepatitis C is now the major cause of hepatocellular carcinoma (HCC) in Japan, having displaced hepatitis B and, according to the CDC, there are 8,000-10,000 deaths each year in the US alone due to HCV-associated cirrhosis and cancer. The mechanism of HCV-associated HCC is unknown. It is mainly assumed to be induced by chronic immune-mediated hepatocellular injury and regeneration over many years of infection. The core gene product of HCV has been shown to interact with a number of cellular proteins. It contains nuclear localization signals, has RNA binding activity, and has been shown to perform multiple functions in cells, such as transactivation and suppression (Matsumoto M., et al., J. Virol., 71:1301-1309 (1997)). These studies identifying transactivator or suppressor functions of HCV core have used expression *levels in vitro* that are several orders of magnitude higher than biological levels. However, the expression of such proteins at low levels for several decades may lead to adverse events in infected cells, which may play a role on HCC progression. The best means to fight HCC are by-prevention, earlier detection and better therapies. Thus, by preventing hepatitis C virus infections, HCC can also be prevented.

The primary reservoir for HCV replication is thought to be the liver, consistent with the liver pathology in infected individuals, although the specific cell types supporting HCV replication are still not identifed. The normal liver plays an important role in mediating immune responses, Unlike most organs, there is evidence that presentation of antigens in the liver can result in activation of naïve T cells (Bertolino P., et al., Cell Biol., 80:84-92 (2002)). Upon entering the liver, antigens in the blood first come into contact with the sinusoidal cell populations, the liver sinusoidal epithelial cells (LSEC) and Kupffer cells. The delivery of antigens to the liver results in the local activation of T cells, possibly by these same cells, both of which have been shown to act as antigen-presenting cells. Presentation of antigen in the liver can result in either T-cell priming or T-cell tolerance.

As opposed to use of the cationic immunoliposomes for cancer treatment and tumor targeting (as in U.S. Published Patent Application No. 2003/0044407 and U.S. Patent Application No. 11/520,796), the present example utilizes TfRscFv-Liposome (including with or without HK peptide) complexes to deliver DNA expressing HCV proteins to be efficiently taken up (receptor mediated endocytosis and enhanced endosomal release) and the antigens presented to T cells wherever they occur in the body, including in the liver.

The presence of the TfRscFv provides receptor-mediated endocytosis to move the cationic liposome complex efficiently into the cells, while the HK peptide enhances (though is not required for) endosomal release of the DNA into the cytoplasm.

Comparative studies using several liposome complexes in vitro to determine the most appropriate formulation to use in vivo for hepatocytes have been performed. Two hepatocyte-specific ligands were tested, galactose (GAL) (binds to the asialoglycoprotein receptor) and L-37pA (binds to the scavenger receptor, SR-B1, a putative receptor for HCV). These were compared to unmodified liposome formulations and complexes modified with the single-chain Fv fragment of the transferrin (Tf) receptor (TfRscFv). Three lipid types were used in these analyses: Lipid A (DOTAP:DOPE, 1 : 1 molar ratio); Lipid D (DOTAP:Chol, 1 :1 molar ratio); and Lipid G (DOTAP:DOPE:Choi, 1 : 1 : 1 molar ratio). Three sample preparation methods were compared. Method A: Pre-coating method in which DNA was mixed directly with the ligand-modified liposome. Method B: Post-coating method in which the DNA was mixed with the liposome to form a cationic lipid/DNA core (lipoplex) before conjugation of the ligand to the surface of the lipoplex. Method C: In which the DNA was mixed with the liposome or liposome with a histidylated oligolysine (HoKC) peptide modification to form a lipoplex after which the anionic(PSchol) or neutral (PCchol) liposome carrying the specific ligand was added to encapsulate the lipoplex.

### A. Methods

### 1. Complex Formation

### Complex Formation by Chemical Conjugation

An exemplary method for preparing cationic immunoliposomes comprising antibodies or antibody fragments that are directly chemically conjugated to the surface of the liposomes is disclosed in U.S. Patent Application No. 09/914,046, filed October 1, 2001. For example, 4-(p-maleimidophenyl)butyrate-DOPE (MPB-DOPE) (Avanti Polar Lipids) is included in the seven liposome formulations described above, to a 5-8% molar of total lipids. The MPB-liposomes are prepared by methods well known in the art. For example, the ethanol injection method modified from that described by Campbell MJ.,(Biotechniques 1995 Jun;18(6):1027-32) can be used in the present invention. In brief, all lipids are solubilized in ethanol and mixed, injected into vortexing pure water of 50-60°C with a Hamilton syringe. The solution is vortexed for a further 10-15 min. The final concentration is 1-2mM total lipids. 1M HEPES, pH7.5 (pH70-8.0) is added to a final concentration of 10-20mM. As the maleimide group is not stable in aqueous solution with pH >7, the liposomes should be prepared in water (pH 5-6.5). The pH can be adjusted to 7.0-8.0 before linking to scFv-SH with 1M HEPES buffer, pH7.0-8.0, to facilitate the post-coating reaction.

The scFv-SH is added to the MPB-liposome at a protein/lipid ratio of about 1:1 to about 1:100, suitably, about 1:10 to about 1:50 (w:w), more suitably of about 1:20 to about 1:40. The solution is mixed by gentle rotation (∼20-30 RPM) for 30 min at room temperature, to produce scFv-Lip. The scFv-Lip is used without purification, although it can be purified by Sepharose CL-4B column chromatography. Plasmid DNA is diluted in water and added to the scFv-Lip at a DNA/Lipid ratio of about 0.5:1 to about 1:40 (µg total nucleic acid:µg lipid), suitably about 1:5 to about 1:20 (µg total nucleic acid:µg lipid), e.g., about 1:10 (µg total nucleic acid:µg lipid) to produce the scFv-Lip-DNA complex. The scFv-Lip-DNA is used without purification, although it can be purified by Sepharose CL-4B column chromatography. 80-100% of the scFv can be expected to be conjugated to the liposome.

### Complex Formation by Simple Mixing

Conjugated TfRscFv-immunoliposornes retain their immunologic activity. It has also been established that the cys-TfRscFv can be chemically conjugated to a lipoplex *(see* U.S. Patent Application No. 09/914,046, filed October 1, 2001) and can efficiently transfect human prostate tumor cells *in vitro* and *in vivo.* It is common practice for single chain antibody fragments to be attached to liposomes using various chemical conjugation methods. It has also been determined that a simple mixing of the cys-TiRscFv and the cationic liposome (which does not contain any lipid with a reducible group such as Maleimide DOPE or any reducible group), instead of chemical conjugation, results in formation of an immunologically active complex, wherein the ligand is bound directly to the liposome without the use of a linker or chemical-conjugation molecule, that still efficiently binds to and transfects tumor cells (see e.g., U.S. Published Patent Application No. 2003/0044407, including the compositions and methods disclosed therein).

The cys-TfRscFv-immunoliposome complexes are prepared by mixing the cys-TfRscFv with liposome composition A at defined ratios of single chain protein to liposome and DNA to n moles (or ug) total lipid in the mixed complex in the ranges described throughout. The preparation of the complexes described below was in accordance with the following general procedure. The appropriate amount of 2 mM liposome (A-H described above) is mixed -with any water (e.g., DI water) required to give a desired volume and inverted to mix. To the liposome- water mixture, the appropriate amount of cys-TfRscFv is added to give the desired ratio and mixed by gentle inversion for about 1 second to about 2 minutes. This mixture is kept at room temperature for about 10 minutes to about 20 minutes (inverted gently for 5-10 seconds after approximately 5 minutes). At the same time, the appropriate amount of DNA is mixed by inversion for about 1 second to about 2 minutes with any water required to give a desired volume. Typically, for use in an in vitro assay, it is desirable that the concentration of DNA is in the range of about 0.01 µg to about 2 µg per well; for in vivo use, it is desirable to provide about 5 µg to about 100 µg of DNA per injection. The DNA solution is quickly added to the cys-TfRscFv-liposome solution and the mixture is inverted for about 1 second to about 2 minutes. The final mixture is kept at room temperature for about 10 minutes to about 20 minutes, gently inverting for 5-10 seconds after approximately 5 minutes. For use in vivo 50% dextrose or 50% sucrose is added to a final concentration of 1-20% (V:V), suitably 5-20% (V:V) and mixed by gentle inversion for about 1 second to about 2 minutes. A specific example at a suitable ratio of 1:30 (cys-TfRscFv: liposome, w:w) and 1 : 14 (µg DNA:n mole total Lipid) is as follows. For 40 µg of DNA in a final volume of 800 µl, mix 183 µl water with 280 µl of 2 mM liposome solution. Add 34 µl of cys-TfRscFv (with a concentration of 0.4 µg/ml). Mix 183 µl water with 40 µl of 1 µg/l µl DNA. Add 80 µl of 50% Dextrose as the last step.

The size of the final complex prepared by the methods of this invention is between about 100 and 400 (ran) with a positive zeta potential as determined by dynamic light scattering using a Malvern ZETASIZER^{®} 3000 or a Malvern ZETASIZER^{®} NANO-ZS.
This size is small enough to efficiently pass through the tumor capillary bed and reach tumor cells, and also small enough to efficiently pass through the capillary bed and reach the target APC cells.

### 2. Assess immune responses to HCV-specific antigens

### Target HCV Antigens

The HCV antigens included in the vaccine studies are NS3 through NS5B. These sequences are derived from the genotype 1 a H77 strain. See Major M. E., et al., Hepatitis C Viruses. In: Fields Virology, Knipe D. M. and Howley P.M. (eds.), Lippincott, Williams & Wilkins, pp. 1127-1161 (2001). Enhancement of the T-cell response to this antigen alone in a recovered animal results in a transient, clinically insignificant infection (see below). For plasmids expressing antigens under the control of the CMV promoter, 293-T and Huh7.5 cells have been used. For adenovirus vectors, the AD-293 cell line was used. Expression was determined by immunofluoresence staining and by western blotting with specific antibodies.

### Adenovirus vectors

Human adenovirus serotype 5 recombinants expressing HCV genes using the ADEASY^{®} system are suitably used in the present invention (Stratagene, CA). This vector has been rendered replication defective by the deletion of the E1 and E3 genes. The gene of interest is introduced into the adenovirus backbone through recombination in E.coli. The recombinant plasmid is then transformed into AD-293 cells where deleted viral assembly genes are complemented to produce virus particles. Adenoviruses are also an attractive viral vector for HCV therapy as this virus could efficiently express transgenes in the liver.

### Interleukin-12 (immune enhancement)

Interleukin-12 (IL-12) induces IFN-γ production, favors differentiation of ThI over Th2 cells, and links innate resistance with adaptive immunity. DCs and phagocytes produce IL-12 in response to pathogens during infection. To improve immunization and stimulate an efficient ThI -type response, plasmids expressing IL-12 are included in the cationic immunoliposome formulations. Plasmids expressing both mouse and human IL-12 have been prepared in order to achieve consistency between the mouse and primate studies.

### Mouse Inoculations

Studies involve DNA prime/adenovirus boosting using recombinant vectors expressing HCV antigens. Mice are inoculated with 20-30 µg of DNA in liposome complexes disclosed herein at 0 and 4 weeks and boosted with recombinant adenovirus (10⁸ infectious units, subcutaneously (s.c.)) at 10-12 weeks. Four weeks after this boost, livers and spleens are analyzed by flow cytometry to determine the characteristics of the T cells in the liver. T cells can be separated from the hepatocytes using a Ficoll gradient and stained for specific surface markers using labeled antibodies. As controls, PBS treated mice, unliganded liposome/DNA complexes, complexes carrying empty vector, naked DNA injected intramuscularly (i.m.) (1 to 50 µg/mouse), intraperitoneally (i.p.), intravenously (i.v.), and intradennally (i.d.) (1-50 µg/mouse) and mice inoculated with complex i.m. or i.d. are included. Dosage and timing are optimized and the persistence and recall of T cells in the liver over a 6 month period are assessed to determine the best system based upon intrahepatic T cell numbers and markers. Since transferrin receptors are expressed on dendritic cells and the complexed DNA is taken up by receptor-mediated endocytosis, i.m. and/or i.d. inoculations of the scL-HCV or scLHK-HCV complex are expected to result in a better systemic response than that of naked plasmid DNA. T cell populations can be identified on the basis of the expression of CD45RA, CD62L, CD27 and CCR7. IL-7Rα provides a useful marker that can distinguish effector cells that are short-lived from those that develop into functional memory cells. These markers are used in the primary mouse studies to analyze the potential of candidate vaccines to establish long-term immunity. There is evidence for selective retention of memory CD8+ T cells in the liver after clearance of HCV in chimpanzees with ∼10-fold higher levels in the liver compared to the blood 6 months after clearance.

The specificity of splenic and intrahepatic T cells are assessed by *in vitro* immunoassays. Initially Elispot assays using purified HCV antigens or overlapping 18 mer peptides are used to analyze cells secreting IFN-γ or IL-4. Proliferation assays are also performed using cells isolated from the spleens of immunized mice and whole proteins purchased from Mikrogen (Germany). HLA-A2 transgenic mice are utilized. These mice express the HLA-A2 MHC molecule and can present HLA-A2 epitopes. These transgenic mice can be immunized using the same protocol as that used for the BALB/c mice but peptides recognized by the HLA-A2 haplotype can be used in immunological tests. For these analyses, HLA-A2 tetramers and epitopes identified for NS5A and NS5B specific for this haplotype are used. Liver derived cells are stained with tetramer-phycoerythrin (PE) presenting specific peptides and FITC labeled antibodies specific for surface markers (CD4, CD8) in FACS buffer (1% BSA, 0.1% sodium azide in PBS without calcium) for 30 minutes at room temperature or on ice. Events are acquired on a FACS-Calibur (Becton Dickenson) using the software Cellquest (Becton Dickenson) for analysis using 7AAD (BD Pharmigen) to exclude dead cells. Non-specific background staining is determined using T cells derived from naive or mock-inoculated mice. For cloning, lymphocytes are antigen stimulated and co-cultured at limiting dilution with gamma-irradiated syngeneic APCs plus IL-2 and IL-7 and tested for HCV specific proliferation.

### B. Results

### 1. Targeting liposome complexes to human hepatocellular carcinoma (Hep3B) cells in vatro.

Hep3B HCC cells (5 x 10⁴) were seeded in 24 well dishes and transfected with liposomes complexed with DNA plasmids (0.05µg/well) as described above expressing the luciferase gene under the control of a mammalian promoter. The ratios used for scL-HCV were 0.33ug:10ug(14nmol):1ug (TfRscFv:LipA:DNA) and for scLHK-HCV they were 0.33ug:7nmol:1ug (TfRscFv:LipA-HoKC:DNA). Twenty-four hours post-transfection, luciferase activity was determined using a Luciferase Assay System (Promega, Madison, WI). Results are shown in Fig. 1 as the means + SD (n=3). Efficient transaction was obtained using liposomes complexed to the anti-transferrin receptor single-chain antibody fragment (sc), with luciferase values of 257,815 RLU and 1,120,643 RLU) for sc/LipA and sc/LipA-HoKC complexes, respectively (Fig. 1). Compare this with a luciferase value of 538.9 RLU/µg protein for the luciferase plasmid complexed with unmodified Lipid A (LipA). This increase in transfection efficiency of DNA into this hepatic cell line when the HoKC peptide is incorporated into the liposome complex is consistent with previous observations using prostrate and pancreatic cell lines.

### 2. Targeting liposome complexes to primary chimpanzee hepatocytes in vitro.

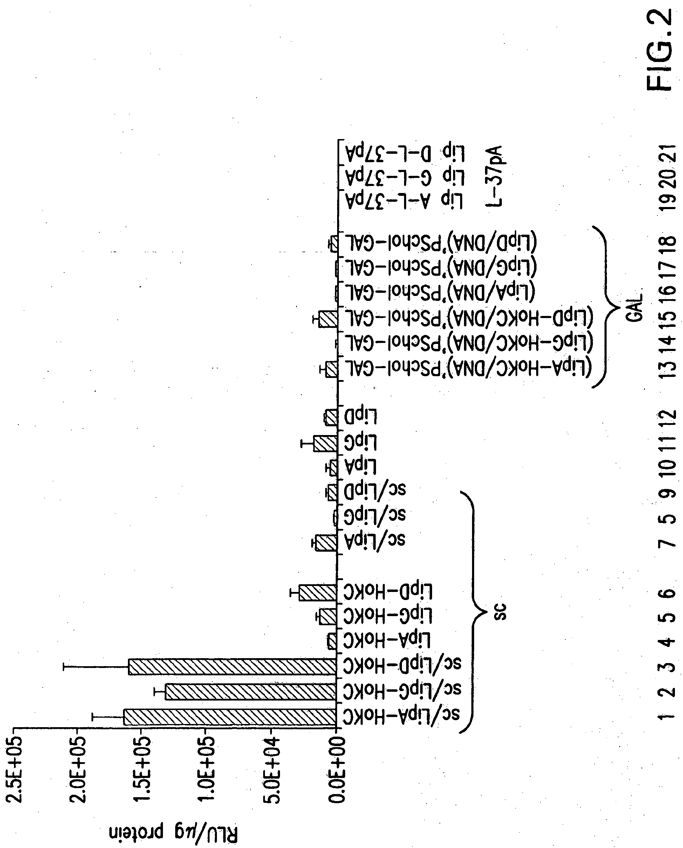

Normal hepatocytes do not have the same level of receptors as hepatocellular carcinoma cells, therefore sets of lipid complexes conjugated to the TfRscFv (sc), Galactose (GAL) and L-37pA ligands were compared in primary chimpanzee hepatocytes. Fifty thousand primary chimpanzee hepatocytes were seeded in 24 well plates and transfected with complexes with or without the HoKC peptide carrying the luciferase reporter gene (0.1µg DNA plasmid per well). The sc/Lip-HoKC/DNA (lanes 1-3) and sc/Lip/DNA (lanes 7-9) complexes were prepared by the simple mixing method disclosed above, i.e. the TfRscFv was mixed with the appropriate liposome (with or without HoKC) at a ratio of 0.33ug sc:7nm Lip-HoKC and 0.33ug sc:14nmol Lip, mixed by gentle hand inversion 10 times or rotated at 20-30 PRM for 15-60 seconds, and kept at room temperature for 10-15 min. The DNA was then added to the sc/Lip-HoKC or sclLip mixture (at a ratio of 1µg DNA:7nmol (for Lip-HoKC) and 1µg DNA:14nmol (for Lip)), and the solution mixed by gentle hand inversion 10 times or rotated at 20-30 PRM for 15-60 seconds, and kept at room temperature for 10-15 min. Unliganded complexes were also prepared as controls at the same ratios (lanes 4-6 and 10-12) and following the same procedure. The complexes using the L-37pA ligand (lanes 19-21 ) were formed by chemically conjugating the L-37pA ligand to the liposomes (A, D, or G) containing Lip-Maleimide at 6.7% molar ratio. The Lip-Mal was mixed with L-37pA at a ratio of 14:7 (nmol:nmol) (Lip:L-37pA) with the addition of 1.5mM HEPES buffer (final concentration) and rotated at 20-30RPM for 3 hours at room temperature. Afterward, DNA was added at a ratio of 1µg DNA:14nmol Lip, mixed as above and held at room temperature for 15 minutes. For comparison, a different method was also used to form the complexes with GAL ligand (lanes 13-18). Here the DNA is added to any necessary water and mixed as above. The liposomes A, D, or G (with or without HoKC) are added to any necessary water, and mixed as above. The two solutions are held at room temperature for 15 minutes, then added together, mixed as above and held at room temperature for 15 minutes to form a cationic liposome/DNA core. The GAL ligand, was previously conjugated to an anionic (phosphitidylserine:cholesterol) liposome at a molar ratio of 1:1:005(PS:chol:GAL). This PScholGAL mixture is then added to the pre-formed cationic liposome/DNA complex, the solution is mixed as above and held at room temperature for 15 minutes to encapsulate the cationic liposome/DNA core. The ratios of the components for these complexes are: 7nmol:7nmol:1ug (PScholGAL:Lip-HoKC:DNA) and 7nmol:14nmol:1ug (PSchoIGAL:Lip:DNA). Forty-eight hours post transfection, luciferase activity was determined using the luciferase assay system as described above. Fig. 2 shows the transfection efficiencies as determined by luciferase RLU per µg of protein in primary chimpanzee hepatocytes. The complex formed with all three methods and using all three ligands were able to transfect these primary chimpanzee liver cells. Using the primary chimpanzee hepatocytes, irrespective of the lipid used, the liposomes complexed with sc gave almost 10-fold higher transfection efficiencies (∼150,000 RLU) than the best liposomes complexed with GAL (15,509 RLU) or L-37pA (19,662 RLU) using different methods. As expected, due to the presumed lower number of the Tf receptor on normal hepatocytes, the overall transfection efficiencies of the sc/liposomes were up to 16-fold lower than when the same complexes were used in Hep3B cells. However, the inclusion of the HoKC peptide increased the efficiency of transfection using the TfRscFv by 10-fold. As previously observed using the Hep3B cells, the best transfection efficiencies with the GAL and L-27pA ligands were observed when the HoKC peptide was included with the GAL ligand. Thus, these studies demonstrate that the scLHK-DNA complex was highly efficient at delivering the DNA into normal liver cells, and that other ligands can also be used in the complex to transfect normal liver hepatocytes.

### 3. Targeting liposome complexes to liver cells in vivo.

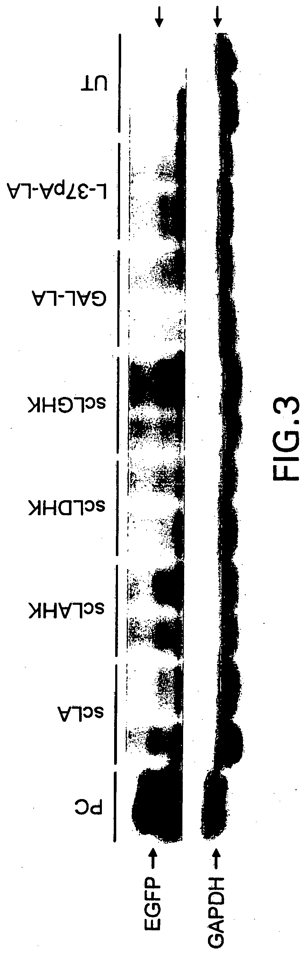

Following the *in vitro* experiments, tests were performed *in vivo* using non-tumor bearing BALB/c mice and specific liposome complexes. Six to nine week old animals were inoculated i.v. via the tail vein with 20-25µg of DNA plasmid expressing EGFP in 400µl of liposome complex, prepared as described above at the ratios given above, 2-3 times over a 24 hour period. At 24-48 hours after the last inoculation pieces of tissue from liver, spleen, kidney, lungs and heart were snap frozen in liquid nitrogen and stored at -70°C. In addition, pieces of liver tissue were fixed in formalin for histological analysis. For western blot analysis liver tissue was extracted using T-PER tissue extraction reagent (Pierce, Rockford, IL) containing protease inhibitor cocktail (Sigma) and supernatants collected after centrifugation to remove cell debris. Protein concentrations were assessed using a BCA protein assay reagent kit (Pierce, Rockford, IL) and 40 µg of total protein was analyzed using a monoclonal anti-GFP antibody (BAbCo) and an ECL weste blot kit(Yu W., et al., Nucleic Acids Research, 32:e48 (2004)). The same membrane was treated with an anti-GAPDH antibody to establish equal protein loading per well. Fig. 3 shows a western blot of liver extracts from duplicate mice inoculated with TfRscFv ligand (sc) conjugated with Lipid A (scLA), Lipid A and HoKC (scLAHK), Lipid D and HoKC (scLDHK), Lipid G and HoKC (scLGHK), Galactose conjugated with Lipid A (GalLA) and L-37pA conjugated with Lipid A (L-37pALA) compared to untreated mice (UT). The left hand lane shows positive controls (PC) for both GFP and GAPDH. All liposome complexes resulted in expression of GFP in the livers of inoculated mice. The inclusion of the HoKC peptide improved delivery of DNA to the livers of mice when Lipid A was used (compare scLA and scLAHK). The highest level of expression was observed with the scLGHK liposome complex, although this was in only one of the two mice and the scLAHK complex appeared to give more consistently strong expression of the GFP protein in replicate animals. The GalLA and L-37pALA modified liposomes also resulted in expression of GFP, but at lower levels.

Overall, this data indicate that although all three ligands can be used, the gene delivery vehicle containing the targeting ligand TfRscFv and the HoKC peptide in the liposome is better for liver cells than galactose or the L-37pA peptide. These results are unexpected since galactose is often used to target liver cells (Wu, J., et al., "Targeting Hepatocytes for Drug and Gene Delivery: Emerging Novel Approaches and Applications," Frontiers in Bioscience 7:d717-725 (2002).

### 4. Construction of DNA and viral vectors to induce immune responses to HCV

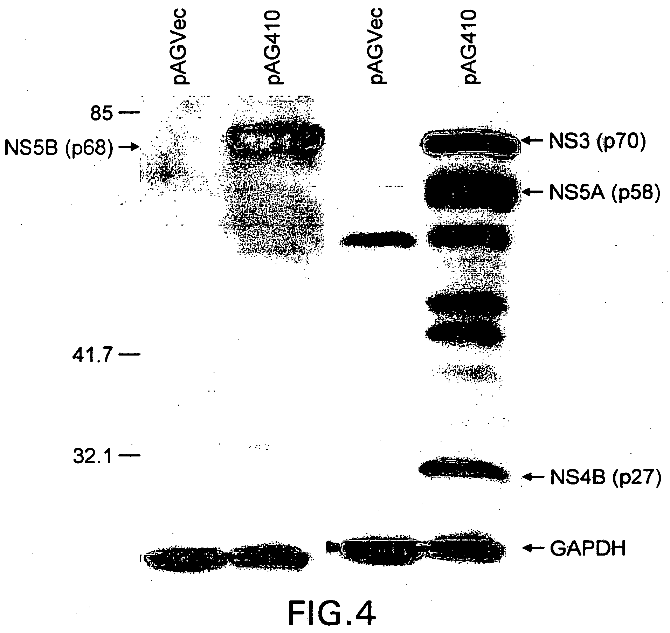

Using standard cloning methods well known to those familiar with the art, a DNA vector has been constructed to express the NS3 through NS5B proteins of HCV under the control of a high expression promoter (pAG410). The backbone vector used to express these HCV-specific proteins has already been approved for clinical use by the FDA and is currently in Phase I clinical trials in patients using a cancer-specific formulation. The expression has been tested *in vitro* using 293T and Huh7.5 (hepatocyte) cells and shown to specifically express HCV antigens through both western blot (Fig. 4) and immunofluorescence (data not shown) analysis. Cells in 6 well dishes were transfecfed using Lipofectin (Invitrogen, CA) using the manufacture's protocol. After 48 hours, cells were analyzed for specific gene expression. For western blot analysis, cells were lysed using 250 µl of M-PER reagent (Pierce, Rockford, IL) containing protease inhibitor cocktail (Sigma). Eighty micrograms of total protein were analyzed by western blot using 8-20% SDS-PAGE gradient gels (Invitrogen). Proteins were transferred to PVDF membranes and probed with a monoclonal antibody to NS5B diluted 1:500, or chronic chimpanzee serum diluted 1 :500. Proteins were visualized using anti-human or anti-mouse IgG HRPO conjugate and chemiluminescence (West Pico system, Pierce, Rockford, IL). Fig. 4 shows specific bands observed for NS3, NS4B, NS5A and NS5B compared to cells transfected with pAGVec (empty vector). Adenovirus recombinants have also been constructed using standard cloning methods to individually express each of these same antigens. Expression from each of these viral vectors was also verified by western blot analysis (data not shown).

### 5. Enhanced Efficiency of human IL-2 cloned into the high expression promoter

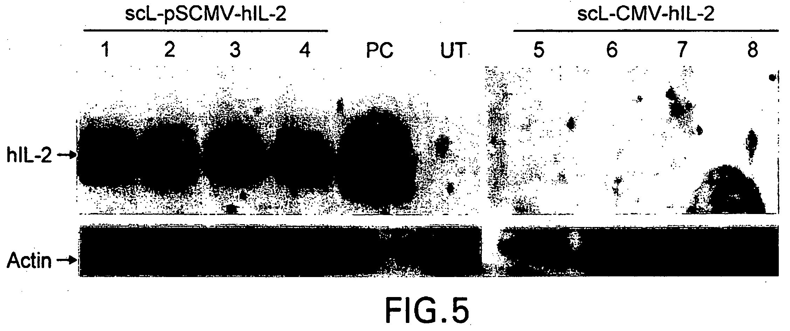

For the ILs to act as efficient adjuvant molecules, they must be expressed at high enough levels to effectively stimulate the Th-J T cell response. Putting them under the control of a high expression promoter is one way to achieve this. To demonstrate the high level of expression of the ILs possible by this approach, human IL-2 was cloned into a pSCMV vector under the control of the high expression promoter (see Published U.S. Patent Application No. 2007/0065432). The level of expression in vivo was tested and compared to that from a standard CMV promoter. In both cases, the plasmid was delivered by the targeted liposome nanocomplex without the HoKC peptide prepare as described herein. PANC-I human pancreatic cancer xenograft tumors were induced in 4-6 week old female athymic nude mice by subcutaneous inoculation of PANC-I tumor tissue. Approximately 3 weeks later, the tumors averaged 400-700 mm and the animals were given 6 i.v. (tail vein) injections over 6 days, with the nanoimmuno-liposome complex, using the ratio (0.33ug: 10ug:lug (TfRscFv:Lip:DNA)) and prepared by simple mixing as described above, carrying the gene for IL-2 under control of either a standard CMV promoter or the high expression promoter (40 ug DNA/injection). The mice were sacrificed 24 hr after the last injection. Tumor and lung tissues were collected, flash frozen in liquid nitrogen and analyzed for level of hIL-2 protein expression by Western Blot analysis using a commercial polyclonal antibody to hIL-2 and ECL detection using standard techniques. As shown in Fig. 5, all of the tumors from the animals that received the complex carrying the psCMV-hIL-2 plasmid demonstrated high levels of expression in the tumor. In contrast, no hIL-2 expression was evident in the tumors where the hIL-2 gene was under control of the standard CMV promoter.

### 6. Detection of HCV DNA sequences/expression in mouse tissue after i.v. delivery via scL

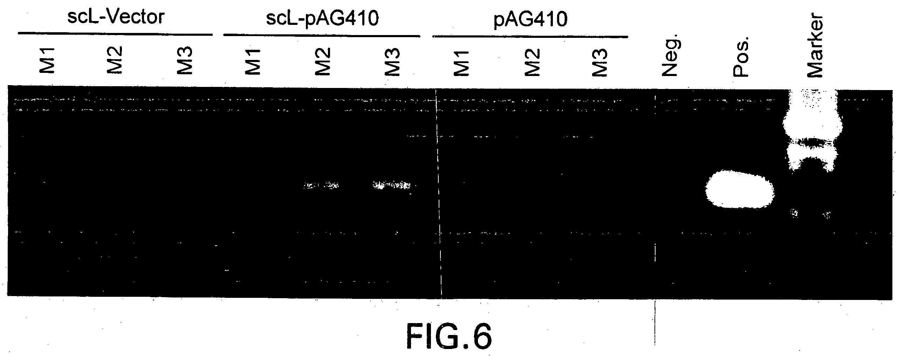

An experiment was performed to assess the presence of genes coding NS3 through NS5B in various non-tumor bearing mouse tissues after systemic administration of the scL-pAG410 complex. BALB/c mice (3/group) were i.v. injected twice (8 hr apart) with plasmid pAG410 DNA either complexed with scL (single chain transferrin antibody-targeted cationic liposomes) or as free plasmid DNA (25 ug/mouse/injection).The complex was prepared by simple mixing at the ratio (0.33ug:10ug:1ug (TfRscFv:Lip:DNA)) as described above. One group of mice received scL carrying the empty pSCMV vector prepared the same way and at the same ratios. 48 hours post-injection the animals were sacrificed. Liver, lung, spleen, and thymus were harvested and flash frozen in liquid N₂. A portion of the sample was used to isolate DNA using the DNeasy Extraction Kit (Qiagen) for PCR. DNA PCR (1 ug DNA) was performed in separate reactions using primers to amplify NS3, NS5A and NS5B using Taq Gold polymerase. PCR conditions were as follows: 6ng DNA, 2 mM MgCl₂; 0.2 mM each of dATP, dCTP, dGTP, and dTTP; 300 nM of each primer; and 1.25 units of AmpliTaq in a total volume of 50ul. Amplification was performed by incubating the reaction mixture at 94°C for 10 min, followed by 40 cycles at 94°C for 15 sec and 60°C for 30 sec, then 60°C for 7 minutes. Fig. 6 shows PCR data for the NS3 gene in liver, although signal differences were similar for NS5A and NS5B. There is a significant difference in the copy level in all organs between the mice receiving complexed vs. free pAG410. Similar to liver (Fig. 6), in lung, spleen and thymus, the scL-pAG410 had a strong PCR band while there was no signal detected with free DNA. As expected, there was no signal from the vector-treated mice. Thus, i.v. delivery of the complexed HCV plasmid DNA results in efficient uptake by cells in the liver and other organs. These results confirm and support the feasibility of this approach for systemic delivery of HCV antigens to normal liver and other normal cells.

### 7. Mouse immune responses

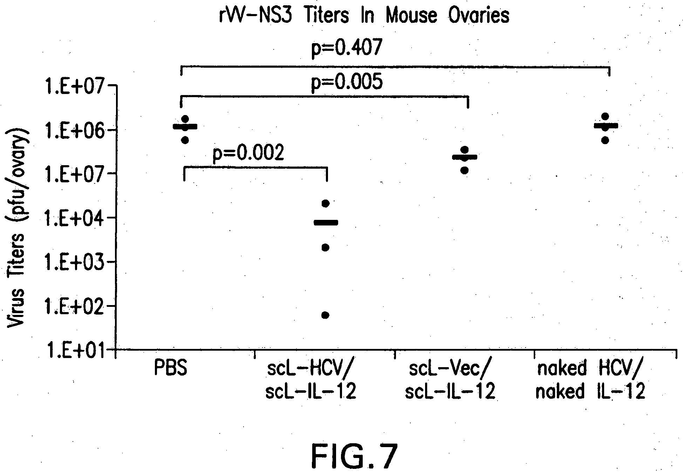

Mice cannot be infected with HCV. However, to assess efficacy of induced T cell responses, a recombinant vaccinia virus (rVV) expressing the NS3 protein of HCV was used as a surrogate virus challenge model. This type of rVV model is well known in the art and is an accepted means to assess *in vivo* efficacy of T cell responses. Mice (3/group) were inoculated I.V. at 0 and 4 weeks with HCV and IL-12 plasmid DNA, either scL complexed as described above or as free plasmid DNA. The TfRscFv/Lip/DNA complex was prepared by simple mixing as described above using the ratio of 0.33ug:10ug:1ug (TfRscFv:Lip:DNA). The DNAs were mixed in equimolar amounts (a total of 50ug/mouse/injection). The mice were challenged at 8 weeks with 10⁶ plaque forming units (pfu) rVV expressing the NS3 protein of HCV. At 5 days post challenge, the ovaries were removed from the mice, homogenized in PBS, and subjected to 3 rounds of freeze/thawing and sonication. For titrations, ovary extracts (100ul) were combined with 100 ul of trypsin and 100 ul of PBS and incubated at 37°C for 30 min. Dilutions (10-fold from 10⁻¹ to 10⁻⁶) were performed in 2% FBS/EMEM and 250 ul were used to infect duplicate wells of BSC-1 cells in 12-well dishes. At 2 days post infection, medium was removed and the cells fixed and stained with crystal violet (20% Ethanol, 20% formaldehyde, 0.3% crystal violet). Group 1 received PBS, Group 2 received scL complexed pAG410 and IL-12 plasmids, Group 3 received scL complexed empty-vector and IL-12 plasmids and Group 4 received the pAG410 and IL-12 plasmids as naked DNA. Fig. 7 shows the rVV titers expressed as pfu/ovary in each group. Dots represent individual mice and black bars the mean titers per group. Immunization with the scL pAG410/IL-12 complex resulted in a highly significant reduction in viral titers compared to the PBS group (p= 0.002), as assessed using the Student's T-distribution. Mean titer of 10⁴ in Gp 2 versus 10⁶ in Gp 1. Interestingly, inoculation of mice with scL complexed IL-12 vector along with empty backbone vector also led to significant reductions in viral titers (mean titer of 2x10⁵ pfu/ovary) although not as great a reduction as that seen when combined with the pAG410 plasmid. This effect on viral replication is a result of delivery using the scL since immunization with pAG410 and IL-12 as naked DNA (Gp 4) did not lead to significant reductions in viral titers. This data indicates that immunization with scL complexed DNA leads to the induction of T cell responses that can protect against viral challenge and that these immune responses are superior to those induced using naked DNA. This data indicates that the scL delivery of a cytokine expressing plasmid alone can also induce immune responses that inhibit viral replication.

### 8. Intrahepatic T-cell infiltration following immunization with scL complexed DNA

To assess whether immunization with scL-complexed DNA led to better intrahepatic T cell infiltration upon infection with a hepatotropic virus, the same 4 groups described above (immunized at 0 and 4 wks with identical complexes as above) were I.V. challenged 8 wks post immunization with 3x10⁸ infectious particles of non-replicating adenovirus recombinants expressing NS3, NS5A and NS5B HCV proteins. Ten days post challenge, liver tissue was harvested, formalin-fixed, and mounted in paraffin blocks using standard protocols well known in the art. Sections were H&E stained and analyzed for T-cell infiltration. The numbers of liver-infiltrating T-cells were assessed in the different mouse groups. Three fields of view were counted for each of 3 mice/group. Counts for each group were combined and averaged to give T cell numbers/ field of view (fov). The data was analyzed by Student's T test. The average T-cell infiltration in mice receiving scL pAG410/IL-12 was significantly higher than that in the mice receiving PBS, 118.8 T cells/fov vs. 70.2 T cells/fov (p=0.000007). This is compared to 71.5 T-cells/fov for mice receiving scL-vector/IL-12. There was also a significantly higher level of T-cell infiltration in the mice receiving naked pAG410/IL-12, 108.75 T-cells/fov (p=0.U001), though to a lesser extent than with scL-pAG410/IL-12. These observations demonstrate that immunization with the scL complexed plasmid expressing the HCV antigens leads to better T-cell responses in the liver upon infection with a recombinant hepatotropic virus.

### 9. Development of tools to express and test for HCV-specific RNA, antigens and immune responses.

Using the Sindbis expression system, expression recombinant viruses have been engineered to express the core, E1B2, and NS3 proteins of HCV. These proteins are then histidine tagged to facilitate purification using nickel columns. Efficient purification is performed and specificity determined by western blot analysis using mouse monoclonal or rabbit polyclonal antibodies. Chronic phase serum has been identified from infected chimpanzees with high titer antibodies to the E1E2, NS3, and NS5A proteins of HCV. This inactivated serum has been used in several western blot and immunofluorescence assays to successfully detect expression of HCV antigens from recombinant DNA and viral vectors. NS3, NS5A and NS5B proteins, commercially available from Mikrogen (germany), have also been used to detect T cell responses to HCV in chimpanzees using ELISpot, proliferation assays, and flow cytometry analysis. A set of overlapping 18 mer peptides have also been obtained from the NIH AIDS Research and Reference Reagent Program corresponding to the 1a H77 genotype sequence that have also been used successfully in Elispot assays using chimpanzee peripheral blood mononuclear cells (PBMCs). ELISAs have been developed to test for antibodies to the majority of HCV antigens, core, E1E2, NS2, NS3, NS5A, and NS5B. Recent studies have shown that the anti-E1E2 titers obtained using ELISA have proven good indicators of neutralizing antibody titers in infected chimpanzees. Both nested and real-time PCR assays have been developed to detect and quantify HCV RNA in serum. The sensitivity and specificity of these assays have been evaluated, and as few as 40 RNA copies/mL can be detected using nested PCR, and 200 RNA copies/mL (74 WHO IU/mL) using real-time PCR. All of these reagents and detection systems are specific to the monoclonal virus derived from plasma collected during the early acute phase of infection in a chimpanzee inoculated with RNA transcribed from an HCV genotypela (HCV-H) infectious clone.

### 10. Prophylactic vaccination of naïve and recovered chimpanzees prior to HCV challenge.

The ability of vaccines eliciting either glycoprotein-specific or nonstructural protein-specific immune responses to alter the course of HCV infection have been examined. These studies show that both neutralizing antibody (nAb) and vaccine induced T cells can modify HCV infection in the chimpanzee model.

### a) Envelope based vaccines modify HCV infection.

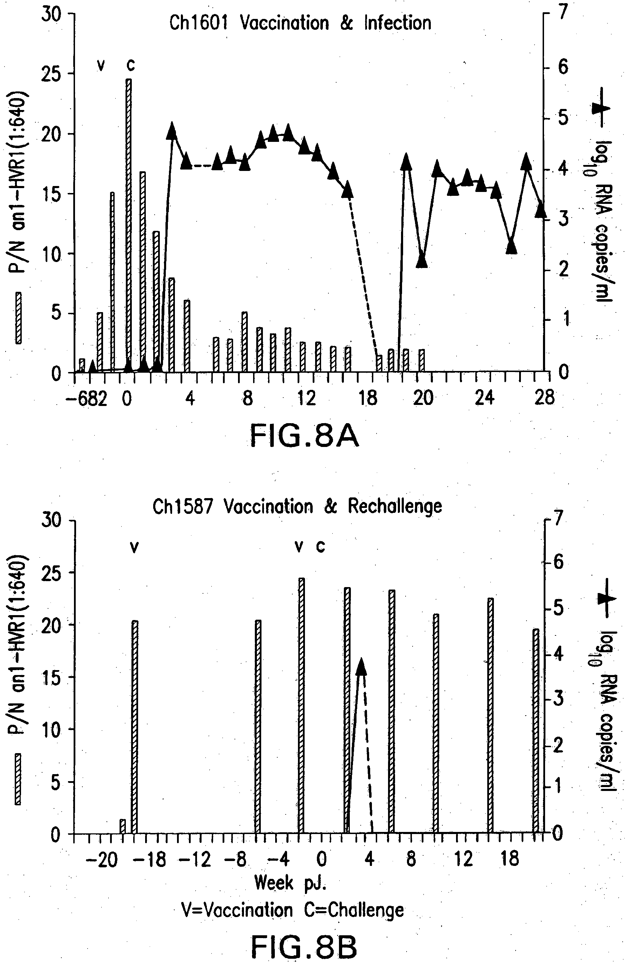

An expression and purification system to produce HCV E1E2 proteins for use in vaccine experiments has been developed. Histidine tagged E1E2 proteins were expressed using a Sindbis expression system and purified using GNL lectin and nickel columns to >80% purity, a method well established in the art. Specificity and purity were assessed by western blots using monoclonal antibodies to E1 and E2 and silver staining of SDS PAGE gels. Two chimpanzees, one naive (Ch1601) and one recovered from acute HCV infection (Ch1587) were immunized with 25 µg of rE1E2 proteins delivered intramuscularly and challenged with 100 chimp infectious doses (CID₅₀) of clonal HCV (Figs. 8A and 8B). Results of the challenge were compared to infection in a non-vaccinated control animal. In addition to strong and specific T-cell proliferative responses, immunization of both animals generated high antibody titers to E1E2 including antibody directed to the hypervariable region of E2 (HVR1). This region has been shown to be the most variable in natural isolates and is also thought to represent a nAb epitope. Antibody was measured using whole protein E1E2 and HVR1 peptide ELISA assays developed employing biotinylated HVR1 peptide or E1E2 protein purified from BSC-1 cells infected with a vaccinia virus recombinant expressing E1E2. Viremia was delayed three weeks in both vaccinated animals, In Ch1601, HCV RNA titers were maintained below 5x10⁴ copies/mi, and ALT levels where minimally elevated, An increase in intrahepatic cytokine mRNA levels coincided with a fall in HCV RNA to non-quantifiable levels. Despite this control, viremia reappeared as the antibody titer waned and the animal became chronically infected, this reappearance was not associated with immune escape. Chimpanzees that recover from acute HCV infection do not develop nAbs. Adding nAb to the already existing memory T cell responses induced in primary infection yielded an immune response capable of protecting the animal from re-infection. In Ch1587, viremia was detectable in only one sample at week 3 (<10⁴ copies/ml) with no evidence of hepatitis.

### b) T cell vaccines modify HCV infection

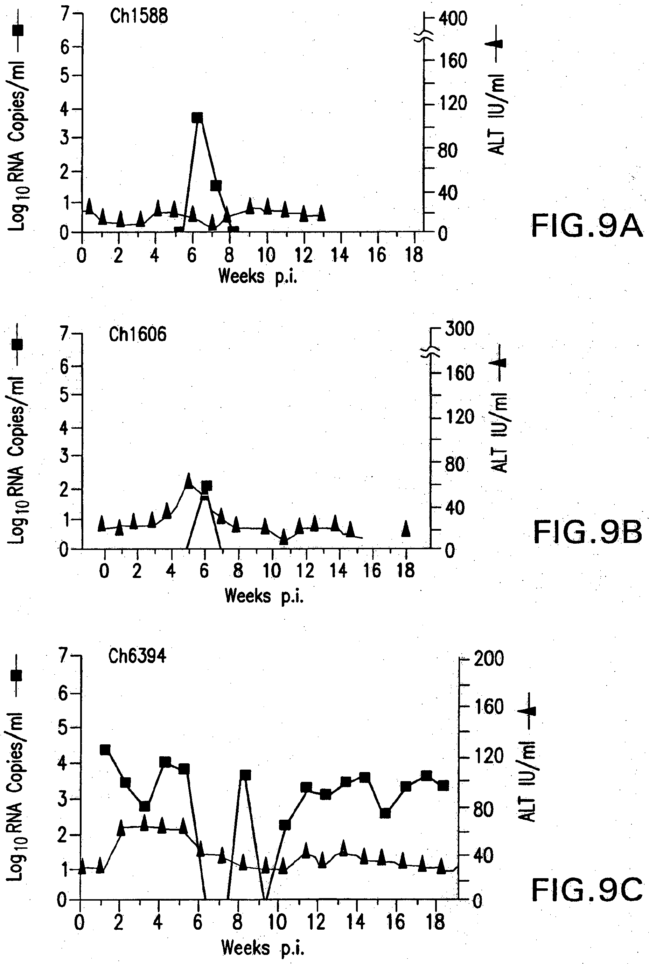

Vaccines not using the methods of this invention were constructed that included only HCV NS antigens NS3, NS5A and NS5B. A recovered chimpanzee (Ch1588) was immunized with a DNA prime/rVV (recombinant vaccinia virus) boost regimen against NS3. A second recovered chimp (Ch1606) was immunized with a vaccine that included NS3, NS5A and NS5B. The chimpanzees were challenged with 100 CID₅₀ of clonal HCV. In both animals, T cell responses were boosted following vaccination. Challenge of both animals resulted in a subclinical infection, with low-level viremia lasting only 1 (Ch1606) or 2 (Ch1588) weeks at 6 weeks post injection (p.i.) (Fig. 9A-9C). In comparison, non-vaccinated recovered chimpanzees develop viremia at 1 week p.i, that is detectable at least through week 14.

A naive chimpanzee (Ch6394) was then vaccinated with the NS3-NS5 vaccine and challenged with 100 CID₅₀ of monoclonal H77. T-cell responses were tested using proliferation and Elispot assays employing whole antigen and overlapping peptides. Ch6394 developed specific T cell responses following vaccination to all vaccine antigens. Upon challenge, this animal became viremic at 1 week p.i. but with a maximum titer of <10⁵ copies/ml and immediate control of virus replication (Fig. 9A-9C). In naïve animals titers increase ∼2-fold every 7 days whereas in Ch6394 the titers decreased 30-fold within the same period. However, at week 10, virus control was lost and the animal developed a chronic infection. This loss was concomitant with a loss of NS3-specific immune responses (IFN-γ-producing T cell frequency and proliferation) and correlated with the appearance of two amino acid mutations, one in the NS3 region and one in the NS5A regions. Elispot assays have shown that peptides carrying these mutations are no longer recognized by PBMCs from Ch6394 compared to the wild type peptides, indicating these changes represent immune escape mutations. Escape from CD4 T cells may have led to the loss of helper function, which could explain the loss of NS3 specific responses in this animal. These results suggest that a T cell based vaccine against HCV has the potential to control infection.

### 11. Prophylactic vaccination of naïve chimpanzees prior to HCV challenge

### a) Specific T cell responses are elicited in naive chimpanzees using the i.v. ligand-liposome nanocomplex (scL-HCV) vaccine strategy and are augmented after challenge

A vaccine study in naïve chimpanzees using a scL-HCV nanocomplex as described throughout has been performed. Injections to prime the immune response were done at 0,4 and 8 wks using the scL-HCV complex followed by a recombinant adenovirus (rAd) boost (16 wks). The complex used to vaccinate the animals was made at ratios of 0.33ug:10ug:1ug (TfRscFv:Lip:DNA), with 5% Dextrose as an excipient, at approximately 0.164 mgDNA/Kg/Injection, based upon the weight of the chimpanzees. The prepared, complex was injected into a 250ml 5% Dextrose bag for i.v. infusion and administered to the animals. Animals were challenged at 32 wks (16 wks post boost). The infusions with the nanocomplex and adenovirus boost were well tolerated with no adverse reactions in any animal.

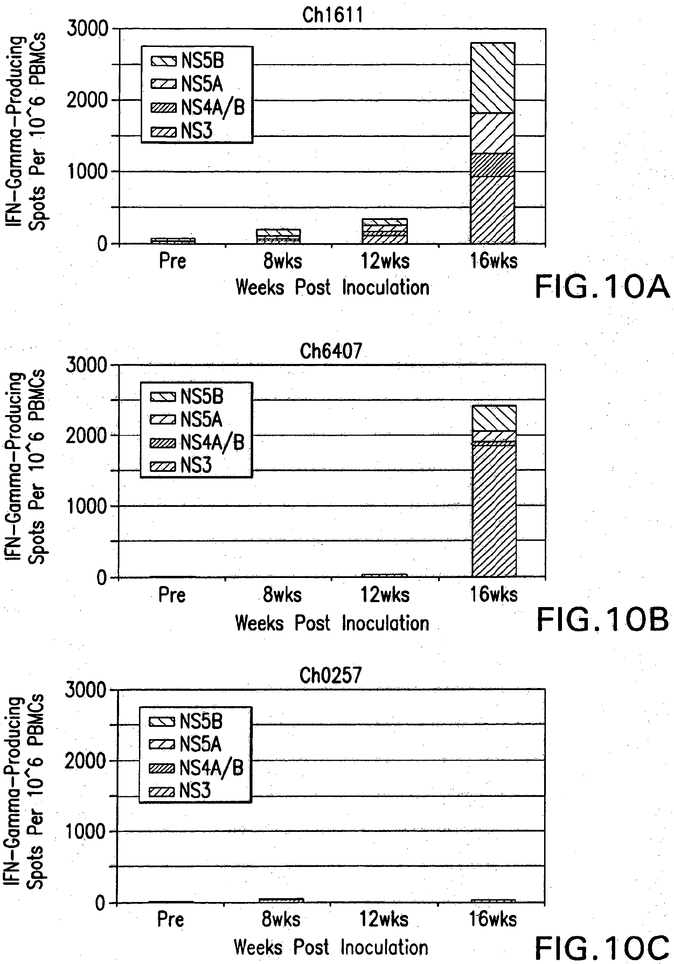

Post-vaccination: Elispot studies showed strong, broadly reactive HCV-specific IFN-γ responses at 16 wks post-vaccination in the 2 vaccinated animals (Ch1611, Ch6407), (Fig. 10A-10C) with minimal or undetectable responses in the control animal (Ch0257) that received empty vector nanocomplex and non-rAd. Low-level IFN-γ specific T cell responses were seen in Ch1611 and Ch6407 following inoculations with the sclHK-HCV, indicating immune responses to this priming component of the vaccine. Increases in intrahepatic IFN- γ and CD3 mRNA levels were also observed in the two vaccinated animals post immunization (Fig 12A-12C wks 12, 16, and 32). The data is expressed as mRNA levels relative to wk 0 (pre vaccination) and were normalized to an endogenous control (GAPDH). The increases in CD3 mRNA at wks 16 and 32 also indicate T cell infiltration in the liver. No increases in cytokines were seen for the control animal (Ch0257) any time after immunization and prior to challenge (wk 32) (Fig. 12A-12C). This data indicates that T cell responses were induced in the liver as well as in the peripheral blood following vaccination using the complex and approach disclosed herein, and are supported by analyses of serum cytokines in Ch6407 and control Ch0257 (using the TransSignal Cytokine Antibody Array kit (Panomics)). Two weeks after the first scL-HCV DNA nanocomplex treatment, we observed up-regulation of cytokines, including ICAM-1, various interleukins, TGF-α and IFN-γ, only in the vaccinated chimp, not in the control animal.

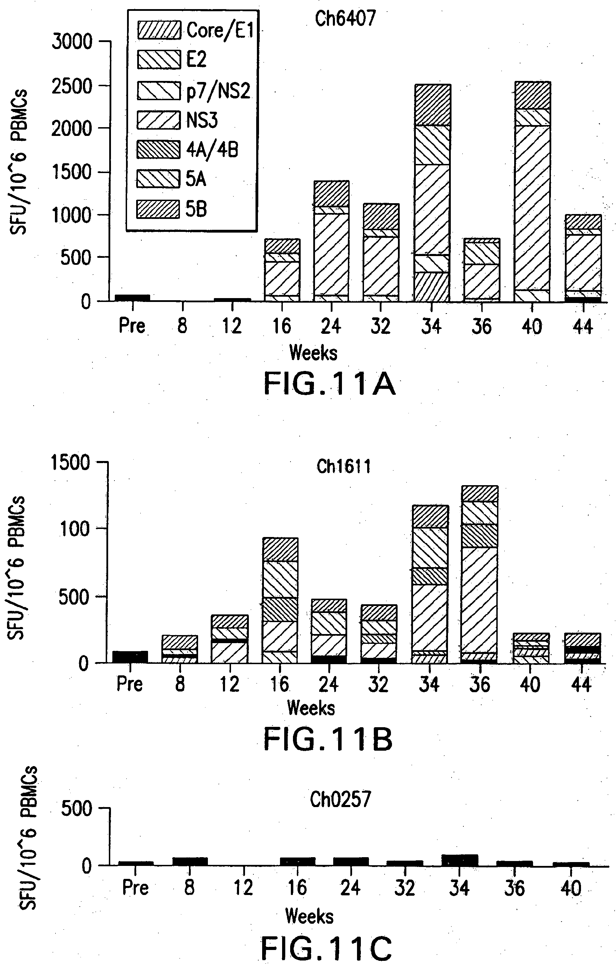

Post-Challenge: The immunized chimps were challenged using standard methods well known in the art, with 100 infectious doses of HCV at wk 32. After challenge both vaccinated animals showed immediate control of viral replication which coincided with increased HCV-specific T-cell responses as measured by Elispot assay (Fig 11A-11C) and increased intrahepatic cytokine responses (Fig. 12A-12C). Upon challenge both animals still had detectable T cell responses specific to the vaccine antigens (NS3-NS513). Following challenge and in response to viral replication, these responses increased. Elispot assays using PBMCs from the control chimpanzee, showed no HCV-specific responses (Fig. 11A-11C). This absence of detectable immune responses in the acute phase of naïve infected animals is consistent with other published observations. The levels of intrahepatic IFN-γ mRNA increased immediately following challenge in the two immunized animals (Fig. 12A-12C). Evidenced by 8- and 10-fold increases above baseline for Ch6407 and Ch1611, respectively at wk 34 (2 wks post challenge). These increases indicate an immediate immune response to the viral infection in the liver. Intrahepatic cytokine levels only began to increase in the control animal several weeks after challenge and did not attain levels comparable to those in the immunized animals until wk 41.

Viral titers were measured using a real-time PCR assay from wks 0 to 8 and serum ALT levels were measured from wks 0 to 6. Fig. 13 shows that both the vaccinated animals exhibited significantly lower virus titers post-challenge than those observed in the control chimp. Moreover, the titers did not increase exponentially during the first 5 wks as can be seen in the control chimp and as is seen in all naïve animals. In fact, in both of the vaccinated animals the viral titer is greater than 4 log₁₀ lower than the control animal by week 5. Both vaccinated chimps, but not the control chimp, also demonstrated ALT elevations 2 wks after infection, confirming the intrahepatic cytokine data indicating immune responses in the livers early (Fig 14). As expected, the ALT levels in the control animal did not increase until later in the infection. Infection of all animals after challenge was expected as this is a T cell vaccine and is not designed to induce neutralizing antibody and sterilizing immunity. An initial replication of virus is required to stimulate the previously induced T cell response. This data indicates that this I.V. scLHK-HCV vaccine strategy induces memory T cells that are recalled upon infection with virus and that this recall response is effective at immediately controlling viral replication.

### 12. Testing immunotherapeutic strategies in chronically infected chimpanzees

Vaccines were constructed that included HCV NS3, NS5A and NS5B to determine if a T cell immunotherapeutic vaccine might prove effective in chronically HCV-infected chimpanzees. A naked DNA prime (NOT scL-HK) (i.m.)/recombinant vaccinia virus (rVV) boost regimen was used to treat a chronically infected chimpanzee which was immunized with an NS3, NS5A and NS5B vaccine delivering 3 doses of naked DNA combined with CpGs as adjuvant i.m. followed by a rVV boost. Following the inoculation of rVV, T cell responses in the peripheral blood increased 12-20 fold against NS3, NS5A and NS5B (Fig. 15). However, the levels of HCV RNA and liver enzymes were not affected; RNA titers remained at ∼10⁴ RNA copies/ml, similar to pretreatment levels. Following analysis of intrahepatic IFN-γ and TNF-α mRNA, no increase for either of these cytokines was noted, suggesting the increased T cell activity in the blood did not lead to increases at the site of HCV replication. Failure to control the virus may have resulted from the failure of T cells to function in the liver.

Four different chronically infected chimpanzees were are i.v. injected with the scL-HCV complexes of the present invention made by simple mixing as described herein, encapsulating pSCMV plasmid DNA carrying the genes encoding for NS3, NS4B, NS5A and NS5B, carrying the gene encoding for IL-2, or empty vector. The complex is made by simple mixing at ratios of 0.33ug:10ug:1ug (TfRscFv:Lip:DNA) at U.2mg/Kg/.injection based on body weight. 5% Dextrose is added as an excipient. The DNAs are mixed prior to complex preparation at a molar ratio of 1:1 for HCV:IL-2 or 2.5:1 for empty vector:IL-2. Three animals are vaccinated with scL/HCV-IL-2 and one receives complex carrying empty vector-IL-2. Each animal receives three injections 4-6 weeks apart. A boost with recombinant adenoviral vector follows 4 weeks after the last vaccination.

Studies involve scL-DNA or scLHK-DNA inoculations delivered i.v. using equimolar amounts of recombinant vectors expressing HCV antigens and IL-2. As all expressed genes are in the same backbone vector, basing the DNA ratios on a molar amount ensures that all expressed inserts are delivered equally. The separate HCV and IL DNAs are mixed at equimolar ratios of the vector inserts and encapsulated into the scL or scLHK. DNA is produced as Endotoxin-free preparations (< 5EU/mg DNA) under GLP conditions (Aldevron, ND). DNA (∼7mg/animal/injection) is administered in the complex based upon the weight of the chimps (0.17mg/kg). This dose is roughly equivalent to that administered to a 60 kg human, and is the dose previously used to vaccinate the naïve chimps. The complex is prepared, lyophilized and shipped to the location of the animals for reconstitution and injection. The scL-HCV/IL-2 or scLHK-HCV/IL-2 i.v., is delivered at 0, 4, 8 and 12 weeks in 3 animals. This schedule is used in order to give the immune system time to respond to the inoculations. If no effect is seen on T-cell responses or RNA titers after 4 weeks, we would not expect to see any new effects without additional inoculations. The 4th (control animal) receives scL or scLHK complexed empty vector and IL-2 plasmid at the same time points. This distinguishes the effect of this cytokine alone on the HCV infection. If no virological response is observed after 4 doses a boost with the rAd (10¹¹ infectious units) that expresses the HCV antigens NS3-NS5B as used in the prophylactic vaccine study is given and monitoring of the immune responses and virus titer continues. The use of a hepatotropic viral vector also achieves the goal of targeting the liver compartment.

### C. Sampling

Animals are studied for up to 24 weeks. Sample collection starts 4 weeks prior to the first inoculation; these samples are used as baseline and negative controls. Sampling continues until 8 weeks after the last inoculation, allowing time in which to determine if treatment is yielding a positive response. Throughout the study liver biopsies are performed bi-weekly by the Menghini technique as defined by NIRC SOPs and blood is drawn via the femoral vein weekly for use in the studies detailed below. 40mL of whole blood is obtained on even weeks (weeks -4, -2, 0, 2 etc.) and 5mL of plasma on odd weeks (-3, -1, 1 etc.). To determine stability of the plasmid 1 mL of blood is collected at pre-dose and 15 min, 1, 3, 8, 24, 48, and 72 hr after the first inoculation. All other blood draws and liver biopsies are obtained prior to inoculations. This bleed schedule is based upon guidelines produced by NIRC for blood collection in non-human primates. 90mL per month are collected, well within allowable limits. Blood is collected with heparin as anticoagulant. The plasma is obtained from the 5 mL and 1 mL volumes by centrifugation at 2700 rpm for 10 minutes. Plasma samples are separated into 500ul aliquots and stored at -80°C. Half of the liver biopsy is fixed in formalin, half is divided into 2mm sections and snap frozen in liquid N₂.

### D. Determining the Presence in the Liver and Stability of scL-HCV or scLHK-HCV Complex in Blood

Our preliminary studies in mice indicate that encapsulation significantly increases the amount of nucleic acid found in the liver and extends the time within which it can be detected in the blood. These findings are confirmed with the encapsulated HCV NS3-NS5B plasmid in the chimpanzees. To determine how long the HCV DNA is present in circulation post-injection, 1 mL of blood is collected predose and 15 min, 1, 3, 8, 24, 48, and 72 hr after the first injection. Plasma is separated into two aliquots and frozen at -80°C. One aliquot is used to isolate plasmid (High Pure PCR Template Preparation kit (Roche)). DNA is isolated from one 2mm piece of snap-frozen liver tissue (DNeasy Extraction Kit (Qiagen)). Negative control samples are plasma and liver tissue from the same animal taken prior to inoculation. Exogenous HCV plasmid is assessed by PCR. Using PCR in the absence of reverse transcriptase distinguishes the plasmid from any viral RNA present due to the chronic infection. In addition, one primer located in the pSCMV backbone and one in the HCV insert are used. Thus, only exogenous DNA is amplified. This approach has been successfully used with other inserts in the pSCMV vector (p53 for IND biodistribution studies). To quantify, the signal standards are prepared by adding known amounts of the NS3-NS5B plasmid to plasma or liver tissue from uninfected chimpanzees, followed by extraction. The FDA has large amounts of archived samples from animals that are used. Scanned gels are quantified using IMAGEQUANT^{®} image analysis software (Molecular Dynamics) and the amounts of plasmid calculated by comparison to the standards. DNA is isolated from the 2^{nd} aliquot of plasma and from a 2^{nd} piece of snap frozen liver tissue for Southern Analysis using the Stratagene DNA Extraction kit. Plasma and liver tissue taken prior to inoculation are included as negative controls. Hybridization is performed using the Gene Image Alkaline Phosphatase DNA Labeling and Detection System (Amersham). As above, the probe for detection of exogenous DNA by Southern Analysis spans the junction between vector and insert. This probe is synthesized by IDT, Inc. (Coralville, IA). This method has also been used to analyze exogenous DNA in tissues. Standards are prepared as above. Samples are then analyzed by agarose gel, blotting and hybridization. A 10- to 50-fold excess of the probe is used to ensure saturation of all bands. Scanned autoradiographs are quantified using IMAGEQUANT^{®} software (Molecular Dynamics) and the amounts of plasmid DNA calculated by comparison to the standards. If the level or sensitivity of this non-radioactive method is too low, '5- end labeling with γ³² :P-ATP can be used or nested PCR can be performed as previously described (Fernandez J., et al., J. Virol, 78, 9782-9789 (2004)). Using both methodologies to assess DNA in the blood and liver helps ensure results are obtained should one method be unsuccessful. Tests are performed in triplicate and the plasmid levels in the sequential plasma samples from each animal are statistically compared with that observed at 15 minutes using the t-test to assess significant decreases in the DNA concentration over time. Statistical comparisons are not made for liver as the studies on this tissue are to demonstrate the presence of the DNA in this organ not the stability over time.

### E. Test responses to scLHK-HCV complex after I.V. inoculation of chronically infected chimpanzees

### 1. Assess Immune Responses in Peripheral Blood

Increased specific T cell activity in the peripheral blood of a chronically HCV-infected chimpanzee receiving a therapeutic vaccine does not necessarily lead to higher T cell activity in the liver and reduced viral titers. Thus, it is crucial to analyze the T cell infiltration and cytokine responses in the livers of these animals. However, the peripheral blood can be used as a first source to assess extent and specificity of induced immune responses. Liver biopsies are precious and limited and more T-cells are obtained from the peripheral blood. Studies of the liver are used to determine if increased immune responses in peripheral blood correlate with greater T-cell activity in the liver. PBMCs are isolated from whole blood obtained bi-weekly using Ficoll gradient centrifugation as previously described in the art (Puig M., et al., Vaccine, 22, 991-1000 (2004)). The plasma is removed from the top of the gradient, and stored at -80°C in 1mL and 200uL aliquots. Approximately 10⁸ PBMCs and 15mL of plasma are obtained from 40mL of whole blood. Single aliquots of frozen cells (10⁷/mL) are thawed and washed in AIM-V medium (Invitrogen) plus 2% Human AB serum (Biowhittaker). Viable cell numbers are assessed and cells are tested by Elispot (Shoukry N:H., et al. J. Exp. Med., 197, 1645-1655 (2003)). The total number of cells used for each analysis depends upon the number of antigens tested in one assay. A full panel of overlapping peptides is used (18-mers overlapping by 11 aa, NIH Reagent Program), covering and corresponding to the la genome. These peptides correspond to the virus used to infect the chimpanzees and to the sequence used in the HCV plasmids in the scL-HCV or scLHK-HCV. 10 peptide pools containing 30-45 overlapping peptides to screen samples are used. A negative ovalbumin peptide control, a positive phytohemagglutinin control and a media control are included in each assay. Samples are tested in triplicate using 10⁵ cells/well. Samples are tested against peptides, covering the entire viral genome. Although the vaccine is expected to stimulate responses to antigens NS3-NS5B, this stimulation may result in improved T helper responses or increased IL-2 production that lead to enhanced responses against viral antigens not included in the vaccine. Pro-stimulation of the PBMCs and Elispot analysis of cells producing EFNγ, IL4, IL2 and IL10 are performed at 1 µg/ml of each individual peptide and human Elispot test kits (U-Cytech, Netherlands) (Shoukry N:H., et al: d. Exp. Med., 197, 1645-1655 (2003)) in 96-well plates. The numbei of specific spot forming units (SFU) is calculated by subtracting the mean value of triplicate control (no antigen) from the mean value of triplicate test wells. T cell proliferation assays are performed on PBMCs as previously described in the art (Puig M., et al., Vaccine, 22, 991-1000 (2004)) using 96-well plates (10⁵ cells/well in triplicate) Cells are stimulated with 1ug/mL of protein (NS3, NS5A or NS5B) (Mikrogen, Germany) or peptide. For later studies CD4+ and CD8+ T cells are depleted from the total population using anti-human CD4+ or CD8+ dynabeads (Dynal) (Puig M., et al., Hepatology, 44, 736-745 (2006)) and tested to determine the specific cell type that recognizes specific antigens. Data generated from these studies gives the best indication of enhanced HCV-specific T cell responses. Results are compared between pre and post-vaccine samples for each animal. The t-test are used to assess significant increases in SFU following vaccination. The results of the studies on the peripheral blood are coupled with analyses of intrahepatic T cell infiltration, cytokine responses and changes in viral RNA levels to determine the efficacy of this therapeutic strategy.

### 2. Detection of Changes in Plasma Cytokine Levels.

Analysis of serum cytokines after inoculation of naïve chimpanzees with scL-HCV showed changes in a number of cytokines (ICAM-1 TGF-β and IFN-β) in the vaccinated animal but not in the control. Changes in a panel of serum cytokines after therapeutic vaccination are examined and compared to pretreatment samples and the control animal. The cytokines are analyzed using the TransSignal Human Cytokine Antibody Array kit with 1-2mL of plasma taken pre and post vaccination. Normal plasma from an uninfected chimpanzee is included as a negative control. Changes seen in serum cytokines (both type and levels) are compared with those found at the mRNA level in the liver. These studies on serum cytokines are performed in addition to the intrahepatic analyses of mRNA. The mRNA analyses are quantified, analyzed statistically, and provide an indication of the cytokine levels in the liver. It is important to couple these analyses to determine if the responses in the peripheral blood reflect those observed in the liver. Any changes in cytokine pattern are correlated with the persistence of memory T cells. A positive correlation is the first step towards developing a more simplified means of determining the presence of specific T cells associated with clearance.

### 3. Assessment of HCV-specific responses in the liver by Histological and Cytokine Analyses

### a) Test for presence of liver infiltrating T-cells.

Evidence of infiltrating T-cells is tested for in the liver by histological analysis and cytokine responses. Increased HCV-specific T cell activity in the peripheral blood of a chronically infected chimpanzee receiving a therapeutic vaccine does not necessarily lead to higher T cell activity in the liver and reduced viral titers. Thus, it is crucial to analyze the T cell infiltration and cytokine responses in the livers of these animals.

### b) Analysis of formalin-fixed liver tissue:

Formalin fixed paraffin-embedded tissues are sectioned, stained for specific proteins and cell markers: CD4; CD8; Perforin; IFN-γ; IL-2; CD45RA/RO and CCR-7. T cell populations can be identified on the basis of the expression of CD45RA and CCR7 (Campbell J.J., et al., Journal of Immunology, 166, 877-884 (2001); Sallusto F., et al. Nature, 401, 708-712 (1999); Seder R.A. and Ahmed R., Nature Immunology, 4, 835-842 (2003)). Immunohistological analyses of liver sections are performed to distinguish the numbers, types and location of lymphocytes present in the liver for each biopsy. Sections are deparaffinized in xylene, hydrated in alcohol then washed in PBS. For antigen retrieval, tissue is treated with 10mM citrate buffer pH 6.0 at 100°C for 10 minutes. Non-specific sites on tissue sections are blocked with 5% milk before incubation with specific antibodies. Liver biopsies obtained from the same animals before infection (historical stored samples) and prior to treatment are used for comparison. These studies are performed with a labeled streptavidin-biotin technique and product developed using DAB. Six fields of view are analyzed for pre and post treatment sections and positive cell types counted. With this staining method the locations of the cells within the liver relative to the portal vein or particular liver cell types is assessed. Statistical analyses are performed using the t-test for T cell infiltration.

### c) Analysis of snap-frozen liver tissue:

Total RNA is isolated from a portion of the snap frozen tissue using the RNeasy Mini Kit (Qiagen, CA). RNA is reverse transcribed using the First Strand cDNA Synthesis Kit (Pharmacia) and tested by Real-Time PCR for cytokine mRNA levels using specific primer probe sets for IFN-γ, IL2, CD3, CD8, and CD4 as previously described in the art (Major M.E., et al. Hepatology, 39, 1709-1720 (2004)). The levels of these cytokines is normalized to an endogenous control (GAPDH) and statistically compared to levels in pretreatment biopsies for each animal (Thimme R., et al. Proc. Natl. Acad. Sci. U. S. A, 99, 15661-15668 (2002)). At the same time a small portion of the frozen liver tissue is used to simultaneously analyze the protein levels of a panel of 36 cytokines using the TransSignal Human Cytokine Antibody Array kit (Panomics).

### F. Assessing Toxicity.

It is particularly important to study the effects of a liver related therapeutic vaccine in chronically infected individuals. While the vaccine may lead to reduced viral titers, due to enhanced immune responses in the liver, it might exacerbate hepatitis and lead to liver disease. Serum and liver tissue from the chimpanzees are analyzed for liver-specific enzymes and histopathologic changes indicative of toxicity. An aliquot (2mL) of plasma is tested weekly by a commercial laboratory (ANTECH Diagnostics) for a panel of markers specific for liver function: total bilirubin, ALT, Alkaline phosphatase (ALP), serum albumin, γ-glutamyl transferase (GOT) and glutathione-S-transferase (GST). Increases in the levels of these markers above the normal range lasting <2 weeks after any inoculation is considered acceptable. Persistent levels above the normal range is indicative of toxicity. As this is one of the few tests specific for liver disease (McIntyre, N. and Rosalki, S. Biochemical investigations in the management of liver disease. In: Oxford Textbook of Clinical Hepatology McIntyre, Benhamou, Bircher, Rizzetto and Rodes (eds). Oxford University Press, 293-309. (1991)), pre and post treatment ALT levels are compared for each animal to statistically assess toxicity.

To assess apoptosis, tissues are stained with a modified fluorescence TUNEL assay using the in-situ cell death detection kit TMR Red (Roche). This signal emits in the far red range and is better than FITC as a signal for apoptosis in liver sections as red blood cells auto fluorescence under the Argon laser. Fluorescence intensity and distribution are assessed using a Laser Scanning Cytometer (LSC). This is a slide-based instrument that performs in a manner similar to a flow cytometer and detects FITC, PE, PI, Cy5 and DAPI. Scan settings are optimized and applied to all samples with the same protocol measuring background, negative and positive signal. Data displayed as a histogram allows statistical analyses of the signals by the Compucyte program and quantitative evaluations of the percentage area of positive apoptosis signal in tissue as well as the intensity of the signal.

H&E stained liver sections are evaluated by a board certified veterinary pathologist for histopathologic changes. Pre and post treatment sections are graded using the Ishak scoring system (0-4 scale) for assessing chronic hepatitis (Ishak K., et al., Journal of Hepatology, 22, 696-699 (1995)). This is an established method for grading liver biopsies and is widely used in the clinical setting. Grading includes assessment of portal, periportal and intra-acinar inflammatory cell infiltration and liver cell damage and necrosis. Using these 3 methods to analyze liver toxicity, a complete picture of the effects of this therapeutic vaccine on this organ to assess safety for future clinical use is provided.

### G. Monitoring Changes in Viral Titers.

The primary test of an immunologic response and success of the scL-HCV or scLHK-HCV therapeutic approach is change in HCV RNA titers in the blood. These are determined using an in-house, real-time RT-PCR assay (Major M.E.,et al, J. Virol, 73, 3317-3325 (1999); Major M.E., et al., J. Virol, 73, 3317-3325 (1999)). Total RNA is isolated from 200 uL of serum from pre and post treatment bleeds using TriZol (Invitrogen) and RT PCR performed as previously described in the art (Major M-.E., et al., J. Virol, 73, 3317-3325 (1999); Fernandez J., et al., J. Virol, 78, 9782-9789 (2004)). The RNA is divided between replicate wells (n=4), the RNA concentration calculated using a set of HCV RNA standards and expressed as RNA copies/mL of plasma. We have extensive experience amplifying HCV sequences under clean, controlled conditions (Major M.E., et al., J. Virol, 73, 3317-3325 (1999); Fernandez J., et al., J. Virol, 78, 9782-9789 (2004)). Procedures are performed in a separate room to avoid contamination and in all cases designated pipettes and reagents are used. In all extractions negative control plasma from uninfected chimpanzees is included and the extracted RNA used in RT-PCR assays. Pre and post treatment RNA titers are compared to determine statistically significant changes in titer. Plasma is analyzed for viral RNA titers rather than liver. Liver biopsies are limited, the titers in plasma are directly indicative of levels in the liver. Only when animals show clearance of virus is the liver tested for viral RNA by a more sensitive nested PCR that detects as few as 40 RNA copies/mL (Major M.E., et al. Hepatology, 39, 1709-1720 (2004), Puig M., et al.. J. Virol. Methods, 105,253-263 (2002)).

If the targeted therapeutic vaccine leads to decreases in RNA titers, increases in intrahepatic T cells, intrahepatic cytokines and peripheral HCV T-cell responses, this is a strong indication that HCV-specific responses are functional in the liver, particularly when compared to the control animal. If intrahepatic T cells and cytokine levels increase but viral RNA titers do not decrease, this suggests that the T cells induced did not recognize viral epitopes due to escape mutations or T cell dysfunction. A transient decrease in RNA titers followed by resurgence to pre-treatment levels is indicative of mutations developing in the virus to escape the newly induced T cell responses. This is investigated through sequence analysis of the virus before and after resurgence. If mutations are observed, T cell escape can be tested *in vitro* using PBMCs and peptides representing the new and old amino acid sequences spanning the region found to contain the mutation.

These studies in chronically HCV-infected chimpanzees are crucial for evaluating any HCV-specific therapeutic vaccine that may be used in chronically infected patients. It is important to show that the enhancement of HCV-specific T cell responses does not increase hepatic inflammation to levels intolerable for a clinical treatment.

### Example 2

### Intranasal (IN) Administration of Immunoliposomes

Vaccine administration via an IN route is especially useful for certain types of viruses, such as those that have a tropism for tissue of the head and neck (this includes the nose and throat). Examples include viruses that induce respiratory illnesses which includes not only influenza but also SARS and avian flu. However, this route could also be used for delivery of other types of vaccines as well. To compare the ability of the scL complex to target and transfect local tissue when given IN, non-tumor bearing BALB/c mice were given either the scL nanoimmunoliposome complex encapsulating a plasmid DNA that expresses the Luciferase gene (scLLUC), or an equal amount of the free, uncomplexed (naked) plasmid DNA (LUC) (10 ug/mouse/dose for both treatments), or no treatment (CTL). The scLLUC complex, prepared as in Example 1, by simple mixing at ratios of 0.33ug: 1 0ug 1 ug (TfRscFv:Lip:DNA), with 5% Dextrose as an excipient. Two doses were administered within 24 hours. 48 hours after the last dose, the animals were injected with the luciferin substrate (ip administration) at ∼3mg/mouse and the whole animals visualized under anesthesia using an IVIS 100 optical imaging system (Xenogen, Alameda CA) (Figures 16A-16C). The intensity of the signal expressed is indicated by color in the actual measurements. Red/yellow has the highest intensity which corresponds to the highest level of expression of the transfected luciferase gene, while blue represents minimal expression, with green being moderate expression. These levels of expression (high/moderate/minimal) have been indicated on the Figures 16A-16C and 17A-17C in place of the color representation. It is clear in Figures 16A-16C that IN administration of the scL complex (scLLUC) (Panel C) results in a much higher level of transfection/expression in the nasal region as compared to the free naked DNA (LUC) (Panel B). Panel A represents an untreated control It is surprising and unexpected that the scL nanoimmunoliposome complex would yield results so much better than naked DNA (as would be currently used) when both are administered directly to the tissue to be transfected (local delivery).

### A. Demonstration of the Enhanced Distribution and Localization of the Plasmid DNA Payload as Compared to Naked DNA After IV Administration

Using the IVIS 100 optical imaging system (Xenogen, Alameda CA) non-tumor bearing BALB/c mice that received, via IV tail vein injection, either the scL nanoimmunoliposome complex encapsulating a plasmid DNA that expresses the Luciferase gene (scLLUC) (as above), or an equal amount of the free, uncomplexed (naked) plasmid DNA (LUC) (25ug/mouse/dose for both treatments) or were not treated (CTL), were also imaged. Two doses were administered within 24 hours. 48 hours after the last dose the animals were injected with the luciferin substrate (ip administration) at ∼3mg/mouse and the whole animals visualized while under anesthesia. As shown in Figures 17A-17C, there is no signal evident in the mouse that received the naked DNA (Panel B) anywhere except the site of injection in the tail (LUC). (The small amount on the mouth is likely due to licking at the injection site). In contrast, after iv administration of the scL complex carrying the same amount of plasmid DNA (scLLUC) (Panel C), there is moderate to high level of signal nearly throughout the animal. Panel A represents an untreated control. The more intense locations where lymph nodes and the liver are located. This is likely due to the fact that dendritic cells and Kupffer cells do express the transferrin receptor and thus can take up the complex. However, this high level of uptake and expression in normal non-tumor tissue, is unexpected and surprising.

### Example 3

### Optimization of delivery of scL-HCV or scLHK-HCV to hepatocytes in vivo using a mouse model.

The scL or scLHK formulated with lipid A is used in the mouse studies. Twenty to thirty micrograms of DNA are delivered via liposome complexes to replicate mice in liposome complexes via the tail vein using 2-3 inoculations over a 24 hour period. To track the endocytosis of the liposomes into specific cell types, phospholipids labeled with rhodamine-DOPE are used at 0.05 molar percent of the total lipid (Avanti Polar Lipids, AL). Tissue samples are analyzed by western blot, histological staining and high-resolution imaging of cells are made by flow cytometry 24-48 hours post injection for expression of HCV specific proteins. For histological analyses tissue is either snap frozen in Tissue-Tek medium or formalin fixed. Tissue is sectioned using a cryostat or microtome and stained for specific proteins or cell markers. For paraffin-embedded formalin fixed tissue, sections are deparaffinized in xylene and hydrated in alcohol before washing with PBS. For antigen retrieval, tissue is treated with 10 mM citrate buffer pH 6.0 in a microwave at 100°C for 10 minutes. After cooling and washing, quenching of endogenous peroxidase activity is achieved using 0.3% H₂O₂. Non-specific sites on tissue sections are then blocked with 5% milk or normal serum before incubation with specific antibodies.

For the analysis of hepatocytes by flow cytometry, a perfusion technique has been developed for isolation of total liver cells and resident T cells from mice for flow cytometry analysis. Mice are maintained under anesthetic throughout the procedure so that the blood pressure is initially maintained. This prevents collapse of the veins and thus increases the success of the subsequent cannulation. Mice are anesthetized with isoflourane and kept anesthetized throughout the procedure via a nose cone attachment. The abdomen is cut open to expose the liver, and the intestine is displaced to the mouse's left side to expose the portal vein. A 24G IV infusion set is inserted into the portal vein. The liver is perfused with pre-warmed Liver Perfusion Medium (Gibco; a buffered balanced salt solution formulated to cleanse the liver of blood, prevent clotting and initiate the loosening of cell to cell contact) for 5 mins at 3ml/min. Pre-warmed Liver Digest Medium (Gibco; a qualified Collagenase-Dispase medium for the dissociation of viable liver cells) is then perfused through the liver at 3ml/min for 10-12 mins. The liver is then removed from the mouse and placed on ice. Hepatocytes are removed and purified from cell debris by passing through a 100 µm sieve followed by low speed centrifugation. Using Rhodamine-labeled liposomes, cells can be directly analyzed by flow cytometry to determine the transfection efficiency or cellular localization of the liposome/DNA complexes. The uptake of the complex into hepatocytes is compared using flow cytometry to uptake by spleen cells or the uptake of non-targeted lipoplexes. Cells are isolated from spleen cell debris by low speed centrifugation. Red blood cells are lysed with ACK lysis buffer (Biowhittaker, MD), after washing, cells are passed through a 100 µm cell strainer (Falcon, BD, NJ). Additional tissues are removed to analyze the uptake of DNA into other organs. Specifically, kidney, heart and lung tissue are removed and protein extracts analyzed by western blot.

### A. Dose Optimization of scL-HCV or scLHK-HCV for In Vivo Efficacy

BALB/c mice are used to determine the optimal biological dose (OBD) to achieve good immune responses in the absence of persistent liver enzyme elevations. As controls, free (uncomplexed) plasmid, modified liposomes carrying empty vector and non-recombinant adenovirus lacking HCV genes are used. Five animals per group are used. The highest dose administered is about 50 µg. Groups of mice receive liposome/DNA complexes containing 1, 5, 10, 20, and 50 µg/injection (prepared as described in Example 1) by i.v. injection. They receive inoculations at 0 and 4 weeks followed by an adenovirus boost at 10 weeks. Four weeks after this boost, livers and spleens are analyzed by flow cytometry and *in vitro* tests to determine the characteristics and specificity of the T cells induced through immunization as described above. To simplify this experiment, control animals receive only the highest doses of DNA and adenovirus. Animals are monitored for liver enzymes and histological changes in the liver. Once the OBD has been established, this dose is used for each injection while the number of injections is varied from 1 to 5 at four week intervals followed by an adenovirsus boost 6 weeks after the last injection. Four weeks later liver and spleen are analyzed as above to determine the number of injections that results in the optimal response.

### B. Cross reactivity of induced T cell responses.

Although the predominant HCV genotype in the US is 1a, there is a need to address the recognition of viral epitopes from more than one HCV genotype in vaccine development. The cross reactivity of induced immune responses is assessed in mice using overlapping peptides corresponding to consensus sequences from genotypes 1b and 2a. A complete set of overlapping 18 mer peptides corresponding to the 1a H77 genome sequence has been provided by the NIH AIDS Research and Reference Reagent Program. This same program has available a similar set of overlapping peptides corresponding to the 1b J4 genotype. The type 2 peptide set was purchased from Mimotopes (Washington, NC). In order to better assess the cross reactivity that may be occurring in patient populations, these analyses are performed using the HLA-A2 transgenic mice described above. These mice express the HLA-A2 MHC molecule and can present HLA-A2 epitopes. The responses to peptide pools representing different genotypes are assessed using Elispot assays and are performed in parallel with peptides corresponding to the 1a H77 sequence that are used for immunization. If no cross reactivity is observed, a tri-valent vaccine is to be tested using nanolipoplexes containing DNA plasmids corresponding to all 3 genotypes followed by a boost using 3 rAd each corresponding to the three different genotypes. Immune responses to each individual genotype are assessed and compared to responses obtained when using the individual genotype sequences alone.

### C. Safety studies in vivo

The Mouse LD₁₀ of the scL-HCV or scLHK-HCV is based on the OBD determined as described above. Starting with the OBD, increasing doses of plasmid DNA (up to 100 µg), are injected once a week for five weeks. As the ratio of components in the complex is set, increasing the amount of plasmid DNA also increases the amount of TfRscFv and Lip or Lip-HK in the complex (prepared as in Example 1). The scL-HCV or scLHK-HCV are injected i.v., i.p., i.m. or i.d. into male and female 4-6 week old BALB/c mice, 10 mice/group. The weights of the animals are assessed twice weekly and they are monitored daily for signs of toxicity (e.g., wasting). Animals receiving free plasmid DNA or unliganded liposome-DNA are used as controls. To assess short-term toxicity, half of the mice are sacrificed two days after the last injection. The remainder are sacrificed two weeks after the last dose to assess long-term toxicity. Various organs and tissues including liver, gonads, lung, pancreas, kidney, heart, skin, spleen, brain, gut, and bone marrow are examined histologically. Liver enzyme levels are also monitored on a weekly basis (Antech Diagnostics). Tissue samples from organs are snap frozen and stored at -70C for extraction of DNA. PCR analysis is performed on all tissue samples to assess biodistribution of the scLHK-HCV and persistence of the DNA in all tissues after inoculations. Primers specific for the HCV genes are available.

Once the LD₁₀ has been established for the DNA/liposome complexes, the dose one step below is used to treat male and female BALB/c mice in combination with increasing doses of recombinant adenovirus. Each group of 10 mice receives 1 or 3 i.v., i.p., i.m., or i.d. injections/week for 5 weeks of scL-HCV or scLHK-HCV (prepared as in Example 1) followed by increasing doses of recombinant adenovirus (10⁸ to 10⁹ infectious units) administered subcutaneously (s.c.) twice weekly for 3 weeks. As above, half of the mice are sacrificed 2 days after the last dose to assess short-term responses, half are sacrifices after 2 weeks to assess long-term responses. Organs and tissues including gonads, lung, pancreas, kidney, heart, skin, spleen, brain, gut, and bone marrow are analyzed histologically, with particular emphasis given to liver histology. Blood is taken and liver enzymes assessed weekly during this study. The spleen is removed and analyzed for T cell responses to assess whether any tolerance induction has occurred in these animals.

Plasma pharmacokinetic analysis and biodistribution studies are performed in male and female nude mice. The animals receive one i.v., i.p., i.m., or i.d. injection of scL-HCV or scLHK-HCV complex (prepared as in Example 1) at the OBD. Blood samples are obtained via cardiac puncture under anesthesia in tubes containing sodium heparin as an anticoagulant at pre-dose and at 5 min, 15 min, 30 min, 1 h, 2 h, 4 h, 8 h, 24 h, 48 and 72 hr after complex administration. The mice are euthanized and liver, pancreas, gonads, spleen, kidney, lung, heart, lymph nodes, and brain are rapidly excised, rinsed in ice-cold normal saline and snap frozen on dry-ice. Blood samples are centrifuged at 3000 rpm for 10 min at 4°C to separate the plasma from the cells. Both plasma and tissue samples are stored at -80°C.

HCV plasmid concentrations in plasma, and tissue samples are determined. Briefly, plasmid DNA is isolated from plasma samples using the phenol-chloroform extraction method and from tissue samples using a DNA extraction kit (Stratagene, La Jolla, CA). The HCV concentration standards are prepared by adding known amounts of plasmid in blank plasma or blank tissue samples, followed by extraction. The extracts are then analyzed by agarose gel, blotting and hybridization. Hybridization is performed using the Gene image Alkaline Phosphatase DNA Labeling and Detection-System (Amersham) using the HCV sequence as the probe. A 10- to 50-fold excess of the probe is used to ensure saturation of all bands. The autoradiographs are scanned and quantified using IMAGEQUANT^{®} software (Molecular Dynamics) and the amounts of plasmid DNA in various samples calculated by comparison to the standards. Alternatively, the probe is 5'- end labeled with γ³² P-ATP or nested PCR specific for the HCV is performed. Plasma pharmacokinetic parameters are assessed by standard methods (see, *e.g.,* Gokhale, P.C., et al., Gene Therapy 4: 1289-1299 (1997)).

Assessing the safety and efficacy of liver targeting in primates.

Dose response studies are performed in rhesus macaques with scL-HCV or scLHK-HCV (prepared as in Example 1) and adenovirus recombinants. The immunization systems and dosages that induce the best HCV-specific T cells carrying specific memory markers are used as a starting dose in the rhesus model. Rhesus macaques are inoculated with scL-HCV or scLHK-HCV and boost with up to 10¹² infectious units of recombinant adenovirus delivered s.c. The proposed vaccination regimen consists of a DNA prime and boost at 0 and 3 months, followed by adenovirus at 6 months. Control groups are inoculated with scL-HCV or scLHK complexed with empty vector free plasmid DNA and non-recombinant adenovirus.

The first study uses 4 groups (3 vaccine and 1 control), two animals per group. Groups 1-3 receive increasing doses of nanocomplex scL-HCV or scLHK-HCV while maintaining rAd inoculation at a level determined from the dose optimization studies in mice described above. Group 4 is the control group, receiving scL or scLHK-empty vector followed by a boost with non-recombinant adenovirus. If a plateau in responses is observed, immunization studies are performed using the 4 remaining animals at the scL-HCV or scLHK-HCV dose in the center of the plateau and increase the rAd dose by 0.5 log₁₀. (2 groups of animals, 2 animals per group, 2 increased doses of rAd). Data from the control groups in study 1 provide control data for these animals. If a plateau is not observed, and immune responses increase relative to dose escalation with minimal ALT elevations, a second study is performed in the remaining 4 animals using increased doses of scLHK-HCV.

In vaccinated macaques, evidence of specific immunity and memory T cells are assessed, both in the peripheral blood and in the liver using Elispot, flow cytometry,and histological analyses. Percutaneous liver biopsies are performed twice per months; half of the tissue sample is placed in culture medium, one-quarter snap frozen in liquid nitrogen and one-quarter fixed in formaldehyde. Immunohistological analyses of liver sections are performed to distinguish the numbers, types and location of lymphocytes present in the liver at 2-4 week intervals following each inoculation. Expression of CD95, CD45RA/CD45RO, CD62L and CD28, are assessed, which, in particular, seems to be a useful marker in the identification of memory T cell subsets in this species.

The haplotypes of the rhesus macaques are determined, and using T cell epitopes identified, in the peripheral blood. Tetramers are made and used to show specificity of memory T cells by flow cytometry analysis as described above for mouse T cell analyses. With a known T cell epitope and multicolor flow cytometry phenotypes, cytokines and cytolytic molecules associated with T cells can be evaluated using a very small number of cells. Blood is analyzed for ALT elevations and tissues are stained for markers of apoptosis to determine if the cells in the liver undergo apoptosis due to the action of cytotoxic T lymphocytes (CTL) specific for HCV antigens induced during priming.

### E. Treatment of chronic chimpanzees with liposome/DNA complexes

The scL-HCV or scLHK-HCV/adenovirus vaccine is used as a therapeutic vaccine in chronic chimpanzees. Four chronically infected chimpanzees are all initially infected with the single sequence monoclonal virus corresponding to the genotype 1a (H77 strain). These animals have been chronically infected for 3.5-5 years. HCV is an RNA virus and therefore the genome undergoes mutation during replication. Virus from animals persistently infected following inoculation with this same monoclonal stock accumulates mutations after approximately 6 months of infection, probably as a result of immune pressure. Sequence data for virus isolated from Ch6412 at 38 weeks post infection has been prepared. Analysis of the complete coding region revealed 9 amino acid changes, 6 of which were located in the NS3, NS5A and NS5B regions. Despite these mutations in the circulating virus genome, the wild type sequences are used in the therapeutic vaccine in these animals. Using the wild type sequence that corresponds to a number of well conserved epitopes is effective at reducing viral titers. As a primary endpoint, reduction in viral RNA titers is assessed in the serum of each animal relative to pre-treatment levels, as secondary endpoints. Increased T cell activity is measured in the liver and increases in intrahepatic cytokine mRNA levels. Decreases in RNA titers greater than 0.5 log₁₀ and greater than 2-fold increases in T cell numbers or cytokine mRNA levels, compared to pre treatment levels, are considered significant. Two animals are inoculated with the therapeutic vaccine and two animals with vector controls.

For the therapeutic vaccine, scL-HCV or scLHK-HCV (prepared as in Example 1) is delivered at the OBD i.v., 3 times at 3-week intervals, or empty DNA vector for the control animals. Four weeks after the last DNA inoculation (week 13), recombinant adenovirus are administered (10¹² infectious units i.v.) expressing these same HCV antigens or non-recombinant virus. Animals will be followed for up to 6 months. Throughout the study, biopsies are obtained bi-weekly and blood (20-50ml) obtained weekly, animals are monitored for serum ALT elevations, HCV RNA titers and intrahepatic T cell infiltration. HCV RNA titers are determined using real-time, RT-PCR assay using primers and probes that target the 5'UTR and core regions of HCV. Evidence of enhanced specific immunity and memory T cells are examined, both in the peripheral blood and in the liver using Elispot, flow cytometry and histological analyses. Percutaneous liver biopsies are performed by Klatskin technique; half of the tissue sample is placed in culture medium, one-quarter snap frozen in liquid nitrogen and one-quarter fixed in formalin.

### F. Analysis of fresh liver tissue

Lymphocytes are isolated from liver biopsies by mechanical homogenization, followed by washing and counting. Cells are stained with antibodies to CD4, CD8, CD45RA/CD45RO, CD95, CD62L and CD28 (BD Pharmingen) and numbers determined by flow cytometry. Events are acquired using a FACS-Calibur (Becton Dickenson) using the software Cellquest (Becton Dickenson) for analysts using 7AAD (BD Pharmigen) to exclude dead cells. As described for the mouse and rhesus study, tetramers are used to study the function of specific T cells from the treated chimpanzees.

In chimpanzees, MHC haplotypes are known as Patr (Pan Troglodytes) types. Several of these Patr alleles have been identified for chimpanzees and for the chronic animals, Patr alleles and HCV epitope sequences recognized by T cells from each of these animals have been identified. The circulating virus in these animals have incorporated mutations during the persistent infection, possibly in previously identified epitopes. Following detailed analyses of T cell epitopes, new tetramers are synthesized with T cell epitopes for use in flow cytometry analysis. T cell clones are also developed *in vitro* for longer-tem analyses. Lymphocytes are cloned with 100,000 gamma-irradiated PBMCs, 0.01 µg/ml of anti-CD3 and 100 U/ml of 1L-2.

### G. Analysis of snap-frozen liver tissue

Total RNA is isolated from the snap frozen tissue using the RNeasy Mini Kit (Qiagen, CA). RNA is reverse transcribed using the First Strand cDNA Synthesis Kit (Pharmacia) and tested by Real-Time PCR for cytokine mRNA levels using specific primer probe sets for IFN-γ, CD3, CD8, and CD4 (Perkin-Elmer Applied Biosystems, CA). The levels of these cytokines are normalized to an endogenous control (GAPDH) and compared to levels in pretreatment biopsies for each animal. Increases or decreases in cytokine mRNA levels of greater than 2-fold are considered significant.

### H. Analysis of formalin-fixed liver tissue

Formalin fixed tissues are sectioned using a microtome and stained for specific proteins, cell markers or markers of apoptosis to determine if the cells in the liver undergo apoptosis due to the action of CTLs specific for HCV antigens induced during vaccination. Immunohistological analyses are performed on liver sections to distinguish the numbers, types and location of lymphocytes present in the liver for each biopsy. Sections are deparaffinized in xylene and hydrated in alcohol before washing with PBS. For antigen retrieval, tissue is treated with 10 mM citrate buffer pH 6.0 in a microwave at 100°C for 10 minutes. Non-specific sites on tissue sections are then blocked with 5% milk or normal serum before incubation with specific antibodies. Liver biopsies obtained from the same animals before infection (historical stored samples) and prior to treatment are used for comparisons.

### I. Formal Pharmacokinetic and Toxicology Studies in Preparation for Phase I Clinical Trials

Toxicology studies are performed with 1 control group (30 mice) and 2 treated groups (30 mice each, half male and half female) with (low 10x below the OBD or LD₁₀) and high (10 fold above the OBD OR LD₁₀) doses of these scL-DNA or scL-HK-DNA complexes prepared as described in Example 1. The complex is injected starting on day 1 at the dosing schedule determined above. Ten mice from each group are sacrificed 2 days, 3 weeks and 6 weeks after the last injection. Body weight and food intake are monitored daily. Clinical pathology (urine hematology, clinical chemistry, specifically liver enzymes) and histopathology on control and treated groups are evaluated. The following organs are included: injection site, brain, pancreas inguinal lymph nodes, bone marrow (histo and smears), liver, kidney, testes/ovaries, spleen, lung heart.

Biodistribution Study: This study includes one control (10 mice) and four treated groups (10 mice each) at the highest dose with 4 time points (2 days, 2 wks, 4 weeks, and 6 weeks) after a single injection of the complex. Analysis by DNA PCR as above on the following organs in all groups is performed: liver, bone marrow, pancreas, kidney, spleen, injection site, testes/ovaries, inguinal lymph nodes, lung, heart, brain.

### Example 4

### Inducing an Immune Response Against a Viral Antigen in Humans by the Nanoimmunoliposome Prepared by Chemical Conjugation

The present invention also provides methods for inducing an immune response against a viral antigen in humans (as well as other mammals and animals) comprising administering to the individual a ligand-targeted cationic liposome complex comprising nucleic acid molecules encoding viral proteins and interleukins, for example, one or more plasmids containing genes encoding for viral proteins that act as an antigen genes encoding one or more interleukins. The complexes comprising various targeted ligands can be directly chemically conjugated to the surface of the liposomes and prepared essentially as described above in Example 1 as follows:

MPB-liposomes are prepared by the ethanol injection method modified from that described by Campbell MJ (Biotechniques 1995 Jun; 18(6):1027-32). 4-(p-maleimidophenyl)butyrate-DOPE (MPB-DOPE) (Avanti Polar Lipids) is included in the liposome formulation [Lipid A (DOTAP:DOPE, 1:1 molar ratio); Lipid B (DDAB:DOPE, 1:1 molar ratio); Lipid D (DOTAP:Chol, 1:1 molar ratio); and Lipid G (DOTAP:DOPE:Chol, 1:1:1 molar ratio)], to a 5-8% molar of total lipids. In brief, all lipids are solubilized in ethanol and mixed, injected into vortexing pure water of 50-60°C with a Hamilton syringe. The solution is vortexed for a further 10-15 min. The final concentration is 1-2 mM total lipids. 1M HEPES, pH7.5 (pH7.0-8.0) is added to a final concentration of 10-20 mM. As the maleimide group is not stable in aqueous solution with pH >7, the liposomes are suitably prepared in water (pH 5-6.5). The pH can be adjusted to 7.0-8.0 before linking to scFv-SH with 1M HEPES buffer, pH7.0-8.0, to facilitate the post-coating reaction.

The scFv-SH or any antibody or antibody fragment with an-SH group (including Fab' or Mab) is added to the MPB-liposome at a protein/lipid ratio of about 1:1 to about 1:100, suitably about 1:10 to about 1:50 (w:w), more suitably at about 1:20 to about 1:40, e.g. at 1:30. The solution is mixed by gentle rotation (∼20-30 RPM) for 30 min at room temperature, to produce scFv-Lip. The scFv-Lip is used without purification, although it can be purified by Sepharose CL-4B column chromatography. To produce the scFv-Lip-DNA complex, plasmid DNA is diluted in water if necessary and added to the scFv-Lip at a DNA/Lipid ratio of about 0.5:1 to about 1:40 (µg total nucleic acid: µg lipid), suitably about 1:5 to about 1:20 (µg total nucleic acid: µg lipid), e.g., at 1:10 (µg total nucleic acid: µg lipid) and the mixture is inverted for 5-10 seconds or for larger volumes rotated at 20-30 RPM for 1-2 minutes. The final mixture is kept at room temperature for 10-15 minutes, gently inverting again for 5-10 seconds after approximately 5 minutes. For use *in vivo* 50% dextrose or 50% sucrose is added to a final concentration of 5-20% (V:V) and mixed by gentle inversion for 5-10 seconds or for larger volumes rotated at 20-30 RPM for 1-2 minutes. The scFv-Lip-DNA is used without purification, although it can be purified by Sepharose CL-4B column chromatography. 80-100% of the scFv (or any antibody with an -SH group) is expected to be conjugated to the liposome.

Prior to association/encapsulation with the complex, the plasmids encoding the viral antigens and the interleukin are mixed together at a molar ratio of insert to insert of 0.1 mole of one or more nucleic acid molecules encoding one or more viral proteins, to 10 mole of one or more nucleic acid molecules encoding one or more interleukins; to 10 mole of one or more nucleic acid molecules encoding one or more viral proteins, to 0.1 mole of one or more nucleic acid molecules encoding one or more interleukins. For example, a ratio of 1 mole of insert of one or more nucleic acid molecules encoding one or more viral proteins, to 1 mole of insert of one or more nucleic acid molecules encoding one or more interleukins is used.

If the disease to be vaccinated against is HIV, the plasmid DNA used is suitably one that contains the gene(s) coding for one or more of the envelope proteins (Vanniasinkam.T and Ertl, H.C. (2005): Adenoviral gene delivery for HIV-1 vaccination. Current Gene Therapy, 5:203-212.; Ferrantelli, F. and Ruprecht, R.M. (2002): Neutralizing antibodies against HIV -- back in the major leagues? Current Opinion in Immunology, 14:495-502.) and/or Nef proteins (Vanniasinkam, T. and Ertl, H.C. (2005): Adenoviral gene delivery for HIV-1 vaccination. Current Gene Therapy, 5:203-212.; Libhterfeld, M., Yu, X.G., Cohen, D., Addo, M.M., Malenfant, J., Perkins, B., Pae, E., Johnston, M.N., Strick, D., Allen, T.M., Rosenberg, E.S., Korber, B., Walker, B.D., and Altfeld, M. (2004): HIV-1 Nef is preferentially recognized by CD8 T cells in primary HIV-1 infection despite a relatively high degree of genetic diversity. AIDS, 18:1383-1392.); and/or gp 140 (Smith, S.M. (2002): HIV vaccine development in the nonhuman primate model of AIDS. Journal of Biomedical Science, 9:100-111.). The protein(s) coded by this DNA acts as the antigen to induce the immune response. In addition, the same complex suitably-comprises a plasmid containing a gene encoding for any interleukin, suitably interleukin 2, and/or interleukin 12 and/or interleukin 15.

If the disease to be vaccinated against is Influenza, the plasmid DNA used is suitably one that contains the gene(s) coding for Hemagglutin (HA) (Tamura, S., Tanimoto, T., and Kurata, T. (2005): Mechanisms of broad cross-protection provided by influenza virus infection and their application to vaccines. Japanese Journal of Infectious Diseases, 58:195-207.; Cox, M.M. (2005): Cell-based protein vaccines for influenza. Current Opinion in Molecular Therapeutics, 7:24-29.; Ze, C., Kurata, T., and Tamura, S. (2000): Identification of effective constituents of influenza vaccine by immunization with plasmid DNAs encoding viral proteins. Japanese Journal of Infectious Diseases, 53:219-228.), and/or Neuraminidase (NA) (Tamura, S., Tanimoto, T., and Kurata, T. (2005): Mechanisms of broad cross-protection provided by influenza virus infection and their application to vaccines. Japanese Journal of Infectious Diseases, 58:195-207.; Cox, M.M. (2005): Cell-based protein vaccines for influenza. Current Opinion in Molecular Therapeutics, 7:24-29.; Ze, C., Kurata, T., and Tamura, S. (2000): Identification of effective constituents of influenza vaccine by immunization with plasmid DNAs encoding viral proteins. Japanese Journal of Infectious Diseases, 53:219-228.), and/or the nucleocapsid (Cox, M.M. (2005): Cell-based protein vaccines for influenza. Current Opinion in Molecular Therapeutics, 7:24-29.) and/or Matrix protein M2 (Cox, M.M. (2005): Cell-based protein vaccines for influenza. Current Opinion in Molecular Therapeutics, 7:24-29.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is SARS, the plasmid DNA used is suitably one that contains the gene(s) coding for the spike glycoprotein, and/or the nucleocapsid and/or structural protein S1, and/or structural protein M, and/or structural protein N (Weiss, S.R. and Navas-Martin, S. (2005): Coronavirus pathogenesis and the emerging pathogen severe acute respiratory syndrome coronavirus. Microbiology & Molecular Biology Reviews, 69:635-664.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is bird flu (also known as H5N1), the plasmid DNA used is suitably one that contains the gene(s) coding for Hemagglutin (Wong, S.S. and Yuen, K.Y. (2006): Avian influenza virus infections in humans. Chest, 129:156-168.; Stephenson, I., Bugarini, R., Nicholson, K.G., Podda, A., Wood, J.M., Zambon, M.C., and Katz, J.M. (2005): Cross-reactivity to highly pathogenic avian influenza H5N1 viruses after vaccination with nonadjuvanted and MF59-adjuvanted influenza A/Duck/Singapore/97 (H5N3) vaccine: a potential priming strategy. Journal of Infectious Diseases, 191:1210-1215.; Treanor, J.J., Wilkinson, B.E., Masseoud, F., Hu-Primmer, J., Battaglia, R., O'Brien, D., Wolff, M., Rabinovich, G., Blackwelder, W., and Katz, J.M. (2001): Safety and immunogenicity of a recombinant hemagglutinin vaccine for H5 influenza in humans. Vaccine, 19:1732-1737.). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is Ebola, Marburg or any of the filoviridae, the plasmid DNA used is suitably one that contains the gene(s) coding for VP24, and/or VP30, and/or VP35, and/or VP40. and/or sGP, and/or the glycoprotein, and/or the nucleoprotein (Hart, M.K. (2003): Vaccine research efforts for filoviruses. International Journal for Parasitology, 33:583-595.; Wilson, J.A., Bosio, C.M., and Hart, M.K. (2001): Ebola virus: the search for vaccines and treatments. Cellular & Molecular Life Science, 58:1826-1841.). The protein(s) coded by this DNA acts as the antigen to induce the immune response. In addition, the same complex suitably comprises a plasmid containing a gene encoding for interleukin 2, and/or interleukin 12 and/or interleukin 15.

If the disease to be vaccinated against is Hepatitis B, the plasmid DNA used is suitably one that contains the gene(s) coding for Hepatitis B surface antigen (HBsAg) (Hanke, T. (2006): On DNA vaccines and prolonged expression of immunogens. European Journal of Immunology, 36:806-809.; Zuckerman, J.N. (2006): Vaccination against hepatitis A and B: developments, deployment and delusions. Current Opinion in Infectious Diseases, 19:456-459.), and/or the nucleocapsid (HBc) (Michel, M.L. and Mancini-Bourgine, M. (2005): Therapeutic vaccination against chronic hepatitis B virus infection. Journal of Clinical Virology, 34 Suppl 1:S108-S114.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is West Nile Virus, Dengue Fever, Yellow Fever, or any of the flaviviridae, the plasmid DNA used is suitably one that contains the gene(s) encoding for E-glycoprotein, the protein which elicits most of the virus-neutralizing antibodies (*see.* Sampathkumar, P., "West Nile Virus: Epidemiology, Clinical Presentation, Diagnosis and Prevention," Mayo Clinic Processings 78:1137-1144 (2003); King, N.J.C., "Immunopathology of Flavivirus Infections, Immunology," Immunology and Cell Biology 85:33-42 (2007)). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

The size of the final complex prepared as above is suitably between about 50 and 500 (nm) with a positive zeta potential as determined by dynamic light scattering using a Malvern ZETASIZER^{®} 3000 of a Malvern ZETASIZER^{®} NANO-ZS. This size is small enough to efficiently pass through the capillary bed and reach the target APC cells.

The complexes, prepared as above, are used as either a prophylactic or a therapeutic vaccine. For prophylaxis, the ligand-tar.geted cationic liposome complex nanolipoplex vaccine as prepared above is injected intravenously into mammals, e.g., humans that have no prior exposure to the viral disease. Injections are done at 0, 4 and 8 wks using the complex. The complex used to vaccinate the subject is suitably made at ratios of 0.33ug: 10ug:1ug (TfRscFv:Lip:DNA), with 5% Dextrose as an excipient. The total amount of DNA in the complex is 0.01 to 10 mg/kg/injection. A suitable amount is 0.164 mg/kg/injection which is 7-12 mg DNA/Injection, based upon the weight of the subject. The prepared complex is injected either into a 250m15% Dextrose bag for i.v. infusion, or injected as a bolus by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

For therapeutic use, the ligand-targeted cationic liposome complex nanolipoplex vaccine prepared as above is injected intravenously into mammals, e.g., humans that are chronically infected with the viral disease (e.g. Hepatitis B), or who are acutely exposed to the viral disease (e.g. Ebola, SARS, H5N1, Smallpox, West Nile Virus). The complex, comprising an amount of DNA in the same range as for prophylactic use, is delivered i.v. in 250 ml of 5% Dextrose solution, or as a bolus, 3 times at weeks 0, 4 and 8, by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

### Example 5

### Inducing an Immune Response Against a Viral Antigen in Humans by the Nanoimmunoliposome Complex Prepared by Simple Mixing

The present invention also provides methods for inducing an immune response against a viral antigen in humans (as well as other mammals and animals) comprising administering to the individual a ligand-targeted cationic liposome complex comprising a nucleic acid molecules encoding viral proteins and interleukins, for example, one or more plasmids containing genes encoding for viral proteins that act as an antigen genes encoding one or more interleukins. The complexes comprising various targeting ligand directly complexed/associated with, but not chemically conjugated to, the surface of the liposomes is prepared essentially as described above in Example 1 as follows:

The liposomes [Lipid A (DOTAP:DOPE, 1:1 molar ratio); Lipid B (DDAB:DOPE, 1:1 molar ratio); Lipid D (DOTAP:Chol, 1:1 molar ratio); and Lipid G (DOTAP:DOPE:Chol, 1:1:1 molar ratio)], are prepared by the ethanol injection method modified from that described by Campbell, MJ (Biotechniques 1995 Jun; 18(6): 1027-32). In brief, all lipids are solubilized in ethanol and mixed, injected into vortexing pure water of 50-60°C with a Hamilton syringe. The solution is vortexed for a further 10-15 min. The final concentration is 1-2mM total lipids.

The TfRscFv or any antibody or antibody fragment (including any scFv, Fab' or Mab) -immunoliposome complexes are prepared by mixing the TfRscFv with liposome composition A (or any of the liposome compositions given above) at defined ratios of single chain protein to liposome and DNA in the mixed complex. The preparation of the complexes is in accordance with the following general procedure. The appropriate amount of 2 mM liposome (A-H described above) is mixed with any water (e.g., DI water) required to give a desired volume and gently inverted 10 times to mix, or for lager volumes rotated at 20-30 RPM for 1-2 minutes. To the liposome-water mixture, the appropriate amount of TfRscFv is added to give the desired ratio and mixed by gentle inversion for about 5-10 seconds or for larger volumes rotated at 20-30 RPM for 1 minute. This mixture is kept at room temperature for 10-15 minutes (again inverted gently for 5-10 seconds after approximately 5 minutes). At the same time, the appropriate amount of DNA is mixed by inversion for 5-10 seconds, or for larger volumes rotated at 20-30 RPM for 1-2 minutes, with any water required to give a desired volume. Typically, for *in vivo* use, it is desirable to provide about 5 µg to about 100 mg of DNA per injection. The DNA solution is quickly added to the TfRscFv-liposome solution and the mixture is inverted for 5-10 seconds or for larger volumes rotated at 20-30 RPM for 1-2 minutes. The final mixture is kept at room temperature for 10-15 minutes, gently inverting again for 5-10 seconds after approximately 5 minutes. For use *in vivo* 50% dextrose or 50% sucrose is added to a final concentration of 5-20% (V: V) and mixed by gentle inversion for 5-10 seconds, or for larger volumes rotated at 20-30 RPM for 1-2 minutes. A specific example at a suitable ratio of 1:30 (TfRscFv:liposome, w:w) and 1:14 (µg DNA:n mole total Lipid) is as follows. For 40 µg of DNA in a final volume of 800 µl, mix 183 µl water with 280 µl of 2 mM liposome solution. Add 34 µl of TfRscFv (with a concentration of 0.4 µg/ml). Mix 183 µl water with 40 µl of 1 µg/l µl DNA. Add 80 µl of 50% Dextrose as the last step.

The Tf-Lip-DNA complex is prepared according to the *in vivo* formulation described previously. Xu et al., Human Gene Therapy, 10(18):2941-52 (1999). Transferrin-liposome-mediated systemic p53 gene therapy in combination witch radiation results in regression of human head and neck cancer xenografts. For a typical preparation with optimized *in vivo* formulation, 25 ml of Tf (5 mg/ml, iron-saturated holo-transferrin; Sigma, St. Louis, MO) and 50 ml of Lip (2 mM total lipids) plus 75 ml of water are mixed in a polypropylene tube and held for 5-15 min at room temperature with frequent rocking, or for larger volumes rotated at 20-30 RPM for 1-2 minutes. Ten micrograms of plasmid DNA in 120 ml of 20 mM HEPES buffer, pH 7.4, is added to the tube, mixed immediately and thoroughly by gentle inversion 10 times, or for larger volumes rotated at 20-30 RPM for 1-2 minutes, and held for 10-20 min at room temperature with frequent rocking. Thirty microliters of 50% dextrose solution is then added to the tube, mixed immediately and thoroughly by gentle inversion 10 times, or for larger volumes rotated at 20-30 RPM for 1-2 minutes, and held at room temperature for 10-15 minutes. The final DNA:lipid:Tf ratio is 1:10:12.5 (mg/nmol/mg). The HEPES buffer can be replaced by water.

### A. Preparation of Lip-HoKC

When the {K[K(H)KKK]₅-K(H)KKC} peptide (HoKC) is included in the complex, the cationic liposomal formulations A (DOTAP:DOPE at a 1:1 molar ratio), B (DDAB:DOPE at 1:1), G (DOTAP:DOPE:cholesterol at 1:1:1) and H (DDAB:DOPE:cholesterol at 1:1:1) (or any of the liposomes formulations given above) are prepared using the ethanol injection method as described above. Each liposome formulation also includes MPB-DOPE at 5 molar percent of total lipid. Since the HoKC peptide carries a terminal cysteine, MPB-DOPE is included in all of the liposome compositions to allow conjugation of peptide to the liposome. The Lip-HoKC liposomes are prepared using a coupling reaction between the cationic liposomes carrying the maleimide group (Lip-MPB) and the peptide as follows. An aliquot of 0.1 mmol of the peptide with a free thiol group on cysteine is added to 2 mmol of Lip-MPB in 10 mM HEPES, pH 7.4, solution and rotated at room temperature (20-30 r.p.m.) for 2 h. The resulting Lip-HoKC has a lipid concentration of 1.4 mM.

When the liposome comprises HoKC, the full complex is formed in a manner identical to that used to produce the TfRscFv-Lip-DNA complex without HoKC. Here also the TfRscFv or any antibody or antibody fragment (including Fab' or Mab) is mixed with Lip-HoKC (by gently inversion 10 times or for larger volumes rotated at 20-30 RPM for 1-2 minutes) at a specific ratio, and incubated at room temperature for 10-15 min. DNA is then added to the TfRscFv-Lip-HoKC solution, mixed by gently inversion 10 times for for larger volumes rotated at 20-30 RPM for 1-2 minutes, and again incubated at room temperature for 10-15 min, after which dextrose or sucrose is added to a final concentration of 5-20%, mixed by gently inversion 10 times, or for larger volumes rotated at 20-30 RPM for 1 minute, and incubated at room temperature for 10-15 minutes. The ratio of the TfRscFv:LipA-HoKC:DNA in the complex is 0.3 mg:7 nmol:1 mg.

Prior to encapsulation in the complex, the plasmids encoding the viral antigens wand the interleukin are mixed together at a molar ratio of insert to insert of 0.1 mole of one or more nucleic acid molecules encoding one or more viral proteins, to 10 mole of one or more nucleic acid molecules encoding one or more interleukins; to 10 mole of one or more nucleic acid molecules encoding one or more viral proteins, to 0.1 mole of one or more nucleic acid molecules encoding one or more interleukins. For examples, a ratio of 1 mole of insert of one or more nucleic acid molecules encoding one or more viral proteins, to 1 mole of insert of one or more nucleic acid molecules encoding one or more interleukins is used.

If the disease to be vaccinated against is HIV, the plasmid DNA used is suitably one that contains the gene(s) coding for one or more of the envelope proteins (Vanniasinkam, T. and Ertl, H.C. (2005): Adenoviral gene delivery for HIV-1 vaccination. Current Gene Therapy, 5:203-212.; Ferrantelli, F. and Ruprecht, R.M. (2002): Neutralizing antibodies against HIV -- back in the major leagues? Current Opinion in Immunology, 14:495-502.) and/or Nef proteins (Vanniasinkam, T. and Ertl, H.C. (2005): Adenoviral gene delivery for HIV-1 vaccination. Current Gene Therapy, 5:203-212.; Lichterfeld, M., Yu, X.G., Cohen, D., Addo, M.M., Malenfant, J., Perkins, B., Pae, E., Johnston, M.N., Strick, D., Allen, T.M., Rosenberg, E.S., Korber, B., Walker, B.D., and Altfeld, M. (2004): HIV-1 Nef is preferentially recognized by CD8 T cells in primary HIV-1 infection despite a relatively high degree of genetic diversity. AIDS, 18:1383-1392.); and/or gp 140 (Smith,S.M. (2002): HIV vaccine development in the nonhuman primate model of AIDS. Journal of Biomedical Science, 9:100-111.). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is Influenza, the plasmid DNA used is suitably one that contains the gene(s) coding for Hemagglutin (HA) (Tamura, S., Tanimoto, T., and Kurata, T. (2005): Mechanisms of broad cross-protection provided by influenza virus infection and their application to vaccines. Japanese Journal of Infectious Diseases, 58:195-207.; Cox, M.M. (2005): Cell-based protein vaccines for influenza. Current Opinion in Molecular Therapeutics, 7:24-29.; Ze, C., Kurata, T., and Tamura, S. (2000): Identification of effective constituents of influenza vaccine by immunization with plasmid DNAs encoding viral proteins. Japanese Journal of Infectious Diseases, 53:219-228.), and/or Neuraminidase (NA) (Tamura, S., Tanimoto, T., and Kurata, T. (2005): Mechanisms of broad cross-protection provided by influenza virus infection and their application to vaccines. Japanese Journal of Infectious Diseases, 58:195-2.07.; Cox, M.M. (2005): Cell-based protein vaccines for influenza. Current Opinion in Molecular Therapeutics, 7:24-29.; Ze, C., Kurata, T., and Tamura, S. (2000): Identification of effective constituents of influenza vaccine by immunization with plasmid DNAs encoding viral proteins. Japanese Journal of Infectious Diseases, 53:219-228.), and/or the nucleocapsid (Cox, M.M. (2005): Cell-based protein vaccines for influenza. Current Opinion in Molecular Therapeutics, 7:24-29.) and/or Matrix protein M2 (Cox, M.M. (2005): Cell-based protein vaccines for influenza. Current Opinion in Molecular Therapeutics, 7:24-29.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is SARS, the plasmid DNA used is suitably one that contains the gene(s) coding for the spike glycoprotein, and/or the nucleocapsid and/or structural protein S 1, and/or structural protein M, and/or structural protein N (Weiss, S.R. and Navas-Martin, S. (2005): Coronavirus pathogenesis and the emerging pathogen severe acute respiratory syndrome coronavirus. Microbiology & Molecular Biology Reviews, 69:635-664.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is bird flu (also known as H5N1), the plasmid DNA used is suitably one that contains the gene(s) coding for Hemagglutin (Wong, S.S. and Yuen, K.Y. (2006): Avian influenza virus infections in humans. Chest, 129:156-168.; Stephenson, I., Bugarini, R., Nicholson, KG, Podda, A., Wood, J.M., Zambon, M.C., and Katz, J.M. (2005): Cross-reactivity to highly pathogenic avian influenza H5N1 viruses after vaccination with nonadjuvanted and MF59-adjuvanted influenza A/Duck/Singapore/97 (H5N3) vaccine: a potential priming strategy. Journal of Infectious Diseases, 191:1210-1215.; Treanor, J.J., Wilkinson, B.E., Masseoud, F., Hu-Primmer, J., Battaglia, R., O'Brien, D., Wolff, M., Rabinovich, G., Blackwelder, W., and Katz, J.M. (2001): Safety and immunogenicity of a recombinant hemagglutinin vaccine for H5 influenza in humans. Vaccine, 19:1732-1737.). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is Ebola, Marburg or any of the filoviridae, the plasmid DNA used is suitably one that contains the gene(s) coding for VP24, and/or VP30, and/or VP35, and/or VP40. and/or sGP, and/or the glycoprotein, and/or the nucleoprotein (Hart, M.K. (2003): Vaccine research efforts for filoviruses. International Journal for Parasitology, 33:583-595.; Wilson, J.A., Bosio, C.M., and Hart, M.K. (2001): Ebola virus: the search for vaccines and treatments. Cellular & Molecular Life Sciences, 58:1826-1841.). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is Hepatitis B, the plasmid DNA used is suitably one that contains the gene(s) coding for Hepatitis B surface antigen (HBsAg) (Hanke, T.(2006): On DNA vaccines and prolonged expression of immunogens. European Journal of Immunology, 36:806-809.; Zuckerman, J.N. (2006): Vaccination against hepatitis A and B: developments, deployment and delusions. Current Opinion in Infectious Diseases, 19:456-459.), and/or the nucleocapsid (HBc) (Michel, M.L and Mancini-Bourgine, M. (2005): Therapeutic vaccination against chronic hepatitis B virus infection. Journal of Clinical Virology, 34 Suppl 1:S108-S114.). The protein(s) coded by this DNA act as the antigen to induce the immune response. In addition, the same complex suitably comprises a plasmid containing a gene encoding for any interleukin, suitably interleukin 2, and/or interleukin 12 and/or interleukin 15.

If the disease to be vaccinated against is West Nile Virus, Dengue Fever, Yellow Fever, or any of the flaviviridae, the plasmid DNA used is suitably one that contains the gene(s) encoding for E-glycoprotein, the protein which elicits most of the virus-neutralizing antibodies (*see* Sampathkumar, P., "West Nile Virus: Epidemiology, Clinical Presentation, Diagnosis and Prevention," Mayo Clinic Processings 78:1137-1144 (2003); King, N.J.C., "Immunopathology of Flavivirus Infections, Immunology," Immunology and Cell Biology 85:33-42 (2007)). The protein(s) coded by this DNA acts as the antigen to induce the immune response. In addition, the same complex suitably comprises a plasmid containing a gene encoding for any interleukin, suitably interleukin 2, and/or interleukin 12 and/or interleukin 15.

The size of the final complexes prepared by these methods are between about 50 and 400 (nm) with a positive zeta potential as determined by dynamic light scattering using a Malvern ZETASIZER^{®} 3000 or a Malvern ZETASIZER^{®} NANO-ZS. This size is small enough to efficiently pass through the capillary bed and reach the target APC cells.

The complex, prepared as above, is suitably used as either a prophylactic or a therapeutic vaccine. For prophylaxis, the ligand-targeted cationic liposome complex nanolipoplex vaccine as prepared above is injected intravenously into mammals, e.g., humans that have no prior exposure to the viral disease. Intravenous injections are done at 0, 4 and 8 wks using the complex. The complex used to vaccinate the subject is suitably made at ratios of 1:10:12.5 (mg/nmol/mg) (DNA:lipid:Tf) with 5-210% Dextrose for Sucrose as an excipient; 0.33ug:10ug:1ug (TfRscFv:Lip:DNA), with 5-20% Dextrose or Sucrose as an excipient when HoKC is not a component of the complex; and at ratios of 0.3 mg:7 nmol:1 mg (TfRscFv:Lip-HoKC:DNA) with 5% Dextrose or Sucrose when the complex comprises HoKC. The total amount of DNA in the complex is 0.01 to 10 mg/kg/injection. A suitable amount is 0.164 mg/kg/injection which is 7-12 mg DNA/Injection, based upon the weight of the subject. The prepared complex is injected either into a 250ml 5% Dextrose bag for i.v. infusion, or injected i.v. as a bolus by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

For therapeutic use, the ligand-targeted cationic liposome complex nanolipoplex vaccine prepared as above is injected into mammals, e.g., humans that are chronically infected with the viral disease (e.g. Hepatitis B), or who are acutely exposed to the viral disease (e.g. Ebola, SARS, H5N1, Smallpox, West Nile Virus). The complex, comprising an amount of DNA in the same range as for prophylactic use, is delivered i.v. in 250 ml of 5% Dextrose solution, or as a bolus, 3 times at weeks 0, 4 and 8 by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

### Example 6

### Inducing an Immune Response Against a Viral Antigen in Humans by Co-Injection of Two Nanoimmunoliposome Complexes Prepared by Chemical Conjugation

The present invention also provides methods for inducing an immune response against a viral antigen in humans (as well as other mammals and animals) comprising administering to the individual a ligand-targeted cationic liposome complex comprising a nucleic acid molecules encoding viral proteins and interleukins, for example, one or more plasmids containing genes encoding for viral proteins that act as an antigen genes encoding one or more interleukins. The complexes comprising various targeting agents directly chemically conjugated to the surface of the liposomes are prepared as described above in Example 4.

The plasmid DNA(s) that carries the gene(s) that code for the viral protein that serves as the antigen is identical to that described in Example 4. A separate ligand-targeted cationic liposome complex comprising a plasmid DNA containing a gene encoding for, for example, interleukin 2, interleukin 12 and/or interleukin 15 is prepared using the identical procedure and ratios as used with the plasmid DNA(s) that carries the gene(s) that code for the viral protein.

The sizes of the final complexes prepared as above are between about 50 and 500 (nm) with a positive zeta potential as determined by dynamic light scattering using a Malvern ZETASIZER® 3000 or a Malvern ZETASIZER^{®} NANO-ZS. This size is small enough to efficiently pass through the capillary bed and reach the target APC cells.

The complexes, prepared as above, are used as either a prophylactic or a therapeutic vaccine. For use as a vaccine the two complexes are mixed together at a ratio of complex:complex that reflects the molar ratio of the plasmid DNA inserts in each complex. Thus, the ratios of the two complexes are the amount of complex that contains 0.1 mole of one or more nucleic acid molecules encoding one or more viral proteins, to the amount of complex that contains 10 moles of one or more nucleic acid molecules encoding one or more interleukins; to the amount of complex that contains 10 moles of one or more nucleic acid molecules encoding one or more viral proteins, to the amount of complex that contains 0.1 mole of one or more nucleic acid molecules encoding one or more interleukins. For example, a ratio of the amount of complex that contains 1 mole of one or more nucleic acid molecules encoding one or more viral proteins, to the amount of complex that contains 1 mole of one or more nucleic acid molecules encoding one or more interleukins is utilized. The complex comprising the plasmid DNA(s) which encoded the viral protein(s) is added to the complex comprising the plasmid DNA(s) which encode the interleukin(s) and the solution is inverted gently 10 times or is rotated at 20-30 RPM for 1 minute to form the final vaccine for use in the human subjects.

For prophylaxis, the ligand-targeted cationic liposome complex nanolipoplex vaccine as prepared above is injected intravenously into mammals, e.g., humans that have no prior exposure to the viral disease. Injections are done at 0, 4 and 8 wks using the complex. Each of the complexes (that comprise the viral gene(s) and that comprise the interleukin gene(s)) used to form the vaccine administered to the subject is suitably made at ratios of 0.33ug:10ug:1ug (TfRscFv:Lip:DNA), with 5% Dextrose as an excipient. The total amount of DNA in the vaccine is 0.01 to 10 mg/kg/injection. A suitable amount is 0.164 mg/kg/injection which is 7-12 mg DNA/Injection, based upon the weight of the subject. The prepared complex is injected either into a 250ml 5% Dextrose bag for i.v. infusion, or injected i.v. as a bolus by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

For therapeutic use, the ligand-targeted cationic liposome complex nanolipoplex vaccine prepared as above is injected into mammals, e.g., humans that are chronically infected with the viral disease (e.g. Hepatitis B), or who are acutely exposed to the viral disease (e.g. Ebola, SARS, H5N1, Smallpox, West Nile Virus). The complex, prepared and comprising an amount of DNA in the same range as for prophylactic use, is delivered i.v. in 250 ml of 5% Dextrose solution, or as a bolus, 3 times at weeks 0, 4 and 8 by intravenous (IV), intratumoral (IT), intralesional (IL), aerosal, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

### Examples 7

### Inducing an Immune Response Against a Viral Antigen in Humans by Co-Injection of Two N,anoimmunoliposome Complex Prepared by Simple Mixing

The present invention also provides methods for inducing an immune response against a viral antigen in humans comprising simultaneously administering to the individual a ligand-targeted cationic liposome complex comprising a nucleic acid molecules encoding viral proteins and interleukins, for example, one or more plasmids containing genes encoding for viral proteins that act as an antigen genes encoding one or more interleukins. The complexes comprising various ligands directly complexed with, but not chemically conjugated to, the surface of the liposomes (with or without the HoKC peptide) are prepared as described above in Example 5. The plasmid DNA(s) that carries the gene(s) that code for the viral protein that serves as the antigen is identical to that described in Example 5. A separate ligand-targeted cationic liposome complex comprising a plasmid DNA containing a gene encoding for any interleukin, for example, interleukin 2, interleukin 12 and/or interleukin 15, is prepared using the identical procedure and ratios as used with the plasmid DNA(s) that carries the gene(s) that code for the viral protein.

The sizes of the final complexes prepared as above are between about 50 and 400 (nm) with a positive zeta potential as determined by dynamic light scattering using a Malvern ZETASIZER® 3000 or a Malvern ZETASIZER^{®} NANO-ZS. This size is small enough to efficiently pass through the capillary bed and reach the target Apt cellos

For use as a vaccine the two complexes are mixed together at a ratio of complex:complex that reflects the molar ratio of the plasmid DNA inserts in each complex. Thus, the ratios of the two complexes are the amount of complex that contains 0.1 mole of one or more nucleic acid molecules encoding one or more viral proteins, to the amount of complex that contains 10 moles of one or more nucleic acid molecules encoding one or more interleukins; to the amourrt of complex that contains 10 moles of one or more nucleic acid molecules encoding one or more viral proteins, to the amount of complex that contains 0.1 mole of one or more nucleic acid molecules encoding one or more interleukins. For example, a ratio of the amount of complex that contains 1 mole of one or more nucleic acid molecules encoding one or more viral proteins, to the amount of complex that contains 1 mole of one or more nucleic acid molecules encoding one or more interleukins is utilized. The complex comprising the plasmid DNA(s) which encoded the viral protein(s) is added to the complex comprising the plasmid DNA(s) which encode the interleukin(s) and the solution is inverted gently 10 times or is rotated at 20-30 RPM for 1 minute to form the final vaccine for use in the human subjects.

The complex, prepared as above, is used as either a prophylactic or a therapeutic vaccine. For prophylaxis, the ligand-targeted cationic liposome complex nanolipoplex vaccine as prepared above is injected into mammals, e.g., humans that have no prior exposure to the viral disease. Intravenous injections are done at 0,4 and 8 wks using the complex. The complex used to vaccinate the subject is suitably made at ratios of 1 : 10: 12.5 (mg/nmol/mg) (DNA:lipid:Tf) with 5-20% Dextrose or Sucrose as an excipient; 0.33ug:10ug:lug (TfRscFv.Lip.DNA), with 5-20% Dextrose or Sucrose as an excipient when HoKC is not a component of the complex and at ratios of 0.3 mg:7 nmol:1 mg (TfRscFv:Lip-HoKC:DNA) with 5% Dextrose or Sucrose when the complex comprises HoKC The total amount of DNA in the complex is 0.01 to 10 mg/kg/injection. A suitable amount is 0.164 mg/kg/injection which is 7-12 mg DN A/Injection, based upon the weight of the subject. The prepared complex is injected either into a 250ml 5% Dextrose bag for i.v. infusion, or injected i.v. as a bolus by intravenous (IV), intratumoral (IT), intralesional (IL), aerosal, percutaneous, oral, endoscopic, topical, intramuscular (IM), intradermal (ID), intraocular (IO), intraperitoneal (EP), transdermal (TD), intranasal (IN), intracereberal (IC), intraorgan (e.g. intrahepatic), slow release implant, or subcutaneous administration, or via administration using an osmotic or mechanical pump.

For therapeutic use, the ligand-targeted cationic liposome complex nanolipoplex vaccine prepared as above is injected into mammals, e.g., humans that are chronically infected with the viral disease (e.g. Hepatitis B), or who are acutely exposed to the viral disease (e.g. Ebola, SARS, H5N1, Smallpox, West Nile Virus). The complex, comprising an amount of DNA in the same range as for prophylactic use, is delivered i.v. in 250 ml of 5% Dextrose solution, or as a bolus, 3 times at weeks 0, 4 and 8 by intravenous (IV), intratumoral (IT), intralesional (IL), aerosol, percutaneous, oral, endoscopic, topical, intramuscular (IM), intradermal (ID), intraocular (IO), intraperitoneal (IP), transdermal (TD), intranasal (IN), intracereberal (IC), intraorgan (e.g. intrahepatic), slow release implant, or subcutaneous administration, or via administration using an osmotic or mechanical pump.

## Claims

1. A ligand-targeted cationic liposome complex comprising a cationic liposome, a ligand which is an anti-transferrin receptor single chain Fv (TfRscFv), one or more nucleic acid molecules encoding one or more viral antigens, and one or more nucleic acid molecules encoding one or more interleukins selected from IL-6, IL-12 and IL-23, wherein said anti-transferrin receptor single chain Fv (TfRscFv) is directly complexed with, but not chemically conjugated to, said cationic liposome.

2. A ligand-targeted cationic liposome complex according to claim 1, wherein said one or more viral antigens are selected from the group consisting of HIV viral antigens, influenza viral antigens, SARS viral antigens, bird flu viral antigens, Ebola viral antigens, Hepatitis B viral antigens and small pox viral antigens.

3. A ligand-targeted cationic complex according to claim 1 or claim 2, wherein said nucleic acid molecules are encapsulated within said cationic liposome.

4. A ligand-targeted cationic complex according to any preceding claim, wherein the ligand targeted cationic liposome further comprises a peptide comprising a K[K(H)KKK]₅-K(H)KKC (HoKC) (SEQ ID NO: 1) peptide associated with said complex.

5. A ligand-targeted cationic liposome complex according to any preceding claim, wherein said nucleic acid molecules are associated with an inner or outer monolayer of said cationic liposome, and wherein said cationic liposome comprises a mixture of dioleoyltrimethylammonium phosphate with dioleoylphosphatidylethanolamine and cholesterol, a mixture of dioleoyltrimethylammonium phosphate with cholesterol, a mixture of dimethyldioctadecylammonium bromide with dioleoylphosphatidylethanolamine and cholesterol, dioleoyltrimethylammonium phosphate with dioleoylphosphatidylethanolamine, a mixture of dimethyldioctadecylammonium bromide with dioleoylphosphatidylethanolamine, or a mixture of dimethyldioctadecylammonium bromide with cholesterol.

6. A ligand-targeted cationic liposome complex according to claim 1, wherein said anti-transferrin receptor single chain Fv (TfRscFv) and said cationic liposome are present at a ratio in the range of 1:1 to 1:100 (w:w).

7. A ligand-targeted cationic liposome complex according to any preceding claim, wherein said nucleic acid molecules are present at a molar ratio of 0.1 mole:10 mole to 10 mole:0.1 mol (one or more nucleic acid molecules encoding one or more viral antigens: one or more nucleic acid molecules encoding one or more interleukins).

8. The ligand-targeted cationic liposome complex according to any preceding claim, wherein said one or more nucleic acid molecules encoding one or more viral antigens, said one or more nucleic acid molecules encoding one or more interleukins, and said cationic liposome are present at a weight ratio of between 1:1 to 1:40 (µg total nucleic acid:µg liposome).

9. A ligand-targeted cationic liposome complex according to any preceding claim for ligand-targeted delivery for use in inducing an immune response against a viral antigen in a mammal and/or for use in treating or preventing a viral disease in a mammal, and wherein said viral antigen is from a virus selected from the group consisting of influenza virus, HIV virus, SARS virus, bird flu virus, Ebola virus, Hepatitis B virus and small pox virus, and/or, wherein said complex is administered as a bolus or as an infusion via route selected from the group consisting of intravenous administration, oral administration intramuscular administration, intralesional administration, intradermal administration, transdermal administration, intraocular administration, intraperitoneal administration, percutaneous administration, aerosol administration intranasal administration, intraorgan administration, intracereberal administration, topical administration, subcutaneous administration, endoscopic administration, slow release implant and administration via an osmotic or mechanical pump.

10. An anti-transferrin receptor single chain Fv (TfRscFv)-targeted cationic immunoliposome complex for TfRscFv-targeted delivery for use in delivering nucleic acid molecules to professional or unprofessional antigen presenting cells (APCs) of a mammal, comprising a cationic liposome, a TfRscFv, and nucleic acid molecules, wherein the TfRscFv is directly complexed with, but not chemically conjugated to, the cationic liposome,
wherein the APCs are located in the liver, spleen, lymph nodes, gut and/or respiratory tract,
wherein said complex is administered as a bolus or as an infusion via route selected from the group consisting of intravenous administration, oral administration intramuscular administration, intralesional administration, intradermal administration, transdermal administration, intraocular administration, intraperitoneal administration, percutaneous administration, aerosol administration intranasal administration, intraorgan administration, intracereberal administration, topical administration, subcutaneous administration, endoscopic administration, slow release implant and administration via an osmotic or mechanical pump,
wherein said nucleic acid molecules encode, one or more viral antigens selected from the group consisting of HIV viral antigens, Ebola viral antigens, influenza viral antigens, SARS viral antigens, bird flu viral antigens, Hepatitis B viral antigens and small pox viral antigens, and wherein said nucleic acid molecules also encode one or more interleukins selected from the group consisting of IL-6, IL-12 and IL-23.

11. A method of preparing a ligand-targeted cationic immunoliposome complex according to any of claims 1 to 9, which comprises:
preparing a ligand;
mixing the ligand with a cationic liposome to form a ligand-targeted cationic liposome, wherein the ligand is directly complexed with, but not chemically conjugated, to the cationic liposome, and
mixing the ligand-targeted cationic liposome with:
one or more nucleic acid molecules encoding one or more viral antigens; and
one or more nucleic acid molecules encoding one or more interleukins, to form the ligand-targeted-cationic liposome complex,
which complex is optionally used in the manufacture of a medicament for inducing an immune response against a viral antigen in a mammal and/or for treating or preventing a viral disease in a mammal, wherein said viral antigen is from a virus selected from the group consisting of influenza virus, HIV virus, SARS virus, bird flu virus, Ebola virus, Hepatitis B virus and small pox virus, wherein said complex is administered as a bolus or as an infusion via route selected from the group consisting of intravenous administration, oral administration intramuscular administration, intralesional administration, intradermal administration, transdermal administration, intraocular administration, intraperitoneal administration, percutaneous administration, aerosol administration intranasal administration, intraorgan administration, intracereberal administration, topical administration, subcutaneous administration, endoscopic administration, slow release implant and administration via an osmotic or mechanical pump.

12. A pharmaceutical composition comprising a ligand-targeted cationic liposome according to any of claims 1 to 9.

13. A pharmaceutical composition according to claim 12, further comprising a second ligand-targeted cationic liposome according to any of claims 1 to 9.,

14. A pharmaceutical composition according to claim 12 or claim 13, further comprising one or more excipients selected from the group consisting of one or more antibacterials, one or more preservatives, one or more buffers and one or more surfactants.

15. The ligand targeted cationic liposome of any one of claims 1-9, wherein the anti-transferrin receptor scFv and said cationic liposome are present at a ratio in the range 1:20 to 1:40 (w:w), wherein said nucleic acids are present at a molar ratio of 1 mole:1 mole (one or more nucleic acid molecules encoding one or more viral antigens: one or more nucleic acid molecules encoding one or more interleukins), and wherein said one or more nucleic acid molecules encoding one or more viral antigens, said one or more nucleic acid molecules encoding one or more interleukins, and said cationic liposome are present at a weight ratio of 1:10 (µg total nucleic acid: µg liposome).

## Patentansprüche

1. Liganden-gerichteter kationischer Liposomen-Komplex, der Folgendes umfasst: ein kationisches Liposom, einen Liganden, der ein Anti-Transferrinrezeptor-Einzelketten-Fv (TfRscFv) ist, ein oder mehrere Nukleinsäuremoleküle, die ein oder mehrere Virusantigene kodieren, und ein oder mehrere Nukleinsäuremoleküle, die ein oder mehrere Interleukine, die aus IL-6, IL-12 und IL-23 ausgewählt sind, kodieren, wobei das Anti-Transferrinrezeptor-Einzelketten-Fv (TfRscFv) direkt mit dem kationischen Liposom komplexiert ist, doch nicht chemisch damit konjugiert ist.

2. Liganden-gerichteter kationischer Liposomen-Komplex nach Anspruch 1, wobei das eine oder die mehreren Virusantigene aus der Gruppe ausgewählt sind, die aus Folgenden besteht: HIV-Virusantigenen, Influenza-Virusantigenen, SARS-Virusantigenen, Vogelgrippe-Virusantigenen, Ebola-Virusantigenen, Hepatitis-B-Virusantigenen und Pocken-Virusantigen.

3. Liganden-gerichteter kationischer Komplex nach Anspruch 1 oder Anspruch 2, wobei die Nukleinsäuremoleküle im Inneren des kationischen Liposoms eingekapselt sind.

4. Liganden-gerichteter kationischer Komplex nach einem vorstehenden Anspruch, wobei das Liganden-gerichtete kationische Liposom weiter ein Peptid umfasst, das ein K[K(H)KKK]₅-K(H)KKC (HoKC) (SEQ.-ID Nr. 1) Peptid umfasst, das mit dem Komplex verbunden ist.

5. Liganden-gerichteter kationischer Liposomen-Komplex nach einem vorstehenden Anspruch, wobei die Nukleinsäuremoleküle mit einer inneren oder äußeren Monoschicht des kationischen Liposoms verbunden sind und wobei das kationische Liposom Folgendes umfasst: eine Mischung aus Dioleoyltrimethylammoniumphosphat mit Dioleoylphosphatidylethanolamin und Cholesterol, eine Mischung aus Dioleoyltrimethylammoniumphosphat mit Cholesterol, eine Mischung aus Dimethyldioctadecylammoniumbromid mit Dioleoylphosphatidylethanolamin und Cholesterol, Dioleoyltrimethylammoniumphosphat mit Dioleoylphosphatidylethanolamin, eine Mischung aus Dimethyldioctadecylammoniumbromid mit Dioleoylphosphatidylethanolamin oder eine Mischung aus Dimethyldioctadecylammoniumbromid mit Cholesterol.

6. Liganden-gerichteter kationischer Liposomen-Komplex nach Anspruch 1, wobei das Anti-Transferrinrezeptor-Einzelketten-Fv (TfRscFv) und das kationische Liposom in einem Verhältnis im Bereich von 1 : 1 bis 1 : 100 (m : m) vorliegen.

7. Liganden-gerichteter kationischer Liposomen-Komplex nach einem vorstehenden Anspruch, wobei die Nukleinsäuremoleküle in einem molaren Verhältnis von 0,1 mol: 10 mol bis 10 mol: 0,1 mol (ein oder mehrere Nukleinsäuremoleküle, die ein oder mehrere Virusantigene kodieren : ein oder mehrere Nukleinsäuremoleküle, die ein oder mehrere Interleukine kodieren) vorliegen.

8. Liganden-gerichteter kationischer Liposomen-Komplex nach einem vorstehenden Anspruch, wobei das eine oder die mehreren Nukleinsäuremoleküle, die ein oder mehrere Virusantigene kodieren, das eine oder die mehreren Nukleinsäuremoleküle, die ein oder mehrere Interleukine kodieren, und das kationische Liposom in einem Gewichtsverhältnis von zwischen 1 : 1 bis 1 : 40 (µg gesamte Nukleinsäuren : µg Liposom) vorliegen.

9. Liganden-gerichteter kationischer Liposomen-Komplex nach einem vorstehenden Anspruch für die Liganden-gerichtete Abgabe für die Verwendung bei der Induktion einer Immunreaktion gegen ein Virusantigen in einem Säugetier und/oder für die Verwendung bei der Behandlung oder Prävention einer Viruserkrankung bei einem Säugetier, und wobei das Virusantigen von einem Virus stammt, das aus der Gruppe ausgewählt ist, die aus Folgenden besteht: Influenza-Virus, HIV-Virus, SARS-Virus, Vogelgrippe-Virus, Ebola-Virus, Hepatitis-B-Virus und Pocken-Virus, und/oder wobei der Komplex als Bolus oder als Infusion über einen Weg verabreicht wird, der aus der Gruppe ausgewählt ist, die aus Folgenden besteht: intravenöser Verabreichung, oraler Verabreichung, intramuskulärer Verabreichung, intraläsionaler Verabreichung, intradermaler Verabreichung, transdermaler Verabreichung, intraokularer Verabreichung, intraperitonealer Verabreichung, perkutaner Verabreichung, Aerosolverabreichung, intranasaler Verabreichung, intraorganer Verabreichung, intrazerebraler Verabreichung, topischer Verabreichung, subkutaner Verabreichung, endoskopischer Verabreichung, Implantat mit langsamer Freisetzung und Verabreichung über eine osmotische oder mechanische Pumpe.

10. Anti-Transferrinrezeptor-Einzelketten-Fv (TfRscFv)-gerichteter kationischer Immunoliposomen-Komplex für die TfRscFv-gerichtete Abgabe für die Verwendung bei der Abgabe von Nukleinsäuremolekülen an professionelle oder nichtprofessionelle Antigen-präsentierende Zellen (APCs) eines Säugetiers, der ein kationisches Liposom, ein TfRscFv und Nukleinsäuremoleküle umfasst, wobei das TfRscFv direkt mit dem kationischen Liposom komplexiert ist, doch nicht chemisch damit konjugiert ist,
wobei sich die APCs in der Leber, Milz, den Lymphknoten, im Darm und/oder Respirationstrakt befinden,
wobei der Komplex als Bolus oder als Infusion über einen Weg verabreicht wird, der aus der Gruppe ausgewählt ist, die aus Folgenden besteht: intravenöser Verabreichung, oraler Verabreichung, intramuskulärer Verabreichung, intraläsionaler Verabreichung, intradermaler Verabreichung, transdermaler Verabreichung, intraokularer Verabreichung, intraperitonealer Verabreichung, perkutaner Verabreichung, Aerosolverabreichung, intranasaler Verabreichung, intraorganer Verabreichung, intrazerebraler Verabreichung, topischer Verabreichung, subkutaner Verabreichung, endoskopischer Verabreichung, Implantat mit langsamer Freisetzung und Verabreichung über eine osmotische oder mechanische Pumpe,
wobei die Nukleinsäuremoleküle ein oder mehrere Virusantigene kodieren, die aus der Gruppe ausgewählt sind, die aus Folgenden besteht: HIV-Virusantigenen, Ebola-Virusantigenen, Influenza-Virusantigenen, SARS-Virusantigenen, Vogelgrippe-Virusantigenen, Hepatitis-B-Virusantigenen und Pocken-Virusantigen, und wobei die Nukleinsäuremoleküle auch ein oder mehrere Interleukine kodieren, die aus der Gruppe ausgewählt sind, die aus IL-6, IL-12 und IL-23 besteht.

11. Verfahren zur Herstellung eines Liganden-gerichteten kationischen Immunoliposomen-Komplexes nach einem der Ansprüche 1 bis 9, das Folgendes umfasst:
Herstellen eines Liganden;
Mischen des Liganden mit einem kationischen Liposom, um ein Liganden-gerichtetes kationisches Liposom zu bilden, wobei der Ligand direkt mit dem kationischen Liposom komplexiert ist, doch nicht chemisch damit konjugiert ist, und
Mischen des Liganden-gerichteten kationischen Liposoms mit:
einem oder mehreren Nukleinsäuremolekülen, die ein oder mehrere Virusantigene kodieren; und
einem oder mehreren Nukleinsäuremolekülen, die ein oder mehrere Interleukine kodieren, um den Liganden-gerichteten kationischen Liposomen-Komplex zu bilden,
welcher Komplex optional verwendet wird bei der Herstellung eines Medikaments für die Induktion einer Immunreaktion gegen ein Virusantigen in einem Säugetier und/oder für die Behandlung oder Prävention einer Viruserkrankung bei einem Säugetier, wobei das Virusantigen von einem Virus stammt, das aus der Gruppe ausgewählt ist, die aus Folgenden besteht: Influenza-Virus, HIV-Virus, SARS-Virus, Vogelgrippe-Virus, Ebola-Virus, Hepatitis-B-Virus und Pocken-Virus, wobei der Komplex als Bolus oder als Infusion über einen Weg verabreicht wird, der aus der Gruppe ausgewählt ist, die aus Folgenden besteht: intravenöser Verabreichung, oraler Verabreichung, intramuskulärer Verabreichung, intraläsionaler Verabreichung, intradermaler Verabreichung, transdermaler Verabreichung, intraokularer Verabreichung, intraperitonealer Verabreichung, perkutaner Verabreichung, Aerosolverabreichung, intranasaler Verabreichung, intraorganer Verabreichung, intrazerebraler Verabreichung, topischer Verabreichung, subkutaner Verabreichung, endoskopischer Verabreichung, Implantat mit langsamer Freisetzung und Verabreichung über eine osmotische oder mechanische Pumpe.

12. Pharmazeutische Zusammensetzung, die ein Liganden-gerichtetes kationisches Liposom nach einem der Ansprüche 1 bis 9 umfasst.

13. Pharmazeutische Zusammensetzung nach Anspruch 12, die weiter ein zweites Liganden-gerichtetes kationisches Liposom nach einem der Ansprüche 1 bis 9 umfasst.

14. Pharmazeutische Zusammensetzung nach Anspruch 12 oder Anspruch 13, die weiter einen oder mehrere Hilfsstoffe umfasst, die aus der Gruppe ausgewählt sind, die aus einem oder mehreren antibakteriellen Mitteln, einem oder mehreren Konservierungsmitteln, einem oder mehreren Puffern und einem oder mehreren Tensiden besteht.

15. Liganden-gerichtetes kationisches Liposom nach einem der Ansprüche 1-9, wobei das Anti-Transferrinrezeptor-scFv und das kationische Liposom in einem Verhältnis im Bereich von 1 : 20 bis 1 : 40 (m : m) vorliegen, wobei die Nukleinsäuren in einem molaren Verhältnis von 1 mol: 1 mol (ein oder mehrere Nukleinsäuremoleküle, die ein oder mehrere Virusantigene kodieren : ein oder mehrere Nukleinsäuremoleküle, die ein oder mehrere Interleukine kodieren) vorliegen und wobei das eine oder die mehreren Nukleinsäuremoleküle, die ein oder mehrere Virusantigene kodieren, das eine oder die mehreren Nukleinsäuremoleküle, die ein oder mehrere Interleukine kodieren, und das kationische Liposom in einem Gewichtsverhältnis von 1:10 (µg gesamte Nukleinsäuren : µg Liposom) vorliegen.

## Revendications

1. Complexe liposome cationique ciblé par un ligand comprenant un liposome cationique, un ligand qui est une chaîne simple du récepteur de l'anti-transferrine Fv (TfRscFv), une ou plusieurs molécules d'acide nucléique codant pour un ou plusieurs antigènes viraux, et une ou plusieurs molécules d'acide nucléique codant pour une ou plusieurs interleukines sélectionnées parmi l'IL-6, l'IL-12 et l'IL-23, dans lequel ladite chaîne simple du récepteur de l'anti-transferrine Fv (TfRscFv) est directement complexée avec, mais pas chimiquement conjuguée, audit liposome cationique.

2. Complexe liposome cationique ciblé par un ligand selon la revendication 1, dans lequel le dit un antigène viral ou plusieurs sont sélectionnés dans le groupe constitué d'antigènes viraux du SIDA, d'antigènes viraux grippaux, d'antigènes viraux du SARS, d'antigènes viraux de la grippe aviaire, d'antigènes viraux de la maladie à virus Ébola, d'antigènes viraux de l'hépatite B et d'antigènes viraux de la variole.

3. Complexe cationique ciblé par un ligand selon la revendication 1 ou la revendication 2, dans lequel lesdites molécules cationiques d'acide nucléique sont encapsulées dans ledit liposome cationique.

4. Complexe cationique ciblé par un ligand selon n'importe quelle revendication précédente, dans lequel le liposome cationique ciblé par un ligand comprend en outre un peptide comprenant un peptide K[K(H)KKK]₅-K(H)KKC(HoKC)(SEQ ID NO :1) associé audit complexe.

5. Complexe liposome cationique ciblé par un ligand selon n'importe quelle revendication précédente, dans lequel lesdites molécules d'acide nucléique sont associées à une monocouche interne ou externe dudit liposome cationique, et dans lequel ledit liposome cationique comprend un mélange de dioléoyltriméthylammonium phosphate avec dioléoylphosphatidyléthanolamine et cholestérol, un mélange de dioléoytriméthylammonium phosphate avec cholestérol, un mélange de diméthyldioctadécylammonium bromure avec dioléoylphosphatidyléthanolamine et cholestérol, de dioléoytriméthylammonium phosphate avec dioléoylphosphatidyléthanolamine, un mélange de diméthyldioctadécylammonium bromure avec dioléoylphosphatidyléthanolamine ou un mélange de diméthyldioctadécylammonium bromure avec cholestérol.

6. Complexe liposome cationique ciblé par un ligand selon la revendication 1, dans lequel ladite chaîne simple du récepteur de l'antitranferrine Fv (TfRscFv) et le dit liposome cationique sont présents selon un rapport compris dans la gamme de 1 : 1 à 1 : 100 (p : p).

7. Complexe liposome cationique ciblé par un ligand selon n'importe quelle revendication précédente, dans lequel lesdites molécules d'acide nucléique sont présentes selon un rapport molaire de 0,1 mole : 10 moles à 10 moles : 0,1 mol (une ou plusieurs molécules d'acide nucléique codant pour un ou plusieurs antigènes viraux ; une ou plusieurs molécules d'acide nucléique codant pour une ou plusieurs interleukines).

8. Complexe liposome cationique ciblé par un ligand selon n'importe quelle revendication précédente, dans lequel ladite une ou plusieurs molécules d'acide nucléique codant pour un ou plusieurs antigènes viraux, ladite une ou plusieurs molécules d'acide nucléique codant pour une ou plusieurs interleukines, et le dit liposome cationique sont présents selon un rapport compris entre 1 : 1 à 1 : 40 (µg d'acide nucléique total : µg d'acide nucléique total : µg de liposome).

9. Complexe liposome cationique ciblé par un ligand selon n'importe quelle revendication précédente conçu pour une libération ciblée par un ligand pouvant être utilisé pour induire une réponse immunitaire contre un antigène viral dans un mammifère et/ou pouvant être utilisé dans le traitement ou la prévention d'une maladie virale chez un mammifère, et dans lequel le dit antigène viral provient d'un virus sélectionné dans le groupe constitué du virus grippal, du virus du SIDA, du virus du SARS, du virus de la grippe aviaire, du virus Ébola, du virus de l'hépatite B et du virus de la variole, et/ou, dans lequel le dit complexe est administré en bolus ou par perfusion par l'intermédiaire d'une voie d'administration sélectionnée dans le groupe constitué d'une administration intraveineuse, d'une administration orale, d'une administration intramusculaire, d'une administration intralésionnelle, d'une administration intradermique, d'une administration transdermique, d'une administration intraoculaire, d'une administration intrapéritonéale, d'une administration percutanée, d'une administration par aérosol, d'une administration intra-organes, d'une administration intracérébrale, d'une administration topique, d'une administration sous-cutanée, d'une administration endoscopique, d'un implant à libération lente et d'une administration par l'intermédiaire d'une pompe osmotique ou mécanique.

10. Complexe immunoliposome cationique ciblé sur une chaîne simple du récepteur de l'anti-transferrine Fv (TfRscFv) conçu pour une libération ciblée par le TfRscFv pouvant être utilisé pour délivrer des molécules d'acide nucléique à des cellules présentatrices d'antigènes (CPA) professionnelles ou non professionnelles d'un mammifère, comprenant un liposome cationique, un TfRscFv, et des molécules d'acide nucléique, dans lequel le TfRscFv est directement complexé avec, mais pas chimiquement conjugué au liposome cationique,
dans lequel les CPA sont situées dans le foie, la rate, les ganglions lymphatiques, les intestins et/ou les voies respiratoires,
dans lequel ledit complexe est administré en bolus ou par perfusion par l'intermédiaire d'une voie d'administration sélectionnée dans le groupe constitué d'une administration intraveineuse, d'une administration orale, d'une administration intramusculaire, d'une administration intralésionnelle, d'une administration intradermique, d'une administration transdermique, d'une administration intraoculaire, d'une administration intrapéritonéale, d'une administration percutanée, d'une administration par aérosol, d'une administration intra-organes, d'une administration intracérébrale, d'une administration topique, d'une administration sous-cutanée, d'une administration endoscopique, d'un implant à libération lente et d'une administration par l'intermédiaire d'une pompe osmotique ou mécanique,
dans lequel lesdites molécules d'acide nucléique codent pour un ou plusieurs antigènes viraux sélectionnés dans le groupe constitué d'antigènes viraux du SIDA, d'antigènes viraux Ébola, d'antigènes viraux grippaux, d'antigènes viraux du SARS, d'antigènes viraux de la grippe aviaire, d'antigènes grippaux de l'hépatite B et d'antigènes grippaux de la variole, et dans lequel lesdites molécules d'acide nucléique codent également pour une ou plusieurs interleukines sélectionnées dans le groupe constitué d'IL-6, d'IL-12 et d'IL-23.

11. Procédé de préparation d'un complexe immunoliposome cationique ciblé par un ligand selon n'importe lesquelles des revendications 1 à 9, qui comprend :
la préparation d'un ligand ;
le mélange du ligand avec un liposome cationique pour former un liposome cationique ciblé par le ligand, dans lequel le ligand est directement complexé, mais pas chimiquement conjugué, au liposome cationique, et
le mélange du liposome cationique ciblé par le ligand avec :
une ou plusieurs molécules codant pour un ou plusieurs antigènes viraux ; et
une ou plusieurs molécules d'acide nucléique codant pour une ou plusieurs interleukines, pour former le complexe liposome cationique ciblé par le ligand,
lequel complexe est facultativement utilisé dans la fabrication d'un médicament pour induire une réponse immunitaire contre un antigène viral chez un mammifère et/ou pour traiter ou empêcher une maladie virale chez un mammifère, dans lequel ledit antigène viral provient d'un virus sélectionné dans le groupe constitué du virus grippal, du virus du SIDA, du virus du SARS, du virus de la grippe aviaire, du virus Ébola, du virus de l'hépatite B et du virus de la variole,
dans lequel ledit complexe est administré en bolus ou par perfusion par l'intermédiaire d'une voie d'administration sélectionnée dans le groupe constitué d'une administration intraveineuse, d'une administration orale, d'une administration intramusculaire, d'une administration intralésionnelle, d'une administration intradermique, d'une administration transdermique, d'une administration intraoculaire, d'une administration intrapéritonéale, d'une administration percutanée, d'une administration par aérosol, d'une administration intra-organes, d'une administration intracérébrale, d'une administration topique, d'une administration sous-cutanée, d'une administration endoscopique, d'un implant à libération lente et d'une administration par l'intermédiaire d'une pompe osmotique ou mécanique.

12. Composition pharmaceutique comprenant un liposome cationique ciblé par un ligand selon n'importe lesquelles des revendications 1 à 9.

13. Composition pharmaceutique selon la revendication 12, comprenant en outre un second liposome cationique ciblé par un ligand selon n'importe lesquelles des revendications 1 à 9.

14. Composition pharmaceutique selon la revendication 12 ou la revendication 13, comprenant en outre un ou plusieurs constituants sélectionnés dans le groupe constitué d'un ou de plusieurs agents antibactériens, d'un ou plusieurs agents de conservation, d'un ou plusieurs tampons et d'un ou plusieurs agents tensioactifs.

15. Liposome cationique ciblé par un ligand de n'importe lesquelles des revendications 1 à 9, dans lequel le récepteur d'anti-transferrine scFv et le dit liposome cationique sont présents selon un rapport compris dans la gamme de 1 : 20 à 1 : 40 (p : p), dans lequel les acides nucléiques sont présents selon un rapport molaire de 1 mole : 1 mole (une ou plusieurs molécules d'acide nucléique codant pour un ou plusieurs antigènes viraux : une ou plusieurs molécules d'acide nucléique codant pour une ou plusieurs interleukines), et dans lequel ladite une ou plusieurs molécules d'acide nucléique codant pour un ou plusieurs antigènes viraux, ladite une ou plusieurs molécules d'acide nucléique codant pour une ou plusieurs interleukines, et le dit liposome cationique sont présents selon un rapport de poids de 1:10 (µg d'acide nucléique total : µg de liposome).


### SEQUENCE LISTING

<110> Georgetown University
<120> Methods for Generating Immune Response Using Cationic-Liposome-Mediated Nucleic Acid Delivery
<130> 2474.012PC01
<140> To Be Assigned
   <141> Herewith
<150> US 60/929,685
   <151> 2007-07-09
<160> 1
<170> PatentIn version 3.3
<210> 1
   <211> 31
   <212> PRT
   <213> Artificial Sequence
<220>
   <223> Chemically synthesized - HoKC
<400> 1
   Lys Lys His Lys Lys Lys Lys His Lys Lys Lys Lys His Lys Lys Lys
   1               5                   10                  15
   Lys His Lys Lys Lys Lys His Lys Lys Lys Lys His Lys Lys Cys
               20                  25                  30